# EUROPEAN PATENT APPLICATION

(11) **EP 3 896 062 A1**
(43) Date of publication of application: **20.10.2021**
(21) Application number: 19893951.4
(22) Date of filing: 06.12.2019
(51) Int. Cl.: C07D 401/04, C07D 401/14, A61K 31/4412, A61K 31/4745, A61P 35/00

(54) **ISOINDOLINE COMPOUND, AND PREPARATION METHOD, PHARMACEUTICAL COMPOSITION, AND APPLICATION OF ISOINDOLINE COMPOUND**

(30) Priority: 06.12.2018 CN 201811488140
(71) Applicant: Shanghai Institute of Materia Medica, Chinese Academy of Sciences, Pudong, Shanghai 201203 (CN)
(72) Inventor: CHEN, Xiaohua, Shanghai 201203 (CN); LI, Jia, Shanghai 201203 (CN); CHENG, Yu, Shanghai 201203 (CN); ZHOU, Yubo, Shanghai 201203 (CN); NIE, Huijun, Shanghai 201203 (CN); WANG, Yujie, Shanghai 201203 (CN); GUO, Andi, Shanghai 201203 (CN); KAN, Weijuan, Shanghai 201203 (CN)
(74) Representative: Lavoix
(86) International application number: PCT/CN2019/123643
(87) International publication number: WO 2020/114482

(57) **Abstract**

The present invention relates to an isoindoline compound as represented by general formula (I) and used as a CRBN regulator, and a preparation method, a pharmaceutical composition, and an application of the isoindoline compound. Specifically, a class of polysubstituted isoindoline compound provided in the present invention, as a class of CRL4^{CRBN} E3 ubiquitin ligase regulator having a novel structure, has good anti-tumor activity and immunoregulatory activity, and can be used for preparing drugs for treating diseases associated with a CRL4^{CRBN} E3 ubiquitin ligase.

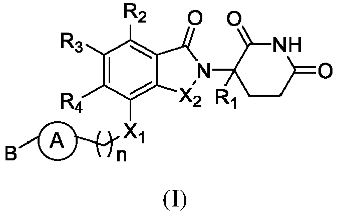

## Description

### TECHNICAL FIELD

The present invention relates to a class of isoindoline compound with novel structure, pharmaceutically acceptable salt, solvate, pharmaceutical composition, and use thereof in the manufacture of medicant for the treatment or prevention of various diseases.

### BACKGROUND OF THE INVENTION

Tight regulation of protein expression in cells plays an important role in cell function, cell survival and division. Many primary or acquired diseases usually involve abnormal protein function. The traditional method of regulating protein dysfunction is mainly to design targeted inhibitors or agonists. These targeted drugs play an important role in the treatment of diseases. Nevertheless, in order to obtain a satisfactory therapeutic effect, these inhibitors or agonists usually need to be maintained at a higher drug concentration to achieve an effective therapeutic effect, which also leads to adverse drug reactions to a certain extent. Another way to regulate the abnormal function of proteins is to change the dynamic balance of pathologically related proteins. The dynamic balance of proteins involves the synthesis and degradation of proteins, for example by using small interfering RNA(siRNA), antisense oligonucleotides, or gene editing techniques to knock out or silence target protein genes. These nucleic acid-based technologies change protein synthesis by acting on the transcription and translation process of the target protein. The biggest limitation of this type of technology lies in low stability and bioavailability of nucleic acid in vivo, which further limits its application to some extent. Another strategy to regulate the dynamic balance of proteins is to regulate the process of protein degradation, which can directly change the expression of target proteins in cells by promoting or inhibiting the degradation of proteins. Ubiquitin-Proteasome System (UPS) plays an important role in the degradation of proteins. Under the action of a series of ubiquitin enzymes, the target protein can be labeled by ubiquitin, and proteins with specific ubiquitin tags can be transported to the proteasome for degradation.

The process of protein ubiquitination is a series of multi-step reactions, mainly involving three types of enzymes: E1 ubiquitin activating enzyme, E2 ubiquitin conjugating enzyme, and E3 ubiquitin ligase. E3 ubiquitin ligases can be divided into three categories according to their conserved domains and mode of action. Among them, TECT family and RBR family, E3 ubiquitin ligase first transfers ubiquitin from E2 ubiquitin activating enzyme to itself and then transfers ubiquitin from E3 ubiquitin ligase to substrate protein during substrate ubiquitination. In comparison, the RING family E3 ubiquitin ligase occupies a larger proportion in the entire E3 ubiquitin ligase. This type of E3 ubiquitin ligase contains the RING domain or RING like domains, they can bind to the E2 ubiquitin conjugating enzyme, and promote the direct transfer of ubiquitin from the E2 ubiquitin conjugating enzyme to the substrate protein. CRL4 ^{CRBN} E3 ubiquitin ligase belongs to the RING family E3 ubiquitin ligase, which is a protein complex assembled from multiple subunits. The complex consists of a substrate protein recognition module (CRBN), an E2 ubiquitin conjugating enzyme recognition module (RING domain) and a linker moiety (Cullin protein) between them. CRBN directly binds to the substrate in the entire protein complex and controls the substrate specificity of the entire ubiquitination process.

Small molecule modulators that act directly on CRBN can control the substrate selectivity of CRL4^{CRBN} E3 ubiquitin ligase. New research found that Cereblon (gene name: CRBN) is a direct target of immunomodulator-thalidomide and its analogues (Science, 2010, 327, 1345; Science, 2014, 343, 301; Science, 2014, 343, 305; Nature, 2015, 523, 183.). It has been demonstrated that dosamine immunomodulators can selectively induce ubiquitination and degradation of transcription factors IKZF1 and IKZF3 in multiple myeloma cell lines by regulating the activity of CRBN-ubiquitin ligase complex. This process changes the functions of T cells and B cells, and at the same time produces toxic effects on multiple myeloma cells, thus achieving a therapeutic effect on malignant myeloid systems including multiple myeloma. Recent studies have shown that lenalidomide, an analog of thalidomide, can selectively induce the ubiquitination and degradation of CK1a through CRL4^{CRBN}E3 ubiquitin ligase, thus achieving the treatment of 5q deletion myelodysplastic syndrome (MDS). However, another structural analogue of thalidomide (CC-885) can selectively induce and degrade GSPT1 by acting on CRL4 ^{CRBN} E3 ubiquitin ligase, and exhibits strong cytotoxicity to a variety of tumor cells.

Existing research results show that different dosamine drug molecules have different specificity of substrate protein degradation after interacting with target CRBN. When lenalidomide is used in the treatment of multiple myeloma, its therapeutic effect is mainly achieved through the selective degradation of IKZF1 and IKZF3; and in the treatment of 5q deletion myelodysplastic syndrome (del(5q) MDS) mainly through degradation of CK1a. Lenalidomide is one main dosamine analogues that have been developed at present, and it shows strong degradation activity against CK1a, so it is the most important clinically effective dosamine drugs in the treatment of myelodysplastic syndrome del(5q) MDS. Thalidomide approved by FDA is used for the treatment of erythema nodosum leprosy, lenalidomide is used to treat prostate cancer in clinical trials, and pomalidomide is used to treat myelofibrosis in clinical trials. The indications of dosamine drugs are expanding with the development of new dosamine drugs and the development of clinical trials in which lenalidomide is used alone or in combination with other therapeutic agents for the treatment of a variety of cancer, pain, central nervous system diseases and immune system-related diseases (see WO2012/015986).

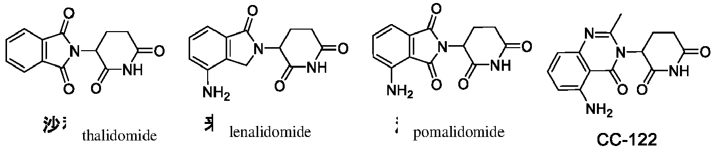

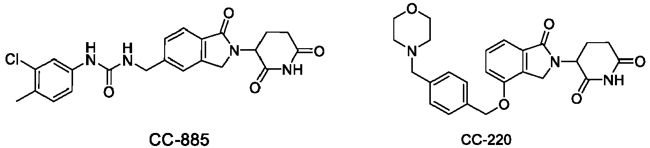

The reported compounds lenalidomide, pomalidomide, CC-122, CC-220, CC-885 are similar to thalidomide in structure. The characteristic of this class of compounds lies in that after structural changes and adjustments, the compounds have different pharmacological activity and completely different therapeutic effects, and can be used clinically to treat different indications.

WO2008115516A2, US8153659B2, US9181216B2, US9920027B2 have disclosed the compound represented by the general formula S1: 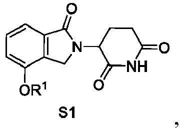 the main representative R1 in the general formula S1 is aryl, arylalkyl, heterocyclylalkyl, etc.

WO2011100380 A1, CN102822165B have disclosed a class of compounds represented by the general formula S2: 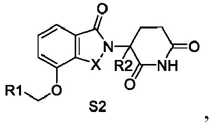 in the general formula S2, R1 is a multi-substituted aryl, and the representative compound is CC-220:

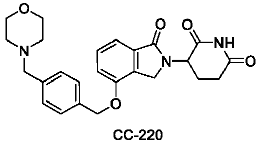

WO2016065980A1, CN105566290A, US10017492B2 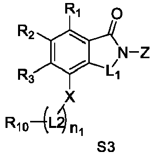 the representative compounds in the general formula S3 are:

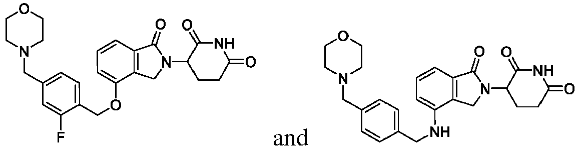

WO2007027527A2, CN101291924A, US8481568B2 have disclosed a class of compounds represented by the general formula S3: 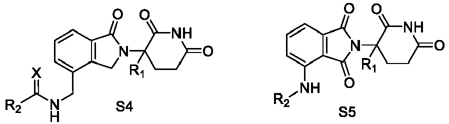 the representative compounds in the general formula S4 and S5 are:

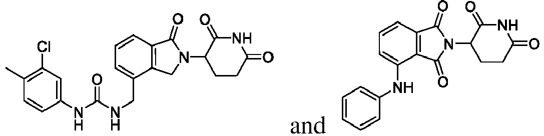

WO2008027542A2, US8877780B2, US9447070B2 have disclosed a class of compounds represented by the general formula S3: 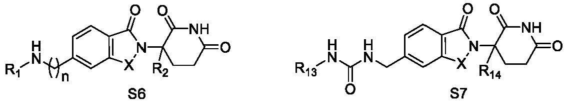 the representative compounds in the general formula S6 and S7 are:

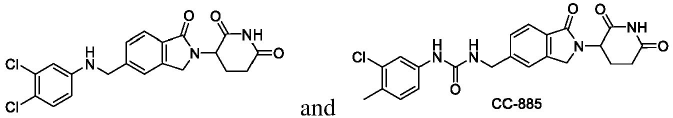

The mechanism of action of lenalidomide and some of the above-mentioned molecules is that compounds of different structures can bind to CRBN, causing the conformational change of the CRBN binding part, thereby recruiting different endogenous biological macromolecules to bind with CRBN; and further ubiquitinate and degrade the potentially different endogenous substrate proteins, which can produce different pharmacological activities and be used in clinical trials to treat different indications.

Summary, lenalidomide is mainly used for the treatment of multiple myeloma and myelodysplastic syndrome, but the effect is not ideal for other indications; other above-mentioned compounds such as CC-122, CC-885 and CC-220 are still in preclinical or clinical research. Therefore, the development of novel structural compounds as CRL4 ^{CRBN} E3 ubiquitin ligase modulators can further improve the therapeutic effect of tumors and expand the clinical needs of new indications of domide drugs. The pharmacological activities and pharmacological properties of the different structure of the domide molecules are not known, and the properties and effects of all aspects are uncertain. Based on the mechanism of action of the dosamine molecule, the development of a new structure of the dosamine molecule can realize the recruitment of new protein substrates, thereby achieving the improvement of the therapeutic effect and the expansion of new indications. Therefore, it is of great research value and practical significance to continue to develop novel structures of CRL4^{CRBN}E3 ubiquitin ligase modulators to expand new indications.

### Summary of the invention

The inventors of the present invention obtained the following important information by analyzing the crystal structure of the complex between CRL4 ^{CRBN} E3 ubiquitin ligase and small molecules (PDB ID: 4CI2, 5HXB): CRL4 ^{CRBN} E3 ubiquitin ligase has multiple binding pockets with small molecules. Therefore, small molecules with complex structure and multiple binding sites can be developed to realize effective binding between CRL4 ^{CRBN} E3 ubiquitin ligase and small molecules. At the same time, molecular dynamics simulation methods are used to analyze the structure dynamics and binding site of the interface between the model molecule and E3 ubiquitin ligase, combining molecular docking and complex-based pharmacophore matching strategy, and scoring binding mode and interaction of the active site of the compound on the E3 ubiquitin ligase by scoring function, and computational simulation and optimization of structural design to obtain a novel specific CRL4 ^{CRBN} E3 ubiquitin ligase small molecule modulator. Based on this information, we designed and synthesized a series of small molecule modulators of CRL4 ^{CRBN} E3 ubiquitin ligase described in this application, and tested the activity of the compounds. The test results of some representative compounds in multiple myeloma cell line (MM.1S), mantle cell lymphoma cell line (Mino), and acute myeloid leukemia cell line (MV-4-11) show that the new small molecule regulator has very high cell growth inhibitory activity. After the molecule acts on organisms, it can regulate the degradation of substrate proteins by regulating the ubiquitin-proteasome mediated protein degradation pathway in organisms, so as to achieve effective disease therapy based on CRBN target.

An aspect of the present invention is to provide the compound of formula (I), the enantiomer, diastereomer, racemate, isotopic compound, metabolic precursor, metabolite, pharmaceutically acceptable salt, ester, prodrug or hydrate thereof.

Another aspect of the present invention is to provide the method for preparing the compound of formula (I), important intermediates for the preparation of the compound and the preparation method thereof.

Another aspect of the present invention is to provide the compound of formula (I), the tautomer, enantiomer, diastereomer, racemate, metabolite, metabolic precursor, isotopic compound, pharmaceutically acceptable salt, ester, prodrug or hydrate thereof, wherein the compound is used for the manufacture of a medicament or diagnostic reagent for the prevention or treatment of diseases related to CRL4 ^{CRBN} E3 ubiquitin ligase, preferably, the diseases related to CRL4 ^{CRBN} E3 ubiquitin ligase include cancer, pain, central nervous system diseases and immune system diseases.

In order to achieve the above object, the present invention provides the compound of formula (I) and the tautomer, enantiomer, diastereomer, racemate, metabolic precursor, metabolite, isotopic compound, pharmaceutically acceptable salt, ester, prodrug or hydrate thereof: 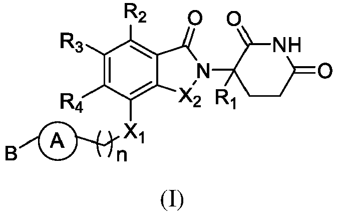 wherein X₁ is -CH₂-, -NH- or -O-;
X₂ is -CH₂- or -CO-;
R₁ is hydrogen, deuterium, fluorine or linear or branched C₁-C₆ hydrocarbyl;
R2 and R4 are each independently selected from hydrogen or deuterium;
R₃ is hydrogen, deuterium or halogen;
n is 1, 2, or 3;
Ⓐ is selected from the following groups: 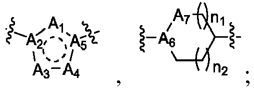
A₁ is elected from C, N, O, S or NR₅, wherein R₅ is selected from C₁-C₆ alkyl, C₁-C₆ haloalkyl or C₃-C₆ cycloalkyl;
A₃ and A₄ are each independently selected from C, N, O or S;
when A₁, A₃ or A₄ is C, A₁, A₃ or A₄ each can be independently substituted by methyl or ethyl;
A₂ and A₅ are each independently selected from C or N;
A₇ is selected from C, N, O or S;
A₆ is C or N, when A₆ is N, then the connection mode between Ⓐ and B is 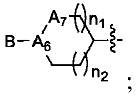
n₁ is 0, 1, 2 or 3;
n₂ is 0, 1, 2 or 3;
B is (6-10 membered aryl)―(CH₂)_{b1}―(CHR₆)_{b2}―, (5-10 membered heteroaryl)―(CH₂)_{b1}―(CHR₆)_{b2}―, (5-14 membered heterocyclyl)―(CH₂)_{b1}―(CHR₆)_{b2}―, (5-16 membered cycloalkyl)―(CH₂)_{b1}―(CHR₆)_{b2}―, the aryl, heteroaryl, heterocyclyl or cycloalkyl is substituted with one or more groups selected from the group consisting of deuterium, halogen, cyano, nitro, hydroxy, carboxy, aminocarbonyl, C₁-C₆alkyl, C₁-C₆alkoxyalkyl, C₁-C₆haloalkyl, C₂-C₁₀ alkenyl, C₂-C₁₀ alkynyl, hydroxyl substituted C₁-C₆ alkyl, C₁-C₆ alkoxy, C₁-C₆ alkylcarbonyl, C₁-C₆ alkylaminocarbonyl, C₁-C₆ alkylsulfonyl, C₁-C₆ haloalkoxy, hydroxyl substituted C₁-C₆ alkoxy, alkoxy substituted C₁-C₆ alkoxy, cyano substituted C₁-C₆ alkoxy, C₃-C₈ cycloalkyl, C₃-C₈ cycloalkyloxy, C₃-C₈ heterocyclyl, C₃-C₈ heterocyclyloxy, C₃-C₈ heterocyclylmethylene, halogen-substituted or unsubstituted phenyl, halogen-substituted or unsubstituted benzyl, halogen-substituted or unsubstituted phenoxy, C₅-C₆ heteroaryl, -NHC(O)Ra₁, -NHC(O)ORa₂, -NRa₃Ra₄, wherein Ra₁, Ra₂, Ra₃ and Ra₄ are each independently hydrogen, C₁₋₆ alkyl unsubstituted or substituted by halogen, hydroxy, cyano, or C3-6 cycloalkyl unsubstituted or substituted by halogen, hydroxy, cyano;
b₁ is 0, 1, 2 or 3;
b₂ is 0 or 1;
R6 is selected from deuterium, C₁-C₆ alkyl, C₁-C₆ haloalkyl, hydroxyl substituted C₁-C₆ alkyl, C₁-C₆ alkoxyalkyl, C₁-C₆ haloalkoxyalkyl, -CH₂NHC(O)Ra₅, -CH₂NRa₆Ra₇, wherein Ra₅, Ra₆ and Ra₇ are each independently hydrogen, C1-3 alkyl unsubstituted or substituted by halogen, hydroxyl, or C3-6 cycloalkyl unsubstituted or substituted by halogen, hydroxyl; when X₁ is -O-, and Ⓐ is 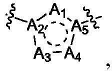 B is not 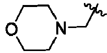 or 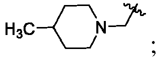
**preferably,** the compound represented by formula (I) and the tautomer, enantiomer, diastereomer, racemate, metabolite, metabolic precursor, isotopic compound, pharmaceutically acceptable salt, ester, prodrug or hydrate thereof:
   wherein X₁ is -CH₂-, -NH- or -O-;
   X₂ is -CH₂- or -CO-;
   R₁ is hydrogen, deuterium, fluorine or linear or branched C₁-C₆ hydrocarbyl;
   R₂ and R₄ are each independently selected from hydrogen or deuterium;
   R₃ is hydrogen, deuterium or halogen;
   n is 1, 2, or 3;
   Ⓐ is selected from the following groups: 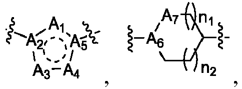
   5-membered heteroaromatic ring containing 1-3 heteroatoms selected from N, O or S, 4-6-membered heterocycle containing 1-3 heteroatoms selected from N, O or S, and a 4-6-membered aliphatic ring, wherein the carbon atom on the 5-membered heteroaromatic ring is optionally substituted by methyl or ethyl;
   when A₆ is N, the connection mode between Ⓐ and B is 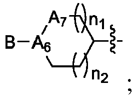
   Ⓐ is 5-membered heteroaromatic ring containing one heteroatom selected from N, O or S, preferably Ⓐ is selected from the group consisting of: 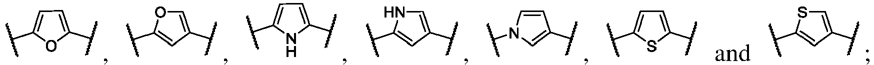
   or Ⓐ is 5-membered heteroaromatic ring containing two heteroatoms selected from N, O or S, preferably Ⓐ is selected from the following groups: 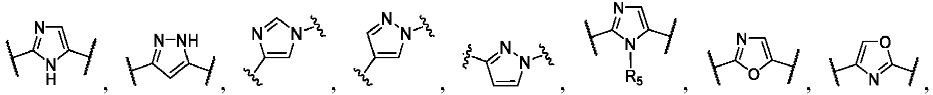 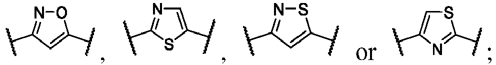
   Ⓐ is 5-membered heteroaromatic ring containing three heteroatoms selected from N, O or S, preferably Ⓐ is selected from the following groups: 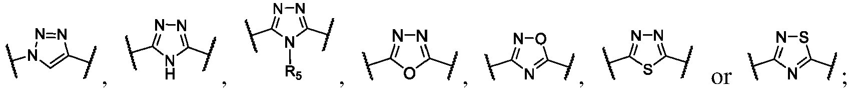
   Ⓐ is 4-membered aliphatic ring or heterocycle, preferably Ⓐ is selected from the following groups: 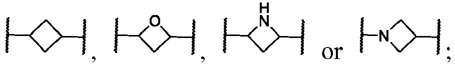
   Ⓐ is 5-membered aliphatic ring or heterocycle, preferably Ⓐ is selected from the following groups: 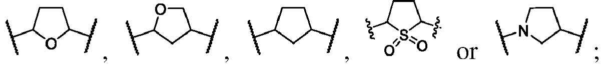
   Ⓐ is 6-membered aliphatic ring or heterocycle, preferably Ⓐ is selected from the following groups: 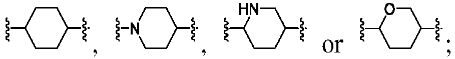
   wherein R₅ is selected from C₁-C₆ alkyl, halogen substituted C₁-C₆ alkyl or C₃-C₆ cycloalkyl;
   B is (6-10 membered aryl)―(CH₂)_{b1}―(CHR₆)_{b2}―, (5-10 membered heteroaryl)―(CH₂)_{b1}―(CHR₆)_{b2}―, (5-14 membered heterocyclyl)―(CH₂)_{b1}―(CHR₆)_{b2}―, (5-16 membered cycloalkyl)―(CH₂)_{b1}―(CHR₆)_{b2}―, the aryl, heteroaryl, heterocyclyl or cycloalkyl is substituted with one or more groups selected from the group consisting of deuterium, halogen, cyano, nitro, hydroxy, carboxy, aminocarbonyl, C₁-C₆ alkyl, C₂-C₁₀ alkenyl, C₂-C₁₀ alkynyl, C₁-C₆ alkoxyalkyl, C₁-C₆ haloalkyl, hydroxyl substituted C₁-C₆ alkyl, C₁-C₆ alkoxy, C₁-C₆ alkoxycarbonyl, C₁-C₆ alkylaminocarbonyl, C₁-C₆ alkylsulfonyl, C₁-C₆ haloalkoxy, hydroxyl substituted C₁-C₆ alkoxy, cyano substituted C₁-C₆ alkoxy, C₃-C₈ cycloalkyl, C₃-C₈ cycloalkyloxy, C₃-C₈ heterocyclyl, C₃-C₈ heterocyclyloxy, C₃-C₈ heterocyclylmethylene, halogen-substituted or unsubstituted phenyl, halogen-substituted or unsubstituted benzyl, halogen-substituted or unsubstituted phenoxy, C₅-C₆ heteroaryl, -NHC(O)Ra₁, -NHC(O)ORa₂, -NRa3Ra4, wherein Ra₁, Ra₂, Ra₃ and Ra₄ are each independently hydrogen, C1-6 alkyl unsubstituted or substituted by halogen, hydroxy, cyano, or C3-6 cycloalkyl unsubstituted or substituted by halogen, hydroxy, cyano;
   b₁ is 0, 1, 2 or 3;
   b₂ is 0 or 1;
   R6 is selected from deuterium, C₁-C₆ alkyl, C₁-C₆ haloalkyl, hydroxyl substituted C₁-C₆ alkyl, C₁-C₆ alkoxyalkyl, C₁-C₆ haloalkoxyalkyl, -CH₂NHC(O)Ra₅, -CH₂NRa₆Ra₇, wherein Ra₅, Ra₆ and Ra₇ are each independently hydrogen, C1-3 alkyl unsubstituted or substituted by halogen, hydroxyl, or C3-6 cycloalkyl unsubstituted or substituted by halogen, hydroxyl;
   when X₁ is -O-, and Ⓐ is 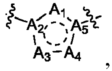 B is not 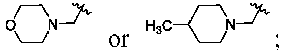 A₁, A₂, A₃, A₄, A₅, A₆ and A₇ are as defined above.

**More preferably,** the compound represented by formula (I) and the tautomer, enantiomer, diastereomer, racemate, metabolite, metabolic precursor, isotopic compound, pharmaceutically acceptable salt, ester, prodrug or hydrate thereof:
wherein R₃ is halogen;
X₁ is -CH₂, -NH- or -O-;
X₂ is -CH₂- or -CO-;
R₁ is hydrogen, deuterium, fluorine or methyl;
R₂ and R₄ are each independently selected from hydrogen or deuterium;
n is 1, 2, or 3;
Ⓐ is 5-membered heteroaromatic ring containing 1-3 heteroatoms selected from N, O or S, 4-6-membered heterocycle containing 1-3 heteroatoms selected from N, O or S, or a 4-6-membered aliphatic ring, wherein the carbon atom on the 5-membered heteroaromatic ring is optionally substituted by methyl or ethyl;
when A₆ is N, the connection mode between Ⓐ and B is 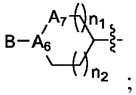
Ⓐ is 5-membered heteroaromatic ring containing one heteroatom selected from N, O or S, preferably Ⓐ is selected from the group consisting of: 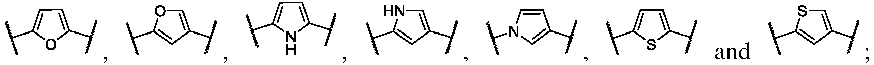
or Ⓐ is 5-membered heteroaromatic ring containing two heteroatoms selected from N, O or S, preferably Ⓐ is selected from the following groups: 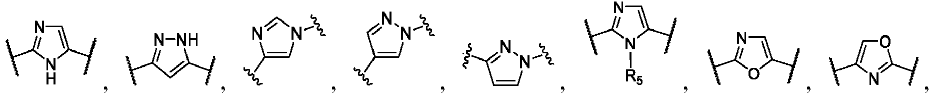 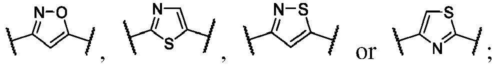
Ⓐ is 5-membered heteroaromatic ring containing three heteroatoms selected from N, O or S, preferably Ⓐ is selected from the following groups: 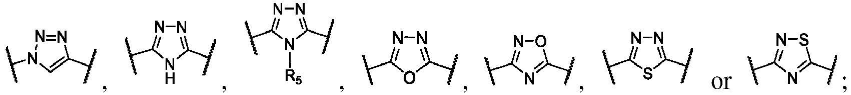
Ⓐ is 4-membered aliphatic ring or heterocycle, preferably Ⓐ is selected from the following groups: 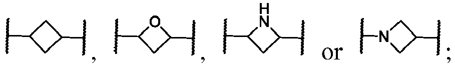
when Ⓐ is a 5-membered aliphatic ring, preferably Ⓐ is selected from the following groups: 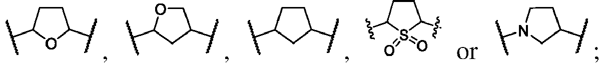
Ⓐ is 6-membered aliphatic ring or heterocycle, preferably Ⓐ is selected from the following groups: 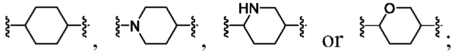
wherein R₅ is selected from C₁-C₆ alkyl, halogen substituted C₁-C₆ alkyl or C₃-C₆ cycloalkyl;
B is (6-10 membered aryl)―(CH₂)_{b1}―(CHR₆)_{b2}―, (5-10 membered heteroaryl)―(CH₂)_{b1}―(CHR₆)_{b2}―, (5-14 membered heterocyclyl)―(CH₂)_{b1}―(CHR₆)_{b2}―, (5-16 membered cycloalkyl)―(CH₂)_{b1}―(CHR₆)_{b2}―, the aryl, heteroaryl, heterocyclyl or cycloalkyl is substituted with one or more groups selected from the group consisting of deuterium, halogen, cyano, nitro, hydroxy, carboxy, aminocarbonyl, C₁-C₆ alkyl, C₂-C₁₀ alkenyl, C₂-C₁₀ alkynyl, C₁-C₆ alkoxyalkyl, C₁-C₆ haloalkyl, hydroxyl substituted C₁-C₆ alkyl, C₁-C₆ alkoxy, C₁-C₆ alkoxycarbonyl, C₁-C₆ alkylaminocarbonyl, C₁-C₆ alkylsulfonyl, C₁-C₆ haloalkoxy, hydroxyl substituted C₁-C₆ alkoxy, cyano substituted C₁-C₆ alkoxy, C₃-C₈ cycloalkyl, C₃-C₈ cycloalkyloxy, C₃-C₈ heterocyclyl, C₃-C₈ heterocyclyloxy, C₃-C₈ heterocyclylmethylene, halogen-substituted or unsubstituted phenyl, halogen-substituted or unsubstituted benzyl, halogen-substituted or unsubstituted phenoxy, C₅-C₆ heteroaryl, -NHC(O)Ra₁, -NHC(O)ORa₂, -NRa3Ra4, wherein Ra₁, Ra₂, Ra₃ and Ra₄ are each independently hydrogen, C1-6 alkyl unsubstituted or substituted by halogen, hydroxy, cyano, or C3-6 cycloalkyl unsubstituted or substituted by halogen, hydroxy, cyano;
b₁ is 0, 1, 2 or 3;
b₂ is 0 or 1;
R6 is selected from deuterium, C₁-C₆ alkyl, C₁-C₆ haloalkyl, hydroxyl substituted C₁-C₆ alkyl, C₁-C₆ alkoxyalkyl, C₁-C₆ haloalkoxyalkyl, -CH₂NHC(O)Ra₅, -CH₂NRa₆Ra₇, wherein Ra₅, Ra₆ and Ra₇ are each independently hydrogen, C1-3 alkyl unsubstituted or substituted by halogen, hydroxyl, or C3-6 cycloalkyl unsubstituted or substituted by halogen, hydroxyl;
A₁, A₂, A₃, A₄, A₅, A₆ and A₇ are as defined above.

**Further preferably,** the compound represented by formula (I) and the tautomer, enantiomer, diastereomer, racemate, metabolite, metabolic precursor, isotopic compound, pharmaceutically acceptable salt, ester, prodrug or hydrate thereof:
wherein X₁ is -CH₂- or -NH-;
X₂ is -CH₂- or -CO-;
R₁ is hydrogen, deuterium, fluorine or methyl;
R₃ is selected from hydrogen, deuterium or fluorine;
R₂, R₄, n, Ⓐ and B are as defined and preferred above.

**In a preferred embodiment,** the compound represented by formula (I) and the tautomer, enantiomer, diastereomer, racemate, metabolite, metabolic precursor, isotopic compound, pharmaceutically acceptable salt, ester, prodrug or hydrate thereof, wherein the compound represented by formula (I) is the compound represented by the general formulas (I-1) to (I-12): 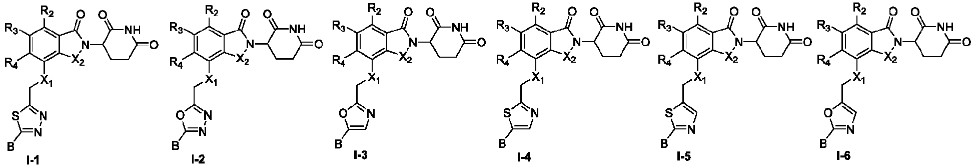 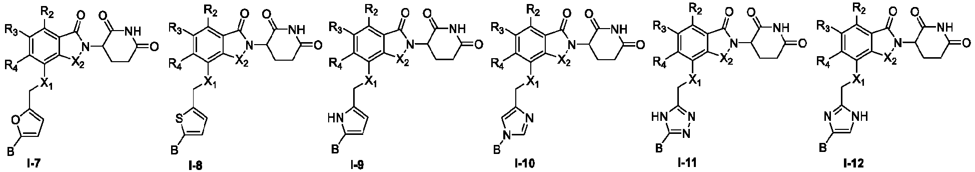 wherein X₁ is -CH₂-, -NH- or -O-;
X₂ is -CH₂- or -CO-;
R₂ and R₄ are each independently selected from hydrogen or deuterium;
R₃ is selected from hydrogen, deuterium or fluorine;
B is as defined above, when X₁ is -O-, B is not

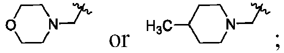

**In a preferred embodiment,** the compound represented by formula (I) and the tautomer, enantiomer, diastereomer, racemate, metabolite, metabolic precursor, isotopic compound, pharmaceutically acceptable salt, ester, prodrug or hydrate thereof, wherein the compound represented by formula (I) is the compound represented by the general formulas (I-13) to (I-18): 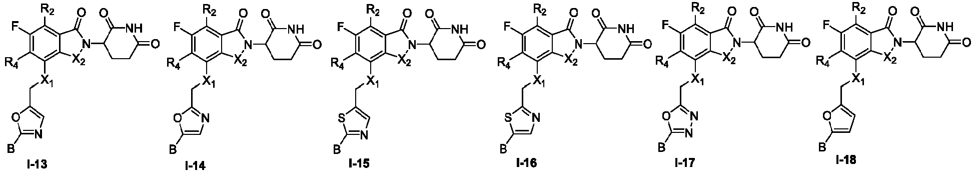 wherein X₁ is -CH₂-, -NH- or -O-;
X₂ is -CH₂- or -CO-;
R₂ and R₄ are each independently selected from hydrogen or deuterium;
B is as defined above.

**In a preferred embodiment,** the compound represented by formula (I) and the tautomer, enantiomer, diastereomer, racemate, metabolite, metabolic precursor, isotopic compound, pharmaceutically acceptable salt, ester, prodrug or hydrate thereof, wherein the compound represented by formula (I) is the compound represented by the general formulas (1-19) to (I-24): 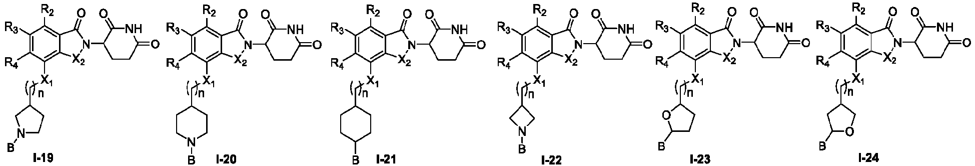 wherein X₁ is -CH₂-, -NH- or -O-;
X₂ is -CH₂- or -CO-;
R₂ and R₄ are each independently selected from hydrogen or deuterium;
R₃ is selected from hydrogen, deuterium or fluorine;
n is 1, 2, or 3;
B is as defined above.

**More preferably,** the compound represented by formula (I) and the tautomer, enantiomer, diastereomer, racemate, metabolite, metabolic precursor, isotopic compound, pharmaceutically acceptable salt, ester, prodrug or hydrate thereof, wherein the compound represented by formula (I) is selected from one of the following compounds:

| **Compound number** | **Compound structure** | **Compound number** | **Compound structure** |
|---|---|---|---|
| **1** | 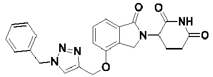 | **2** | 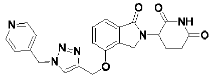 |
| **3** | 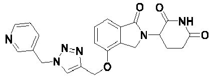 | **4** | 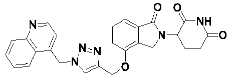 |
| **5** | 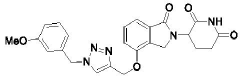 | **6** | 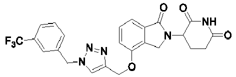 |
| **7** | 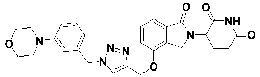 | **8** | 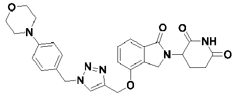 |
| **9** | 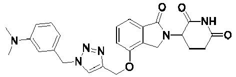 | **10** | 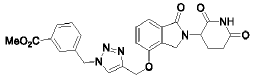 |
| **11** | 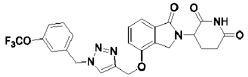 | **12** | 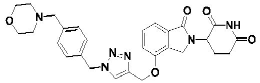 |
| **13** | 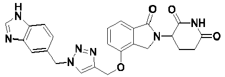 | **14** | 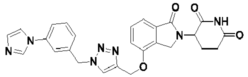 |
| **15** | 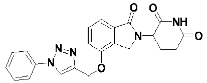 | **16** | 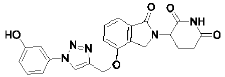 |
| **17** | 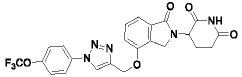 | **18** | 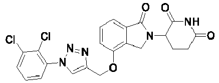 |
| **19** | 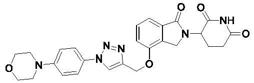 | **20** | 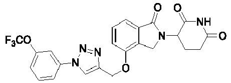 |
| **21** | 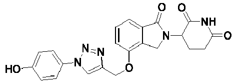 | **22** | 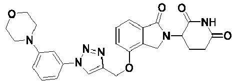 |
| **23** | 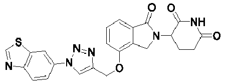 | **24** | 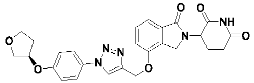 |
| **25** | 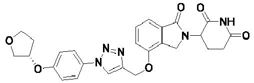 | **26** | 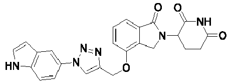 |
| **27** | 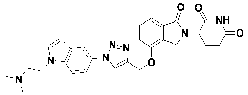 | **28** | 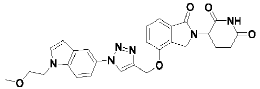 |
| **29** | 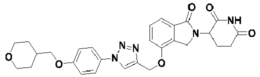 | **30** | 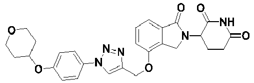 |
| **31** | 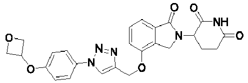 | **32** | 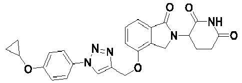 |
| **33** | 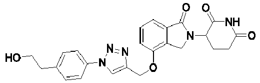 | **34** | 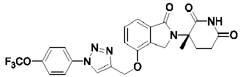 |
| **35** | 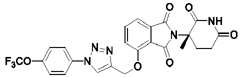 | **36** | 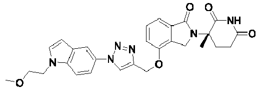 |
| **37** | 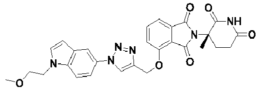 | **38** | 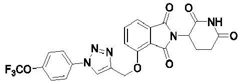 |
| **39** | 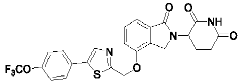 | **40** | 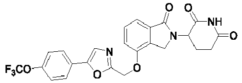 |
| **41** | 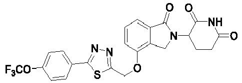 | **42** | 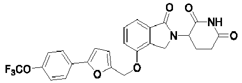 |
| **43** | 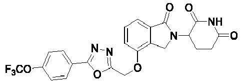 | **44** | 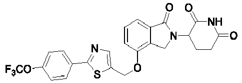 |
| **45** | 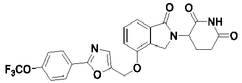 | **46** | 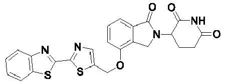 |
| **47** | 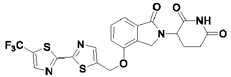 | **48** | 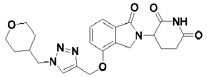 |
| **49** | 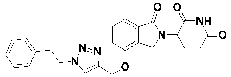 | **50** | 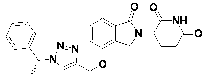 |
| **51** | 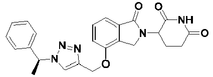 | **52** | 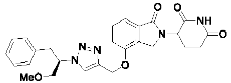 |
| **53** | 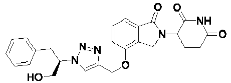 | **54** | 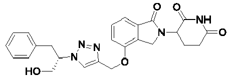 |
| **55** | 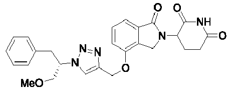 | **56** | 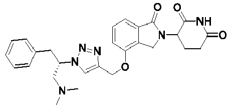 |
| **57** | 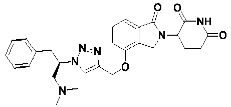 | **58** | 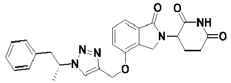 |
| **59** | 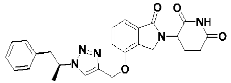 | **60** | 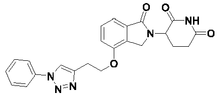 |
| **61** | 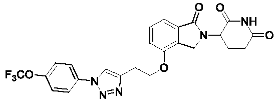 | **62** | 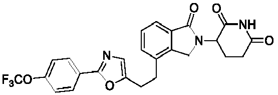 |
| **63** | 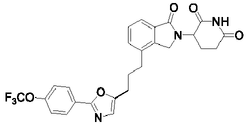 | **64** | 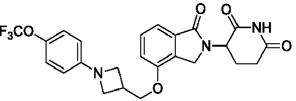 |
| **65** | 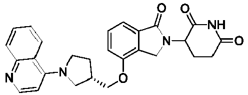 | **66** | 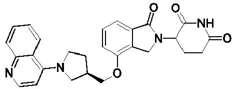 |
| **67** | 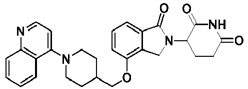 | **68** | 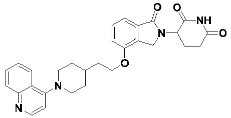 |
| **69** | 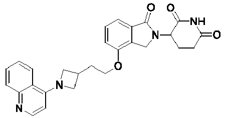 | **70** | 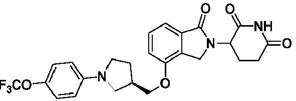 |
| **71** | 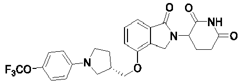 | **72** | 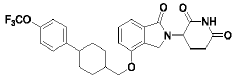 |
| **73** | 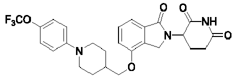 | **74** | 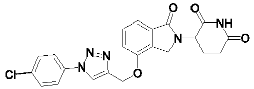 |
| **75** | 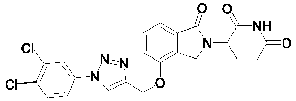 | **76** | 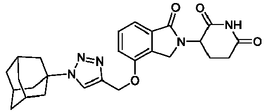 |
| **77** | 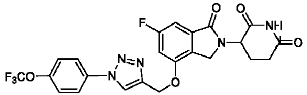 | **78** | 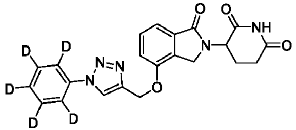 |
| **79** | 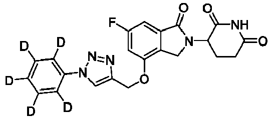 | **80** | 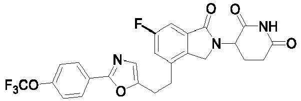 |
| **81** | 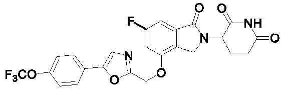 | **82** | 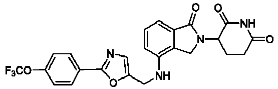 |
| **83** | 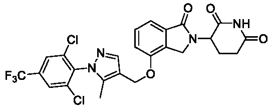 | **84** | 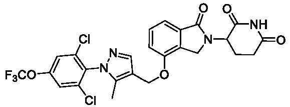 |
| **85** | 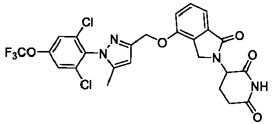 | **86** | 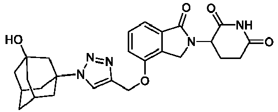 |
| **87** | 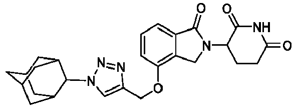 | **88** | 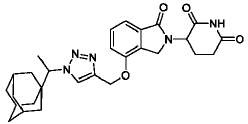 |
| **89** | 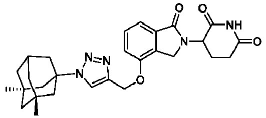 | **90** | 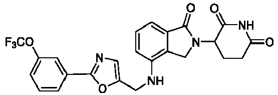 |
| **91** | 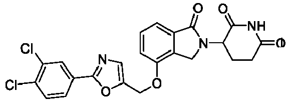 | **92** | 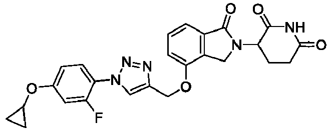 |
| **93** | 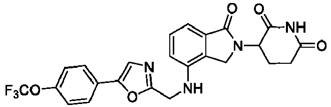 | **94** | 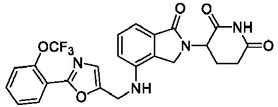 |

and the tautomer, enantiomer, diastereomer, racemate, metabolite, metabolic precursor, isotopic compound, pharmaceutically acceptable salt, ester, prodrug or hydrate thereof.

The content of the present invention also encompasses any of the novel intermediates disclosed herein.

A further aspect of the present invention provides a method for the preparation of a compound represented by formula (I), the method is selected from one of the following methods:
The synthetic for the initial compounds 1A and 2E in this application refers WO2008115516A2, WO2011100380A1, WO2016065980A1, WO2007027527A2, WO2008027542A2, the synthesis of intermediate compounds 1B and 2B refers to the examples in this application. 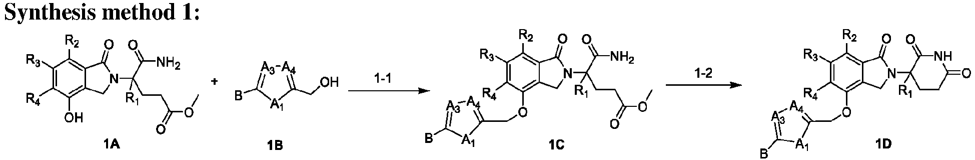 wherein R₁, R₂, R₃, R₄, A₁, A₃, A₄ and B have the same definitions as above;
step 1-1: compound 1A and 1B were reacted under triphenylphosphine and diisopropyl azodicarboxylate to obtain compound 1C;
step 1-2: compound 1C was reacted to obtain compound 1D in the presence of potassium tert-butoxide; 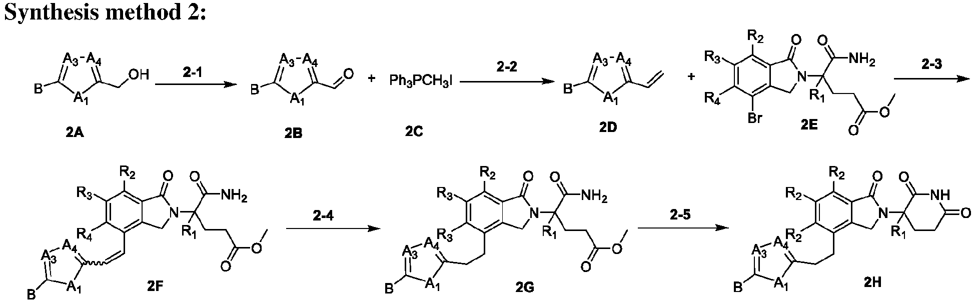
wherein R₁, R₂, R₃, R₄, A₁, A₃, A₄ and B have the same definitions as above;
Step 2-1: compound 2A was reacted to obtain compound 2B in the presence of manganese dioxide;
Step 2-2: compound 2B and compound 2C were reacted in the presence of potassium tert-butoxide in tetrahydrofuran to obtain compound 2D;
Step 2-3: compound 2D and compound 2E were reacted under the conditions of palladium catalyst (e.g., palladium acetate), a phosphine ligand (e.g., tris(2-methylphenyl)phosphine), and organic base (e.g., N, N-diisopropylethylamine) to obtain compound 2F;
Step 2-4: Compound 2F was reacted under palladium carbon and hydrogen at normal pressure to obtain compound 2G;
Step 2-5: compound 1C was reacted to obtain compound 2H in the presence of potassium tert-butoxide;

Another aspect of the present invention is to provide a use of the compound of formula (I), the tautomer, enantiomer, diastereomer, racemate, metabolite, metabolic precursor, isotopic compound, pharmaceutically acceptable salt, ester, prodrug, hydrate, crystalline hydrate, and solvate thereof for the manufacture of a medicament or a diagnostic reagent for the prevention or treatment of diseases related to CRL4^{CRBN}E3 ubiquitin ligase.

Another aspect of the present invention is to provide a use of the compound of formula (I), the tautomer, enantiomer, diastereomer, racemate, metabolite, metabolic precursor, isotopic compound, pharmaceutically acceptable salt, ester, prodrug, hydrate, crystalline hydrate, and solvate thereof for the manufacture of a medicament for the treatment or prevention the diseases, disorders or conditions that are produced by TNF-α or regulated by TNF-α activity, produced by IL-2 or regulated by IL-2 activity, produced by IFNγ or abnormally regulated by IFNγ activity.

Another aspect of the present invention is to provide a pharmaceutical composition comprising therapeutically effective doses of the compounds represented by formula (I) and the tautomer, enantiomer, diastereomer, racemate, metabolite, metabolic precursor, isotopic compound, pharmaceutically acceptable salt, ester, prodrug, hydrate, crystalline hydrate or solvate thereof, and other pharmaceutically acceptable carriers.

Another aspect of the present invention is to provide a pharmaceutical composition comprising therapeutically effective doses of the compounds represented by the formula (I) and the tautomer, enantiomer, diastereomer, racemate, metabolite, metabolic precursor, isotopic compound, pharmaceutically acceptable salt, ester, prodrug, hydrate, crystalline hydrate or solvate thereof, and one or more other ingredients with pharmaceutically therapeutic activity. In the present invention, the compound of formula (I) and tautomer, enantiomer, diastereomer, racemate, metabolite, metabolic precursor, isotopic compound, pharmaceutically acceptable salt, ester, prodrug, hydrate, crystalline hydrate or solvate thereof can be combined with one or more other ingredients with pharmaceutically therapeutic activity to produce synergistic effects in the prevention or treatment of specific diseases or dysfunctions. In the present invention, the compound of formula (I) and tautomer, enantiomer, diastereomer, racemate, metabolite, metabolic precursor, isotopic compound, pharmaceutically acceptable salt, ester, prodrug, hydrate, crystalline hydrate or solvate thereof can also reduce or eliminate the toxic and side effects of one or more other ingredients with pharmaceutically therapeutic activity in the prevention or treatment of specific diseases or dysfunctions, and vice versa.

Another aspect of the present invention is to provide a pharmaceutical composition, wherein the another one or more ingredients with pharmaceutically therapeutic activity as described above comprise macromolecular compound, such as protein, polysaccharide, nucleic acid, etc., and small molecular compound, such as inorganic compound, organometallic compound, synthetic or natural organic small molecule compound, etc.

Another aspect of the present invention is to provide a pharmaceutical composition, in the preferred embodiment, the pharmaceutical composition further comprises other therapeutic agents, and the other therapeutic agent is one or more of dexamethasone, rituximab, trastuzumab, PD-1 inhibitor, PDL-1 inhibitor, pemetrexed, topotecan, adriamycin, bortezomib, gemcitabine, dacarbazin, clarithromycin, vincristine, cytarabine, prednisone, docetaxel, clofarabine injection, HDAC inhibitor, androgen receptor inhibitor, androgen biosynthesis inhibitor, BTK inhibitor, erythrocyte growth hormone, minocycline, Elotuzumab, Palbociclib, Nivolumab, Pembrolizumab, Panobinostat, Ublituximab, Romidepsin, Eltrombopag, CAR-T and melphalan.

Another aspect of the present invention is to provide a use of the compound of formula (I) for the manufacture of a medicament for the treatment or prevention of diseases related to CRL4^{CRBN} E3 ubiquitin ligase, and the diseases include but are not limited to cancer, pain, nervous system diseases and immune system diseases. The disease, disorder or condition comprises: Myelodysplastic syndrome, Multiple myeloma, Mantle cell lymphoma, Non-Hodgkin's lymphoma, Chronic lymphocytic leukemia, Chronic myelomonocytic leukemia, Myelofibrosis, Burkitt's lymphoma, Hodgkin's lymphoma, Large cell lymphoma, Diffuse large B-cell lymphoma, Follicular lymphoma, Ciliary body and chronic melanoma, Melanoma of iris, Recurrent interocular melanoma, T-cell lymphoma, Erythroid lymphoma, monoblast and monocytic leukemia, Myeloid leukemia, Central nervous system lymphoma, Brain tumors, meningiomas, Spinal cord tumor, Thyroid cancer, Non-small cell lung cancer, Ovarian cancer, skin cancer, Renal cell carcinoma, Astrocytoma, Amyloidosis, type I complex local pain syndrome, malignant melanoma, radiculopathy, myelofibrosis, glioblastoma, gliosarcoma, malignant glioma, refractory plasmacytoma, extraocular extension melanoma, solid tumor, papillary and follicular thyroid cancer, breast cancer, prostate cancer, hepatocellular carcinoma or primary macroglobulinemia.

In another aspect of the present invention, a pharmaceutical composition is provided, it comprises a therapeutically effective amount of one or more of the compounds represented by formula (I) and the stereoisomers, pharmaceutically acceptable salts, prodrugs, solvates, hydrates and polymorphs thereof, and at least one excipient, diluent or carrier. A typical formulation is prepared by mixing the compound of formula (I) of the present invention with carrier, diluent or excipient. Suitable carriers, diluents or excipients are well known to those skilled in the art, including such as carbohydrates, waxes, water-soluble and / or swellable polymers, hydrophilic or hydrophobic substances, gelatin, oils, solvents, water and other substances. The specific carrier, diluent or excipient used will depend on the mode and purpose of the compound of the present invention. The solvent is generally selected on the basis of the solvent considered by those skilled in the art to be safe and effective for administration to mammals. Generally speaking, safe solvents are non-toxic aqueous solvents such as pharmaceutical water, and other non-toxic solvents that are soluble or miscible with water. Suitable aqueous solvents include one or more of water, ethanol, propylene glycol, polyethylene glycol (e.g.PEG400 or PEG300) and the like. The formulation may also include one or more of buffer, stabilizer, surfactant, wetting agent, lubricant, emulsifier, suspending agent, preservative, antioxidant, opacifier, glidant, processing aid, coloring agent, sweetening agent, spices, flavoring agent or other known additives, so that the drug can be manufactured or used in an acceptable form.

When the compound of formula (I) of the present invention is used in combination with at least one other drug, the two drugs or more drugs can be used separately or in combination, and are preferably administered in the form of pharmaceutical composition. The compound of formula (I) or pharmaceutical composition of the present invention can be administered in any known oral, intravenous, rectal, vaginal, transdermal, or other local or systemic administration form, separately or together administered to the subject.

These pharmaceutical compositions may also contain one or more of buffer, stabilizer, surfactant, wetting agent, lubricant, emulsifier, suspending agent, preservative, antioxidant, opalizer, glidant, processing aid, coloring agent, sweetening agent, spices, flavoring agent or other known additives, so that the pharmaceutical composition can be manufactured or used in an acceptable form.

The drug of the present invention is preferably administered by oral route. Solid-state formulations for oral administration may include capsules, tablets, powders, or pellets. In the solid-state formulation, the compound or pharmaceutical composition of the present invention is mixed with at least one inert excipient, diluent or carrier. Suitable excipients, diluents or carriers include substances such as sodium citrate or dicalcium phosphate, or starch, lactose, sucrose, mannose alcohol, silicic acid, etc.; binders such as carboxymethyl cellulose, alginate, gelatin, polyvinylpyrrolidone, sucrose, Arabic Gum, etc.; wetting agents such as glycerin, etc.; disintegrating agents such as agar, calcium carbonate, potato or tapioca starch, alginic acid, specific complexing silicate, sodium carbonate, etc.; solution blockers such as paraffin, etc.; absorption promoters such as quaternary ammonium compounds, etc.; adsorbents such as kaolin, bentonite, etc.; lubricants such as talc, calcium stearate, magnesium stearate, solid polyethylene glycol, sodium lauryl sulfate, etc. In the case of capsules and tablets, the formulation may also include buffer. Similar types of solid compositions can also be used as fillers for soft and hard filled gelatin capsules, where lactose and high molecular weight polyethylene glycol are used as excipients.

Liquid formulations for oral administration include pharmaceutically acceptable emulsions, solutions, suspensions, syrups and elixirs. In addition to the compound of the present invention or the composition thereof, the liquid formulations may contain an inert diluent commonly used in the art, such as water or other solvents; solubilizers and emulsifiers such as ethanol, isopropanol, ethyl carbonate, ethyl acetate, benzyl alcohol, benzyl benzoate, propylene glycol, 1,3-butanediol, dimethylformamide; oils (such as cottonseed oil, peanut oil, corn germ oil, olive oil, castor oil, sesame oil, etc.); glycerin; tetrahydrofurfuryl alcohol; fatty acid esters of polyethylene glycol and sorbitan; or a mixture of several of these substances, etc.

In addition to these inert diluents, the composition may also contain excipients, such as one or more of wetting agent, emulsifier, suspending agent, sweetening agent, flavoring agent and spices. In terms of suspension, in addition to the compound or composition of the present invention, it may further contain carrier such as suspending agent, such as ethoxylated stearyl alcohol, polyoxyethylene sorbitol, sorbitan ester, microcrystalline cellulose, aluminum metahydroxide, bentonite, agar and tragacanth, or a mixture of several of these substances.

The composition for rectal or vaginal administration is preferably suppository, which can be prepared by mixing the compound or composition of the present invention with suitable non-irritating excipient or carrier, such as cocoa butter, polyethylene glycol or suppository wax. The excipient or carrier is solid at normal room temperature and liquid at body temperature, and can be melt in the rectum or vagina to release the active compound.

The compound or pharmaceutical composition of the present invention can be administered in other topical formulations, including ointment, powder, spray and inhalant. The compound can be mixed under sterile conditions with pharmacically acceptable excipient, diluent or carrier and with any preservative, buffer or propellant as required. Ophthalmic formulation, ophthalmic ointment, powder and solution are also intended to be included within the scope of the present invention.

The present invention also provides a use of the compound of formula (I), and the tautomer, the enantiomer, diastereomer, racemate, metabolic precursor, metabolite, isotopic compound, pharmaceutically acceptable salt, ester, prodrug or hydrate, solvate or polymorph thereof as a selective regulator of CRL4^{CRBN}E3 ubiquitin ligase to regulate the activity of CUL4^{CRBN}E3 ubiquitin ligase.

The present invention also provides a use of the compound of formula (I) and the tautomer, enantiomer, diastereomer, racemate, metabolic precursor, metabolite, isotopic compound, pharmaceutically acceptable salt, ester, prodrug or hydrate, solvate or polymorph thereof for the manufacture of a medicament for the treatment or prevention of diseases related to CRL4^{CRBN}E3 ubiquitin ligase. The related diseases involved by CRL4^{CRBN}E3 ubiquitin ligase include (but are not limited to) tumors, central system diseases and immune diseases.

In a preferred embodiment, the present invention relates to a method for the treatment or prevention of a disease, disorder or condition associated with TNF- α production or regulation of TNF- α activity, IL-2 production or abnormal regulation of IL-2 activity, the method comprises administering to the subject a therapeutically or prophylactically effective amount of one or more of an isoindoline derivative of formula (I), the pharmaceutically acceptable salt, solvate, stereoisomer, isotopic compound, metabolite and prodrug thereof. According to the method of the invention, examples of such disease, disorder or condition to be treated or prevented include but are not limited to cancer (including solid tumor), TNF- α related disorder, undesirable angiogenesis-related diseases and conditions, pain, macular degeneration (MD)-related syndrome, skin diseases, keratosis, respiratory diseases (e.g., lung diseases), immunodeficiency diseases, central nervous system (CNS) diseases, autoimmune diseases, atherosclerosis, heredity, allergies, viruses, sleep disorders and related syndromes, inflammatory diseases, PDE-4-related diseases or IL-2-related diseases. Examples of such disease, disorder or condition well known in the art include but are not limited to those described in PCT patent publications WO2012015986 and WO2006018182 and U.S. patent publication US20100204227, some of which are incorporated herein by reference in their entirety.

The compound represented by formula (I) of the present invention, and the stereoisomer, pharmaceutically acceptable salt, prodrug, solvate, hydrate or polymorph thereof can be used in monotherapy or combination therapy. When used in combination therapy, it contains a therapeutically effective dose of the compound of formula (I) described in **claim** 1, the enantiomer, diastereomer, racemate and the mixture thereof, as well as the pharmaceutically acceptable sals, crystalline hydrate and solvate, as well as one or more ingredients with pharmaceutically therapeutic activity. The other one or more ingredients with pharmaceutically therapeutic activity, comprising macromolecular compound, such as protein (antibody or polypeptide), polysaccharide, nucleic acid (DNA or RNA), etc., and small molecular compound, such as inorganic compound, organometallic compound, synthetic or natural organic small molecule compound, etc. In addition, it also includes radiation, surgery, cell therapy, hormone therapy or cytokine therapy, etc.. The compound of formula (I) described in **claim** 1 of the present invention, the prodrug, enantiomer, diastereomer, racemate and mixture thereof, and the pharmaceutically acceptable salt, crystalline hydrate and solvate may be combined with one or more other ingredients with pharmaceutically therapeutic activity to produce synergistic effects in the prevention or treatment of specific diseases or dysfunctions. The compound of formula (I) described in **claim** 1 of the present invention, the prodrug, enantiomer, diastereomer, racemate and mixture thereof, and the pharmaceutically acceptable salt, crystalline hydrate and solvate may be combined with one or more other ingredients with pharmaceutically therapeutic activity to reduce or eliminate side effects produced in the prevention or treatment of specific diseases or dysfunctions, vice versa.

In another preferred embodiment, the disease or dysfunction includes but is not limited to cancer, angiogenesis-related diseases or dysfunction, pain (including but not limited to complex local pain syndrome), macular degeneration and related dysfunction, skin diseases, pulmonary dysfunction, immunodeficiency diseases, central nervous system damage and dysfunction, TNFα related diseases or dysfunctions.

In another preferred embodiment, the cancer includes (but is not limited to) skin cancer (such as melanoma), lymphatic system cancer, breast cancer, cervical cancer, uterine cancer, digestive tract cancer, lung cancer, ovarian cancer, prostate cancer, colon cancer, rectal cancer, oral cancer, brain tumor, head and neck cancer, throat cancer, testicular cancer, kidney cancer, pancreatic cancer, spleen cancer, liver cancer, bladder cancer, laryngeal cancer and cancers related to AIDS. The compound provided by the present invention is also effective against hematologic tumor and myeloma, such as can be used to treat multiple myeloma and acute and chronic leukemia. The compounds provided by the present invention can be used to prevent or treat primary tumors and metastatic tumors.

It should be understood that in the present invention, any of the technical features specifically described above and below (such as in the Example) can be combined with each other, thereby constituting new or preferred technical solutions. The foregoing description is not intended to limit aspects of the invention in any form.

The compound of formula (I) may contain one or more asymmetric or chiral centers, and therefore may exist in the form of different stereoisomers. The compound of the present invention includes all stereoisomeric forms including but not limited to diastereomer, enantiomer, atropisomer and the mixture thereof (such as racemates), metabolic precursor, metabolite, isotopic compound, pharmaceutically acceptable salt, ester, prodrug or hydrate thereof, and the compound of formula (I) can also exist in different tautomeric forms, which all are included in the scope of the present invention.

The term "substitution" refers to the substitution of one or more hydrogen atoms on a specific group by specific substituent. The specific substituents are those described in the preceding paragraph or those present in each example. Unless otherwise specified, an arbitrarily substitueted group may have a substituent selected from a specific group at any substitutable position of the group, and the substituent may be the same or different in each position. Cyclic substituents, such as heterocycloalkyl, can be attached to another ring, such as cycloalkyl, to form a spirobicyclic ring system, for example, two rings share one carbon atom.

Those skilled in the art should understand that the combinations of substituents contemplated by the present invention are those that are stable or chemically achievable. Substitution on the relevant structure in the present invention includes substituted and unsubstituted, for example, "optionally" substituted by a certain substituent, which includes the meaning of being substituted or unsubstituted by a certain substituent.

In the present invention, when the number of substituent is greater than 1, the substituents can be the same or different substituents, which means that when the number of substituent in a certain structure is more than one, the combination of substituents can be selected from multiple different types of substituents.

The term "substitution" can only apply to the site that can be substituted by substituent, and does not include substitution that cannot be achieved on the basis of existing chemical knowledge.

The term "tautomer" refers to the constitutional isomers with different energies that are mutually converted via a low energy barrier. The reaction generally results in the shift of hydrogen atoms or protons accompanying the conversion of single bonds and adjacent double bonds.

The term "enantiomer" refers to stereoisomers that are mirror images of each other and are not superimposable.

"Diastereomers" refer to stereoisomers that have two or more chiral centers and are not mirror images.

"Racemate" refers to two stereoisomers that are mirror images of each other, with opposite optical rotations, which neutralize optical rotations.

"Pharmaceutically acceptable salt" refers to the drug molecule forms a corresponding salt with the corresponding organic acid, inorganic acid or organic base or inorganic base, such as hydrochloric acid, formic acid, trifluoroacetic acid, succinic acid, methylsulfonic acid and the like.

"Hydrate" refers to a compound containing water.

As use herein, the term "metabolite" refers to an active substance produced by a change in the chemical structure of a drug molecule in vivo, generally a derivative of the aforementioned drug molecule, which may also be chemically modified.

As used herein and unless otherwise specified, the term "polymorph" refers to one or more crystal structures formed by different arrangements of molecules in the lattice space during crystallization.

As used herein, that term "solvate" refers to a crystalline form of a compound of formula (I), pharmaceutically acceptable salt, polymorph, stereoisomer, isotopic compound, metabolite, or prodrug thereof, and further comprises one or more solvent molecules incorporated into the crystalline structure. The solvate may include a stoichiometric amount or a non-stoichiometric amount of the solvent, and the solvent molecules in the solvent may exist in an ordered or non-ordered arrangement. A solvate contain non-stoichiometric amounts of solvent molecule may result from that loss of at least one (but not all) solvent molecule in the solvate. In a particular embodiment, the solvate is hydrate, meaning that the crystalline form of the compound further comprises water molecules which are used as solvent.

As used herein and unless otherwise specified, that term "prodrug" refer to a derivative of a compound comprising a bioreactive function such that, under biological conditions (in vitro or in vivo), the bioreactive function may cleave from the compound or otherwise react in other modes to provide the compound. Generally, the prodrug is inactive, or at least less active than the compound itself, so that its activity cannot be exerted until the compound is cleaved from the biological reaction function. The bioreactive function may be hydrolyzed or oxidized under biological conditions to provide the compound. For example, the prodrug may comprise a biohydrolyzable group. Examples of biohydrolyzable groups include but are not limited to biohydrolyzable phosphates, biohydrolyzable esters, biohydrolyzable amides, biohydrolyzable carbonates, biohydrolyzable carbamates, and biohydrolyzable ureides. For the review of prodrug, see, for example, J.Rautioetal., Nature Reviews Drug Discovery 2008, 7, 255-270 and Prodrugs: Challenges and Rewards (V. Stellaetal.ed., Springer, 2007).

The term "halogen" includes fluorine, chlorine, bromine or iodine.

The term "hydrocarbyl" refers to a substituent containing only carbon atoms and hydrogen atoms, and includes but not limited to methyl, ethyl, isopropyl, propyl, cyclohexyl, phenyl, etc.

The term "C1-C6 alkyl" refers to a straight or branched chain alkyl having from 1 to 6 carbon atoms, including but not limited to methyl, ethyl, propyl, isopropyl, butyl, isobutyl, sec-butyl, tert-butyl, pentyl and hexyl etc.

The term "cycloalkyl" refers to a saturated or partially unsaturated monocyclic or polycyclic cyclic hydrocarbon substituent. Monocyclic cycloalkyl includes but not limited to cyclopropyl, cyclobutyl, cyclopentenyl, and cyclohexyl. Polycyclic cycloalkyl includes spiro, fused, and bridged cycloalkyl. "Cycloalkyl" refers to cycloalkyl comprising substituted or unsubstituted. Non-limiting examples of cycloalkyl include:

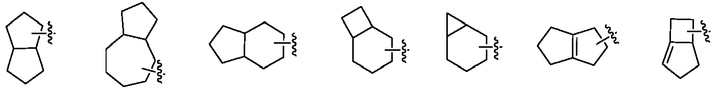

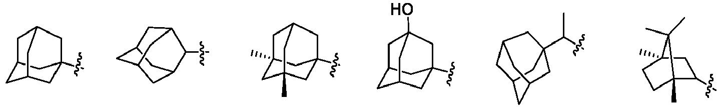

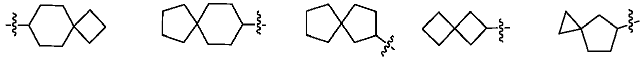

The term "aryl" refers to 6-14 membered all-carbon monocyclic or fused polycyclic group with conjugated p electron system, preferably 6 to 10 membered ring, more preferably phenyl and naphthyl, most preferably phenyl. The aryl ring may be fused to heteroaryl, heterocyclyl or cycloalkyl ring, and the ring attached to the core structure is aryl ring. The aryl group may be substituted or unsubstituted, and non-limiting examples include:

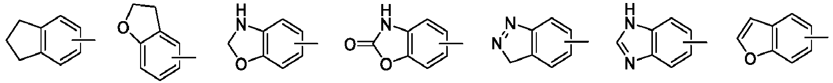

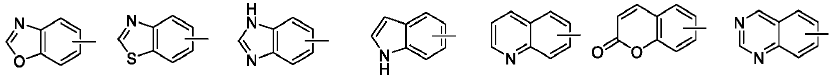

The term "heteroaryl" refers to 5-14 membered aryl having 1 to 4 heteroatoms as ring atoms, and the remaining ring atoms are carbon, wherein the heteroatoms include oxygen, sulfur and nitrogen. Preferably 5-10 membered ring. The heteroaryl is preferably 5 or 6 membered ring, such as thienyl, furyl, pyridyl, pyrrolyl, N-alkyl pyrrolyl, pyrimidyl, pyrazinyl, imidazolyl, tetrazyl, etc. The heteroaryl ring may be fused to aryl, heterocyclyl or cycloalkyl ring, and the ring attached to the core structure is heteroaryl ring. The aryl group may be substituted or unsubstituted, and non-limiting examples include:

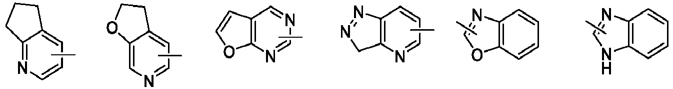

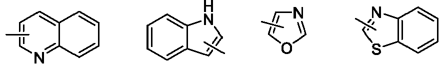

The term "heterocyclyl" refers to ring substituents containing one or more saturated and / or partially saturated monocyclic or polycyclic rings, which include 3 to 20 ring atoms, wherein one or more ring atoms are heteroatoms selected from nitrogen, oxygen, sulfur or S(O)m (wherein m is an integer from 0 to 2), and the remaining ring atoms are carbon. Preferably include 3 to 12 ring atoms, wherein 1-4 ring atoms are heteroatoms; such as epoxypropan, tetrahydrofuranyl, pyrrolidinyl, tetrahydropyranyl, piperidinyl, piperazinyl, morpholinyl, thiomorpholinyl. Polycyclic heterocyclyl includes spiro, fused, and bridged heterocyclyl.

The term "spiroheterocyclic group" refers to 5-20 membered polycyclicheterocyclyl that shares one atom between single rings (referred to spiro atom), in which one or more ring atoms are heteroatom selected from nitrogen, oxygen, sulfur or S(O)m (wherein m is an integer from 0 to 2), and the remaining ring atoms are carbon. Spiroheterocyclic ring can be fused with 6-10 membered aryl or 5-10 membered heteroaryl ring, wherein the ring attached to the core structure is spiroheterocyclic ring. Non-limiting examples of spiroheterocyclyl include:

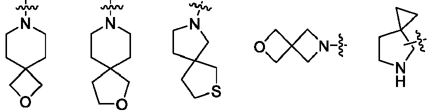

"Fused heterocyclyl" refers to 5-20 membered polycyclicheterocyclyl that each ring in the system shares an adjacent pair of atoms with other rings in the system, one or more rings may contain one or more double bonds, but none of the rings has a fully conjugated p-electron system, wherein one or more ring atoms are heteroatoms selected from nitrogen, oxygen, sulfur or S (O) m (wherein m is an integer from 0 to 2), and the remaining ring atoms are carbon. According to the number of constituent rings, it can be divided into bicyclic, tricyclic, tetracyclic or polycyclic fused heterocycloalkyl, and non-limiting examples of fused heterocyclyl include:

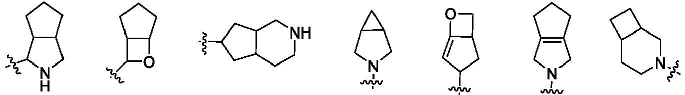

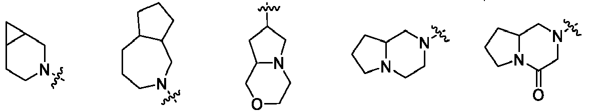

"Bridged heterocyclyl" refers to 5-14 membered polycyclicheterocyclyl that any two rings share two atoms that are not directly connected, and the rings may contain one or more double bonds, but none of the rings has a fully conjugated p-electron system, wherein one or more ring atoms are heteroatoms selected from nitrogen, oxygen, sulfur or S (O) m (wherein m is an integer from 0 to 2), and the remaining ring atoms are carbon. According to the number of constituent rings, it can be divided into bicyclic, tricyclic, tetracyclic or polycyclic bridged heterocyclyl.

The heterocyclic ring may be fused to aryl, heteroaryl, or cycloalkyl. The ring attached to the parent structure is a heterocyclyl, and non-limiting examples include:

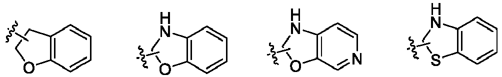

The term "C1-C6 alkoxyl" refers to a straight or branched chain alkoxyl having from 1 to 6 carbon atoms, including but not limited to methoxyl, ethoxyl, propoxyl, isopropoxyl and butoxyl, etc.

The term "C1-C6 alkoxycarbonyl" includes but not limited to methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentoxycarbonyl and hexoxycarbonyl, etc.

The term "haloalkyl" refers to a linear, branched or cyclic alkyl substituted by single or multiple halogens, and includes but not limited to 2-bromoethyl, 2-bromopropyl, etc.

The term "C2-C10 alkenyl" refers to alkenyl of 2-10 carbons, such as vinyl, propenyl, butenyl, styryl, phenpropenyl.

The term "C2-C10 alkynyl" refers to alkynyl of 2-10 carbons, such as ethynyl, propynyl, butynyl, phenylethynyl, phenylpropynyl.

The term "C3-C8 cycloalkyl" refers to a cyclic alkyl having 3 to 8 carbon atoms in the ring, and includes but not limited to cyclopropyl, cyclobutyl, cyclopentyl, and cyclohexyl, etc.

The term "5-10 membered heterocyclyl" means containing one or more saturated and / or partially saturated rings, which includes 5 to 10 ring atoms, of which one or more ring atoms are heteroatoms selected from nitrogen, oxygen, sulfur or S(O)m (wherein m is an integer from 0 to 2), and the remaining ring atoms are carbon; such as epoxypropane, tetrahydrofuranyl, pyrrolidinyl, tetrahydropyranyl, piperidinyl, piperazinyl, morpholinyl, thiomorpholinyl.

The term "C3-C6 heterocyclyl" refers to containing one or more saturated and / or partially saturated rings, which include 3 to 6 ring atoms, of which one or more ring atoms are heteroatoms selected from nitrogen, oxygen, sulfur or S(O)m (where m is an integer from 0 to 2), and the remaining ring atoms are carbon; such as epoxypropyl, tetrahydrofuranyl, pyrrolidinyl, tetrahydropyranyl, piperidinyl, piperazinyl

The term "hydroxy-substituted alkyl" refers to a linear, branched or cyclic alkyl substituted by single or multiple hydroxyls, including but not limited to (S)-1-hydroxyisobutyl-2-yl and (R)-1-hydroxyisobutyl-2-yl, etc.

As used herein, that term "pharmaceutically acceptable salt" refers to a pharmaceutically acceptable organic or inorganic salt. Exemplary salts include, but are not limited to: sulfate, hydrochloride, hydrobromide, hydrofluorates, phosphate, citrate, acetate, propionate, malonate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, Gentisinate, fumarate, gluconate, glucuronate, gluconate, formate, benzoate, lactate, malate, picrate, acidic amino acid (such as glutamate, aspartate, glutamate), methane sulfonate, ethane sulfonate, benzene sulfonate, p-toluenesulfonate and pamoate(i.e., 1-1-methylene-bis (2-hydroxy-3-naphthoate)). The compounds used in the present invention can form pharmaceutically acceptable salts with various amino acids. Suitable base salts include but are not limited to aluminum salt, calcium salt, lithium salt, magnesium salt, potassium salt, sodium salt, zinc salt, bismuth salt, and diethanolamine salt. A review of pharmaceutically acceptable salts can be found in the Hand book of Pharmaceutical Salts: Properties, Selection, and Use (P. Heinrich Stahland Camille G.Wermuth ed., Wiley-VCH, 2002).

The term "deuterium (D)" used in the present invention is a stable non-radioactive isotope of hydrogen with an atomic weight of 2.0144. Natural hydrogen is present as a mixture of H (hydrogen or protium), D (2H or deuterium) and T(3H or tritium) isotopes, with deuterium in an abundance of 0.0156%. According to the general technical knowledge of the field, in the structural formulas of all compounds containing natural hydrogen atoms, hydrogen atoms are actually a mixture of H, D, and T. Therefore, when the deuterium abundance at any site in a compound is greater than its natural abundance 0.0156%, these compounds should be considered unnatural or deuterium-enriched.

The term "isotopic compound" used in the present invention refers to the compound of formula (I) of the present invention, the pharmaceutically acceptable salt, solvate, stereoisomer, metabolite, or prodrug containing one or more atomic isotopes of natural or unnatural abundance. The present invention also covers isotopically-labeled compounds of the present invention, except for the fact that one or more atoms are replaced by the atom with atomic mass or the mass number different from the atomic mass or mass number common in nature. It is the same as the one mentioned here. Examples of isotopes that may be included in compounds of the present invention include the isotopes of hydrogen, carbon, nitrogen, oxygen, phosphorus, sulfur, fluorine, iodine and chlorine, such as: ²hydrogen, ³hydrogen, ¹¹carbon, ¹³carbon, ¹⁴carbon, ¹³nitrogen, ¹⁵nitrogen, ¹⁵oxygen, ¹⁷oxygen, ¹⁸oxygen, ³¹phosphorus, ³²phosphorus, ³⁵sulfur, ¹⁸ fluorine, ¹²³iodine, ¹²⁵iodine and ³⁶chlorine, respectively.

Certain isotopically labeled compounds of the present invention (such as those labeled with 3H and 14C) are used in compound and/or substrate tissue distribution tests. Tritium (3H) and carbon-14 (14C) isotopes are particularly preferred because they are easy to prepare and detect. Moreover, replacement with heavier isotopes such as deuterium (i.e. 2H) can provide some therapeutic advantages (for example, increased half-life in vivo or reduced dosage requirements) provided by greater metabolic stability, so it may be preferable in some cases. Positron emission isotopes, such as 150, 13N, 11C and 18F are used for positron emission tomography (PET) research to check substrate receptor occupancy rate. Isotopically-labeled compound of the present invention can generally be prepared by following methods similar to those disclosed in the scheme and/or the examples below, by substituting isotopically-labeled reagents for non-isotopically-labeled reagents. All isotopic variants of the compounds of the present invention, whether radioactive or not, are included within the scope of the present invention.

The positions of compounds that can be deuterated in the present invention can be deuterated at a plurality of different positions, and the positions of deuteration have the following forms, but are not limited to the following forms: 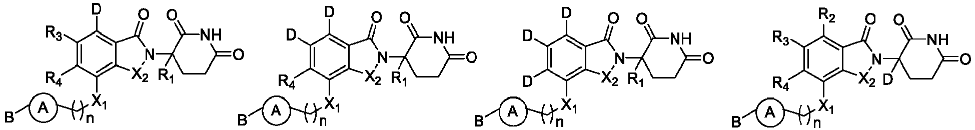 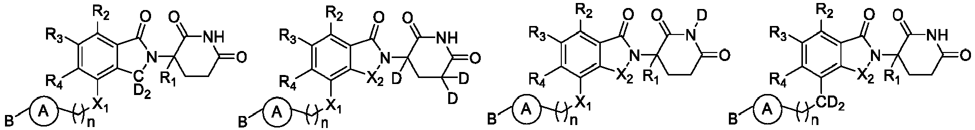 the position deuterated of the compound of formula (I) can also be selected from the positions of X1, X2, Ⓐ or B which can be deuterated at one or more different positions.

In the present invention, unless otherwise specified, the terms used have the general meanings known to those skilled in the art.

### DESCRIPTION OF FIGURES

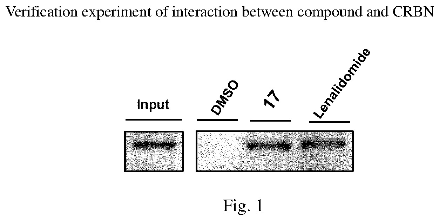

Fig.1 shows the experimental results of the interaction between the compound and CRBN.

### DETAILED DESCRIPTION OF THE INVENTION

### 1. Preparation Example

### Synthesis of Key Intermediates:

### Intermediate 1: methyl 2-methyl-3-(methoxymethoxy) benzoate

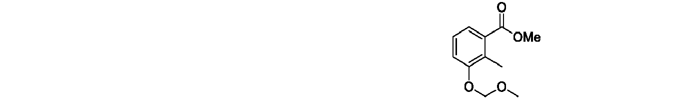

Methyl 2-methyl-3-hydroxybenzoate (10.0 g, 60.18 mmol) and N, N-diisopropylethylamine (20 mL, 120.36 mmol) were dissolved in 200 mL of dichloromethane, and bromomethyl methyl ether (7.4 mL, 90.27 mmol) was added dropwise under ice bath cooling condition. The obtained reaction solution was raised to room temperature and stirred at room temperature for 5 hours. After the reaction was completed, the reaction solution was diluted with dichloromethane, washed with water and saturated salt water in turn, dried over anhydrous magnesium sulfate, filtered, and concentrated under reduced pressure. The obtained oil was subjected to silica gel column chromatography to obtain methyl 2-methyl-3-(methoxymethoxy) benzoate 10.27 g, yield 81%; ¹H NMR (400 MHz, DMSO-d₆) δ 7.26 (s, 1H), 7.01 (s, 1H), 6.80 (d, *J =* 8.4 Hz, 1H), 3.58 (s, 3H), 2.30 (t, *J* = 8.0 Hz, 2H), 1.94 - 1.82 (m, 1H), 1.80 - 1.67 (m, 1H), 1.37 (s, 3H).

### Intermediate 2: methyl 2-bromomethyl-3-(methoxymethoxy) benzoate

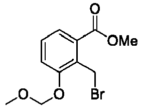

N-bromosuccinimide (8.96 g, 50.32 mmol) and 2, 2 '-dimethyl-2, 2'-azodipropionitrile (800 mg, 4.89 mmol) were added to a solution of methyl 2-methyl-3-(methoxymethoxy) benzoate (10.27 g, 48.85 mmol) in carbon tetrachloride (250 mL). The obtained reaction solution was refluxed at 88 °C for 3.5 hours, the solvent was removed under reduced pressure, and the obtained residue was subjected to silica gel column chromatography to obtain methyl 2-bromomethyl-3-(methoxymethoxy) benzoate 12.74 g, yield 90%;¹H NMR (400 MHz, CDCl₃) δ 7.57 (dd, *J* = 6.4, 2.6 Hz, 1H), 7.30 (dd, *J* = 9.6, 5.4 Hz, 2H), 5.29 (s, 2H), 5.07 (s, 2H), 3.92 (s, 3H), 3.52 (s, 3H).

### Intermediate 3: methyl 5-amino-4-(4-(methoxymethoxy)-1-oxoisoindolin-2-)-5-oxopentanoate

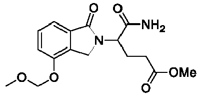

Methyl 2-bromomethyl-3-(methoxymethoxy) benzoate (6.0 g, 20.75 mmol) was dissolved in 255 mL of acetonitrile and methyl(S)-4, 5-diamino-5-oxopentanoate hydrochloride (4.49 g, 22.83 mmol) and N, N-diisopropylethylamine (7.2 mL, 43.58 mmol) were added in turn. The obtained reaction solution was first stirred at room temperature for 1 hour, and then transferred to 40° C and reacted for 21.5 hours. The acetonitrile was removed under reduced pressure, the obtained residue was dissolved in dichloromethane, the organic phase was washed with saturated ammonium chloride, dried over anhydrous sodium sulfate, filtered, and concentrated under reduced pressure. The obtained oil was washed with a mixed solution of hexane/ethyl acetate (5: 1), and dried under reduced pressure to give methyl 5-amino-4-(4-(methoxymethoxy)-1-oxoisoindolin-2-)-5-oxopentanoate(5.9 g, 84%); ¹H NMR (400 MHz, CDCl₃) δ 7.47 (d, *J* = 7.1 Hz, 1H), 7.41 (t, *J* = 7.7 Hz, 1H), 7.28 (s, 1H), 5.59 (s, 1H), 5.35 - 5.20 (m, 2H), 4.93 (dd, *J* = 8.9, 6.1 Hz, 1H), 4.45 (q, *J* = 17.5 Hz, 2H), 3.64 (s, 3H), 3.51 (s, 3H), 2.52 - 2.15 (m, 4H).

### Intermediate 4: methyl 5-amino-4-(4-hydroxy-1-oxoisoindolin-2-)-5-oxopentanoate

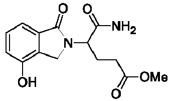

Methyl 5-amino-4-(4-(methoxymethoxy)-1-oxoisoindolin-2-)-5-oxopentanoate (3.35 g, 9.96 mmol) was dissolved in 5 mL anhydrous methanol, and saturated dioxane hydrochloride solution (45 mL) was added under the condition of stirring at room temperature. The obtained mixed solution continued to react at room temperature for 1 hour under stirring. After the reaction was completed, the solvent was removed under reduced pressure. The residue was subjected to silica gel column chromatography to obtain the target product methyl 5-amino-4-(4-hydroxy-1-oxoisoindolin-2-)-5-oxopentanoate2.48 g, yield 85%; ¹H NMR (400 MHz, DMSO) δ 10.04 (s, 1H), 7.57 (s, 1H), 7.31 (t, *J* = 7.7 Hz, 1H), 7.19 (dd, *J* = 34.5, 24.1 Hz, 2H), 6.99 (d, *J* = 7.9 Hz, 1H), 5.76 (s, 1H), 4.72 (dd, *J* = 10.4, 4.7 Hz, 1H), 4.48 (d, *J* = 17.4 Hz, 1H), 4.31 (d, *J* = 17.4 Hz, 1H), 3.50 (s, 3H), 2.33 - 2.12 (m, 3H), 2.12 - 1.96 (m, 1H).

### Intermediate 5: methyl 5-amino-4-(4-(2-propargyloxy)-1-oxoisoindolin-2-)-5-oxopentanoate

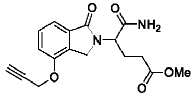

Methyl 5-amino-4-(4-hydroxy-1-oxoisoindolin-2-)-5-oxopentanoate(1.0 g, 3.42 mmol), propargyl alcohol (398µL, 6.84 mmol) and triphenylphosphine (1.79 g, 6.84 mmol) were dissolved in 30ml of dry tetrahydrofuran, DIAD (1.35 ml, 6.84 mmol) was added dropwise at 0°C, and reacted at room temperature for 2h. The solvent was removed under reduced pressure, and 1.06 g of methyl 5-amino-4-(4-(2-propargyloxy)-1-oxoisoindolin-2-)-5-oxopentanoatewas obtained by separation on flash column chromatography (dichloromethane/ethyl acetate=4: 1→dichloromethane/methanol=20: 1), yield 94%.¹H NMR (400 MHz, DMSO) δ 7.60 (s, 1H), 7.50 (t, *J* = 7.8 Hz, 1H), 7.31 (dd, *J* = 15.3, 7.6 Hz, 2H), 7.22 (s, 1H), 4.96 (d, *J* = 2.4 Hz, 2H), 4.72 (dd, *J* = 10.5, 4.7 Hz, 1H), 4.52 (d, *J* = 17.6 Hz, 1H), 4.39 (d, *J* = 17.6 Hz, 1H), 3.65 (t, *J* = 2.4 Hz, 1H), 3.51 (s, 3H), 2.30 - 2.15 (m, 3H), 2.13 - 2.02 (m, 1H).

### Intermediate 6:3-(1-oxo-4-(2-propargyloxy)isoindolin-2-)piperidine-2,6-dione

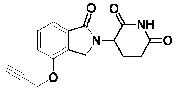

Methyl 5-amino-4-(4-(2-propargyloxy)-1-oxoisoindolin-2-)-5-oxopentanoate(997mg, 3.02 mmol) was cooled sufficiently at 0°C, potassium tert-butoxide (356mg, 3.17 mmol) was added in batches, after reacting at the same temperature for 15min, 350ul 1N HCl was added to quench, then 80ml ethyl acetate was added, washed with water and saturated sodium chloride solution successively, dried over anhydrous sodium sulfate, filtered, and the solvent was removed under reduced pressure to obtain 862mg of 3-(1-oxo-4-(2-propargyloxy) isoindoline-2-) piperidine-2, 6-dione, yield 96%. ¹H NMR (400 MHz, DMSO) δ 10.99 (s, 1H), 7.52 (t, *J* = 7.8 Hz, 1H), 7.36 (d, *J* = 7.5 Hz, 1H), 7.32 (d, *J* = 7.7 Hz, 1H), 5.11 (dd, *J* = 13.3, 5.1 Hz, 1H), 4.96 (d, *J* = 2.4 Hz, 2H), 4.40 (d, *J* = 17.5 Hz, 1H), 4.23 (d, *J* = 17.5 Hz, 1H), 3.64 (t, *J* = 2.4 Hz, 1H), 2.91 (ddd, *J* = 17.4, 13.6, 5.3 Hz, 1H), 2.62 - 2.54 (m, 1H), 2.48 - 2.38 (m, 1H), 2.04 - 1.94 (m, 1H).

### Intermediate 7: methyl 5-Amino-4-(4-(3-butyn-1-oxo)-1-oxoisoindolin-2-)-5-oxopentanoate

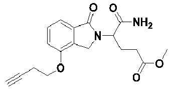

191mg of white solid was obtained, yield 54%;¹H NMR (400 MHz, DMSO) δ 7.63 (s, 1H), 7.46 (t, *J* = 7.8 Hz, 1H), 7.29 (d, *J* = 7.3 Hz, 1H), 7.24 (d, *J* = 8.3 Hz, 2H), 4.72 (dd, *J* = 10.4, 4.9 Hz, 1H), 4.51 (d, *J* = 17.6 Hz, 1H), 4.36 (d, *J* = 17.6 Hz, 1H), 4.20 (t, *J* = 6.3 Hz, 2H), 3.50 (s, 3H), 2.93 (t, *J* = 2.6 Hz, 1H), 2.69 (td, *J* = 6.4, 2.6 Hz, 2H), 2.29 - 2.13 (m, 3H), 2.11 - 1.99 (m, 1H).

### Intermediate 8: 3-(4-(3-butyn-1-oxy)-1-oxoisoindolin-2-)piperidine-2,6-dione

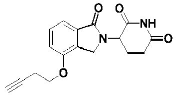

144mg of white solid was obtained, yield 78%;¹H NMR (400 MHz, DMSO) δ 10.99 (s, 1H), 7.49 (t, *J* = 7.8 Hz, 1H), 7.33 (d, *J* = 7.3 Hz, 1H), 7.27 (d, *J* = 8.1 Hz, 1H), 5.12 (dd, *J* = 13.3, 5.1 Hz, 1H), 4.37 (d, *J* = 17.4 Hz, 1H), 4.22 (dd, *J* = 12.0, 5.6 Hz, 3H), 2.97 - 2.83 (m, 2H), 2.62-2.54(m, 2H), 2.62 - 2.53 (m, 1H), 2.48 - 2.37 (m, 1H), 1.98 (dt, *J* = 10.2, 4.0 Hz, 1H).

### Intermediate 9:(S)-3-(4-hdrox-1-oxoisoindolin-2-)-3-methlieridine-2,6-dione

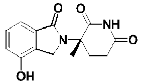

N, N-diisopropylethylamine (818ul, 4.95 mmol) was added to a suspension (20ml) of (S)-3-amino-3-methylpiperidine-2, 6-dione hydrobromide monohydrate (542mg, 2.25 mmol) and methyl 2-bromomethyl-3-methoxymethylbenzoate (651mg, 2.25 mmol) in acetonitrile, and the reaction system was heated to 60°C and reacted for 24h, concentrated under reduced pressure. 20ml acetic acid was added and refluxed for 24h, and then acetic acid was removed under reduced pressure, 399mg of (*S*)-3-(4-hydroxy-1-oxoisoindolin-2-)-3-methylpiperidine-2,6-dione was obtained by separation on flash column chromatography, yield 65%;¹H NMR (400 MHz, DMSO) δ 10.86 (s, 1H), 10.14 (s, 1H), 7.31 (t, *J* = 7.7 Hz, 1H), 7.09 (d, *J* = 7.3 Hz, 1H), 7.00 (d, *J* = 7.9 Hz, 1H), 4.59 (d, *J* = 17.4 Hz, 1H), 4.48 (d, *J* = 17.4 Hz, 1H), 2.78 - 2.52 (m, 3H), 1.94-1.82 (m, 1H), 1.68 (s, 3H).

### Intermediate 10: (S)-3-methyl-3-(1-oxo-4-(2-propyn-1-oxy) isoindolin-2-) piperidine-2, 6-dione

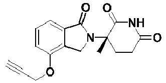

(*S*)-3-(4-hydroxy-1-oxoisoindolin-2-)-3-methylpiperidine-2,6-dione (100mg,0.365mmol) and triphenylphosphine (144mg, 0.548 mmol) were dissolved in 5ml of dry THF, propargyl alcohol (26ul, 0.438 mmol) was added, cooled sufficiently at 0°C, diisopropyl azodicarboxylate (108ul, 0.548 mmol) was added dropwise, then raised to room temperature to react for 2h, the solvent was removed under reduced pressure, and 85mg of white solid was obtained by separation on flash column chromatography, yield 75%;1HNMR (400 MHz, DMSO) δ 10.85 (s, 1H), 7.49 (t, J = 7.8 Hz, 1H), 7.29 (dd, J = 15.2, 7.8 Hz, 2H), 4.97 (d, J = 2.3 Hz, 2H), 4.67 (d, J = 17.6 Hz, 1H), 4.54 (d, J = 17.6 Hz, 1H), 3.64 (t, J = 2.3 Hz, 1H), 2.78-2.52(m, 3H), 1.89 (dt, J = 9.1,4.1 Hz, 1H), 1.69 (s, 3H).

### Intermediate 11: (S)-4-hydroxy-2-(3-methyl-2,6-dioxopiperidine-3-)isoindolin-1,3-dione

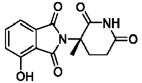

4-hydroxyisobenzofuran-1,3-dione (200mg, 1.22 mmol) and (S)-3-amino-3-methylpiperidine-2, 6-dione hydrobromide monohydrate (294mg, 1.22 mmol) were added to a 100ml round bottom flask, then dry toluene (20ml) was added, triethylamine (187ul, 1.34 mmol) was added under stirring. The reaction solution was heated to 120°C for water separation reaction for 48h (connected with water separator). After the reaction was completed, the solvent was removed under reduced pressure, the residue was diluted with ethyl acetate and washed with water and saturated sodium chloride solution in turn. The organic layer was dried over anhydrous sodium sulfate, filtered, concentrated under reduced pressure, and subjected to column chromatography to obtain 110mg of (S)-4-hydroxy-2-(3-methyl-2, 6-dioxopiperidine-3-) isoindoline-1, 3-dione as a white solid, yield 31%;¹H NMR (400 MHz, DMSO) δ 11.07 (s, 1H), 10.97 (s, 1H), 7.62 (dd, *J* = 8.3, 7.3 Hz, 1H), 7.22 (dd, *J* = 15.6, 7.7 Hz, 2H), 2.72 - 2.64 (m, 1H), 2.57-2.52 (m, 2H), 2.05 - 1.99 (m, 1H), 1.86 (s, 3H).

### Intermediate 12: (S)-2-(3-methyl-2, 6-dioxopiperidine-3-)-4-(2-propyn-1-oxy) isoindolin-1, 3-dione

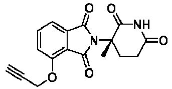

(S)-4-hydroxy-2-(3-methyl-2, 6-dioxopiperidine-3-) isoindoline-1, 3-dione (105mg, 0.364 mmol), propargyl alcohol (42ul, 0.73 mmol) and triphenylphosphine (191mg, 0.73 mmol) were dissolved in 15mL of dry tetrahydrofuran under nitrogen protection, the reaction solution was cooled with ice bath, then DIAD (144ul, 0.73 mmol) was added, the reaction solution was raised to room temperature for reaction after the addition. After the reaction was completed, the solvent was removed by concentrating under reduced pressure, and 98mg of white solid was obtained by column chromatography, yield 84%. ¹H NMR (400 MHz, DMSO) δ 10.98 (s, 1H), 7.85 - 7.80 (m, 1H), 7.52 (d, *J* = 8.5 Hz, 1H), 7.43 (d, *J* = 7.1 Hz, 1H), 5.04 (d, *J* = 2.3 Hz, 2H), 3.69 (t, *J* = 2.3 Hz, 1H), 2.75 - 2.61 (m, 1H), 2.57-2.52 (m, 3H), 2.07 - 1.97 (m, 1H), 1.87 (s, 3H).

### General Synthesis Methods of Azide Intermediates;

**Synthesis method 1 of azides:** Aromatic amine (1equiv.) was dissolved in the mixed solvent of water and concentrated hydrochloric acid (v/v=5: 1) under the condition of ice bath, sodium nitrite (1.3 equiv.) aqueous solution) was added dropwise, and the reaction solution was reacted under coolling and stirring for 15 minutes at 0°C, then sodium azide aqueous solution (1.2 equiv.) was added, the reaction solution was transferred to room temperature and reacted for 2 hours, after the reaction was completed, diluted and extracted with ethyl acetate, separated by silica gel column to obtain the corresponding aryl azide compounds.

**Synthesis method 2 of azides:** The alkyl bromide (1eqiv.) was dissolved in DMF, sodium azide (2 eqiv.) was added, and the reaction solution was raised to 80°C and reacted overnight. After the reaction was completed, the reaction solution was extracted with ethyl acetate and separated by silica gel column to obtain alkyl azide compounds.

**Synthesis method 3 of azides:** The alcohol derivative of the compound (1 equiv.) was dissolved in dry dichloromethane, triethylamine (2 equiv.), DMAP (0.1 equiv.) and 4-toluenesulfonyl chloride (1.1 equiv.) were added, reacted at room temperature for 2h, diluted with dichloromethane, and washed with water, saturated ammonium chloride and saturated NaCl in turn. The organic layer was dried over anhydrous sodium sulfate, filtered and concentrated under reduced pressure. The crude product was dissolved in DMF, sodium azide (1.2 eq) was added, and the temperature was raised to 80°C and reacted overnight. After the reaction was completed, diluted with ethyl acetate, washed with water and saturated NaCl in turn, dried over anhydrous sodium sulfate, filtered, and the solvent was removed under reduced pressure, the product was obtained by separation on flash column chromatography,

**Synthesis method 4 of azides:** The alcohol derivative of the compound (1equiv.) was dissolved in dry tetrahydrofuran, triphenylphosphine (2eqiv.) and diethyl azodicarboxylate (2eqiv.) were added, and cooled sufficiently at 0°C. Diphenyl azide phosphate (2eqiv.) was added under the protection of nitrogen, and the reaction was raised to room temperature for 2h. After the reaction was completed, the solvent was removed under reduced pressure, and the product was separated by silica gel column.

**Synthesis method 5 of azides:** Alcohol derivative (1eqiv.) was dissolved in tetrahydrofuran, diphenyl azidophosphate (1.5 eqiv.) and DBU (2eqiv.) were added, and heated and refluxed for 6h. The solvent was removed under reduced pressure, and the residue was diluted with ethyl acetate, washed with saturated sodium bicarbonate and saturated sodium chloride in turn, dried over anhydrous sodium sulfate, concentrated under reduced pressure, and the product was obtained by separation on flash column chromatography.

**Synthesis method 6 of azides:** (a) NaN₃ (9 eqiv.) was dissolved in 2mL of water, 3mL of dichloromethane was added, trifluoromethanesulfonic anhydride (1.8 eqiv.) was added dropwise under ice bath, reacted at the same temperature for 2h, extracted, dichloromethane (2x2 mL) was used for aqueous layer, combined the organic layers, washed the organic layer with saturated sodium carbonate solution, extracted, and directly used in the next step. (b) The alkylamine derivative was dissolved in lOmL of methanol and 2mL of water, copper sulfate pentahydrate (0.02 eqiv.) and anhydrous potassium carbonate (1eqiv.) were added, dichloromethane solution of the product of the first step was added dropwise under stirring, stirred at room temperature overnight, extracted with dichloromethane, the aqueous layer was neutralized with 1N HCl, extracted with dichloromethane once, combined the organic layers, dried over anhydrous magnesium sulfate, and spin-dried to give the product.

### Synthesis of Examples:

### Synthetic route 1:

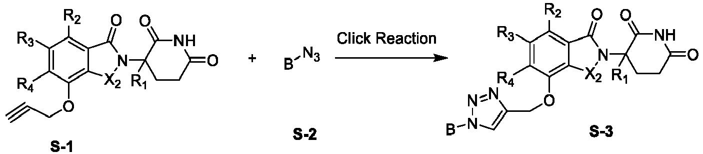

Wherein the definitions of R₁, R₂, R₃, R₄, X₂ and B are the same as above, S-1 is the above intermediate and S-2 is the above azide intermediate. The reaction conditions are shown in the following specific examples.

### EXAMPLE 1: 3-(4-(1-benzyl-1H-1,2,3-triazol-4-(methoxy)-1-oxoisoindolin-2-)piperidine-2, 6-dione (1)

Benzyl azide and intermediate 6 were used as raw materials through synthesis route 1, the preparation method was as follow:

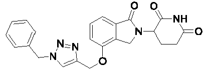

3-(1-oxo-4-(2-propargyloxy)isoindolin-2-) piperidine-2, 6-dione (intermediate 6, 40 mg, 0.134 mmol, 1equiv.), benzylazide (27mg, 0.201 mmol, 1.5 equiv.), and copper sulfate pentahydrate (6.7 mg, 0.0268 mmol, 0.2 equiv.) were dissolved in a mixed solution of dimethyl sulfoxide and water (v/v=4: 1, 5ml), diisopropylethylamine (22µ L, 0.134 mmol, 1equiv.) was added to the reaction solution, and sodium ascorbate(13mg, 0.067 mmol, 0.5 eq) was added after the reaction solution was uniformly mixed, the reaction was continued under stirring for 1 minute, tris [(1-benzyl-1H-1,2, 3-triazol-4-yl) methyl] amine (TBTA, 7mg, 0.0134 mmol) was added to the reaction solution, and the obtained reaction solution was stirred at room temperature for 30 minutes. After the reaction was completed, water and a copper ion adsorbent (CupriSorb) were added to the reaction mixture, the reaction mixture was extracted with ethyl acetate, the organic phase was washed with saturated ammonium chloride and saturated sodium chloride solutions, dried over anhydrous sodium sulfate, filtered, and dried under reduced pressure, and the crude product obtained was separated by HPLC to give 34 mg of pure 3-(4-(1-benzyl-1*H*-1,2, 3-triazol-4-(methoxy)-1-oxoisoindolin-2-)piperidine-2, 6-dione as a white solid, yield 59%; ¹H NMR (400 MHz, DMSO) δ 10.97 (s, 1H), 8.33 (s, 1H), 7.54 -7.47 (m, 1H), 7.44 (d, *J* = 8.0 Hz, 1H), 7.35 (dt, *J* = 15.5, 7.1 Hz, 6H), 5.61 (s, 2H), 5.29 (s, 2H), 5.09 (dd, *J* = 13.3, 5.0 Hz, 1H), 4.33 (d, *J* = 17.5 Hz, 1H), 4.17 (d, *J* = 17.4 Hz, 1H), 2.96 - 2.82 (m, 1H), 2.56 (d, *J* = 15.9 Hz, 1H), 2.41 (dt, *J* = 13.2, 11.2 Hz, 2H), 1.96 (dd, *J* = 13.7, 6.9 Hz, 1H). UPLC-MS (ESI) calculated for C₂₃H₂₁N₅O₄ [M+H]⁺: 432.16, found 432.30.

### EXAMPLE 2: 3-(1-oxo-4-(1-(pyridin-4-methyl)-1H-1, 2, 3-triazol-4-(methoxy) isoindolin-2-) piperidine-2, 6-dione (2)

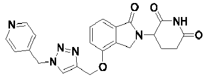

Pyridin-4-methylazide and intermediate 6 were used as raw materials, the preparation method was the same as that of synthetic route 1 and Example 1 to obtain 23.7 mg of 3-(1-oxo-4-(1-(pyridin-4-niethyl)-1*H*-1, 2, 3-triazol-4-(methoxy) isoindolin-2-) piperidine-2, 6-dione, yield 12%; ¹H NMR (400 MHz, DMSO) δ 10.96 (s, 1H), 8.56 (d, *J* = 5.7 Hz, 2H), 8.38 (s, 1H), 7.55 -7.48 (m, 1H), 7.45 (d, *J* = 7.9 Hz, 1H), 7.34 (d, *J* = 7.2 Hz, 1H), 7.19 (d, *J* = 5.7 Hz, 2H), 5.70 (s, 2H), 5.33 (s, 2H), 5.10 (dd, *J* = 13.3, 5.1 Hz, 1H), 4.35 (d, *J* = 17.5 Hz, 1H), 4.19 (d, *J* = 17.4 Hz, 1H), 2.90 (ddd, *J* = 17.4, 13.8, 5.4 Hz, 1H), 2.58 (d, *J* = 2.3 Hz, 1H), 2.47 - 2.34 (m, 1H), 2.01 - 1.88 (m, 1H). UPLC-MS (ESI) calculated for C₂₂H₂₀N₆O₄ [M + H]⁺: 433.16, found 433.30.

### EXAMPLE 3: 3-(1-oxo-4-(1-(pyridin-3-methyl)-1H-1, 2, 3-triazol-4-)methoxy) isoindolin-2-) piperidine-2, 6-dione (3)

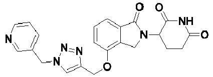

Pyridin-3-methylazide and intermediate 6 were used as raw materials, the preparation method was the same as Example 1 to obtain 29.2 mg of 3-(1-oxo-4-(1-(pyridin-3-methyl)-1*H*-1, 2, 3-triazol-4-)methoxy) isoindolin-2-) piperidine-2, 6-dione, yield 17 %; ¹H NMR (400 MHz, DMSO) δ 10.96 (s, 1H), 8.61 (d, *J* = 1.7 Hz, 1H), 8.55 (dd, *J* = 4.7, 1.2 Hz, 1H), 8.38 (s, 1H), 7.73 (dt, *J* = 7.7, 1.7 Hz, 1H), 7.53 -7.47 (m, 1H), 7.43 (d, *J* = 8.8 Hz, 1H), 7.42 -7.38 (m, 1H), 7.34 (d, *J* = 7.2 Hz, 1H), 5.68 (s, 2H), 5.30 (s, 2H), 5.09 (dd, *J* = 13.3, 5.1 Hz, 1H), 4.34 (d, *J* = 17.4 Hz, 1H), 4.18 (d, *J* = 17.4 Hz, 1H), 2.90 (ddd, *J* = 17.7, 13.7, 5.4 Hz, 1H), 2.59 (s, 1H), 2.41 (qd, *J* = 13.3, 4.4 Hz, 1H), 2.01 - 1.91 (m, 1H). UPLC-MS (ESI) calculated for C₂₂H₂₀N₆O₄ [M + H]⁺: 433.15, found 433.30.

### EXAMPLE 4: 3-(1-oxo-4-(1-(quinolin-4-methyl)-1H-1, 2, 3-triazol-4-(methoxy) isoindolin-2-) piperidine-2, 6-dione (4)

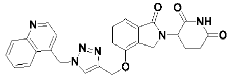

Quinolin-4-methylazide and intermediate 6 were used as raw materials, the preparation method was the same as that of synthetic route 1 and Example 1 to obtain 10.1 mg of 3-(1-oxo-4-(1-(quinolin-4-methyl)-1*H*-1, 2, 3-triazol-4-)methoxy) isoindolin-2-) piperidine-2, 6-dione, yield 14 %; ¹H NMR (400 MHz, DMSO) δ 10.96 (s, 1H), 8.87 (d, *J* = 4.4 Hz, 1H), 8.40 (s, 1H), 8.24 (d, *J* = 8.3 Hz, 1H), 8.08 (d, *J* = 8.3 Hz, 1H), 7.86 - 7.77 (m, 1H), 7.74 - 7.65 (m, 1H), 7.53 -7.47 (m, 1H), 7.44 (d, *J* = 7.9 Hz, 1H), 7.34 (d, *J* = 7.3 Hz, 1H), 7.06 (d, *J* = 4.4 Hz, 1H), 6.22 (s, 2H), 5.75 (s, 1H), 5.33 (s, 2H), 5.09 (dd, *J* = 13.3, 5.1 Hz, 1H), 4.34 (d, *J* = 17.4 Hz, 1H), 4.18 (d, *J* = 17.4 Hz, 1H), 2.96 - 2.83 (m, 1H), 2.58 (d, *J* = 2.1 Hz, 1H), 2.40 (ddd, *J* = 26.2, 13.1, 4.4 Hz, 1H), 1.96 (dt, *J* = 10.2, 3.1 Hz, 1H). UPLC-MS (ESI) calculated for C₂₆H₂₂N₆O₄ [M + H]⁺: 483.17, found 483.35.

### EXAMPLE 5: 3-(4-(1-(3-methoxybenzyl)-1H-1, 2, 3-triazol-4-)methoxy)-1-isoindolin-2-) piperidine-2, 6-dione (5)

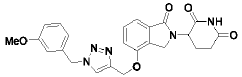

Step 1: 223mg of 3-methoxybenzyl azide was obtained as a colorless oil with a yield of 92% according to above method for preparation of azide compounds; ¹H NMR (400 MHz, CDCl₃) δ 7.30 (t, *J* = 7.8 Hz, 1H), 6.93 - 6.84 (m, 3H), 4.32 (s, 2H), 3.83 (s, 3H).

Step 2: 3- methoxybenzyl azide and intermediate 6 were used as raw materials, the preparation method was the same as that of synthetic route 1 and Example 1 to obtain 19.5 mg of 3-(4-(1-(3-niethoxybenzyl)-1H-1, 2, 3-triazol-4-)methoxy)-1-isoindolin-2-) piperidine-2, 6-dione, yield 46%; ¹H NMR (400 MHz, DMSO) δ 10.98 (s, 1H), 8.33 (s, 1H), 7.53 -7.47 (m, 1H), 7.44 (d, *J* = 7.7 Hz, 1H), 7.33 (d, *J* = 7.2 Hz, 1H), 7.31 -7.25 (m, 1H), 6.92 - 6.88 (m, 2H), 6.86 (d, *J* = 7.6 Hz, 1H), 5.57 (s, 2H), 5.30 (s, 2H), 5.09 (dd, *J* = 13.3, 5.0 Hz, 1H), 4.33 (d, *J* = 17.5 Hz, 1H), 4.17 (d, *J* = 17.5 Hz, 1H), 3.72 (s, 3H), 2.90 (ddd, *J* = 17.5, 13.8, 5.4 Hz, 1H), 2.61 - 2.52 (m, 1H), 2.41 (ddd, *J* = 17.6, 13.3, 5.0 Hz, 1H), 2.02-1.93 (ddd, *J* = 11.1, 8.4, 5.9 Hz, 1H). UPLC-MS (ESI) calculated for C₂₄H₂₃N₅O₅ [M + H]⁺: 462.17, found 462.38.

### EXAMPLE 6: 3-(1-oxo-4-((1-(3-(trifluoromethyl)benzyl)-1H-1, 2, 3-triazol-4-)methoxy) isoindolin-2-) piperidine-2, 6-dione (6)

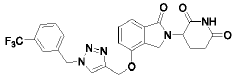

Step 1: azide was prepared as the preparation method 2 of azides, to obtain 180.4 mg of 3-(trifluoromethyl) benzyl azide as a colorless oil, yield 71%; ¹H NMR (400 MHz, CDCl₃) δ 7.60 (dd, *J* = 8.9, 3.6 Hz, 2H), 7.52 (d, *J* = 5.3 Hz, 2H), 4.44 (s, 2H).

Step 2: 3-trifluoromethyl benzyl azide and intermediate 6 were used as raw materials, the preparation method was the same as that of synthetic route 1 and Example 1 to obtain 4.3 mg of 3-(1-oxo-4-((1-(3-(trifluoromethyl)benzyl)-1*H*-1, 2, 3-triazol-4-)methoxy) isoindolin-2-) piperidine-2, 6-dione, yield 9 %; ¹H NMR (400 MHz, DMSO) δ 10.97 (s, 1H), 8.39 (s, 1H), 7.72 (d, *J* = 7.0 Hz, 2H), 7.62 (q, *J* = 8.1 Hz, 2H), 7.52 - 7.47 (m, 1H), 7.43 (d, *J* = 7.6 Hz, 1H), 7.34 (d, *J* = 7.1 Hz, 1H), 5.74 (s, 2H), 5.31 (s, 2H), 5.10 (dd, *J* = 13.4, 5.1 Hz, 1H), 4.34 (d, *J* = 17.4 Hz, 1H), 4.18 (d, *J* = 17.4 Hz, 1H), 2.90 (ddd, *J* = 17.2, 13.8, 5.4 Hz, 1H), 2.60 - 2.53 (m, 1H), 2.40 (ddd, *J* = 17.6, 13.5, 4.7 Hz, 1H), 2.01 - 1.91 (m, 1H). UPLC-MS (ESI) calculated for C₂₄H₂₀F₃N₅O₄ [M+H]+: 500.15, found 500.38.

### EXAMPLE 7: 3-(4-((1-(3-morpholinbenzyl)-1H-1, 2, 3-triazol-4-) methoxy)-1-oxoisoindolin-2-) piperidine-2, 6-dione (7)

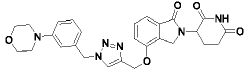

Step 1: m-Bromobenzyl alcohol (2.5 g, 13.37 mmol) and imidazole (1.82 g, 26.74 mmol) were dissolved in 25ml DMF, tert-butyldimethylchlorosilane (3.02 g, 20.05 mmol) was added under cooling at 0°C, the reaction was raised to room temperature overnight, diluted with ethyl acetate, washed with water and saturated sodium chloride solution in turn, dried over anhydrous sodium sulfate, filtered, the solvent was removed under reduced pressure, and 3.89 g of colorless oil was obtained by silica gel column chromatography with a yield of 96.5%;¹H NMR (400 MHz, CDCl₃) δ 7.48 (s, 1H), 7.37 (d, *J* = 7.6 Hz, 1H), 7.21 (dt, *J* = 15.3, 7.6 Hz, 2H), 4.71 (s, 2H), 0.95 (s, 9H), 0.11 (s, 6H).

Step 2: 3-bromobenzyloxydimethyl tert-butylsilyl ether (2g, 6.64 mmol), morpholine (1.65 ml, 18.98 mmol), Pd2(dba)3 (61mg, 0.067 mmol), (+)-BINAP (108mg, 0.17 mmol), sodium tert-butoxide (1.28 g, 13.28 mmol) were added into a 100 mL two-mouth bottle, 20ml of toluene was added, replaced with nitrogen 3 times, and that reaction was refluxed overnight under nitrogen protection. After the reaction was completed, filtered with diatomite, the filtrate was diluted with ethyl acetate, the organic phase was washed with saturated sodium bicarbonate and saturated sodium chloride in turn, dried over anhydrous sodium sulfate, filtered, the solvent was removed under reduced pressure, and the crude product was directly used in the next step. The crude product was dissolved in 20mL of tetrahydrofuran, tetrabutylammonium fluoride (1M/L tetrahydrofuran solution, 10.8 mL) was added, reacted for 1h at room temperature, the solvent was removed under reduced pressure, dissolved with ethyl acetate, and the organic phase was washed with saturated sodium bicarbonate and saturated sodium chloride in turn, dried over anhydrous sodium sulfate, filtered, concentrated under reduced pressure, and subjected to silica gel column chromatography (PE: EA=3: 1 to 1: 1) to obtain 1g of 3-hydroxymethylphenylmorpholine as a yellow solid, the total yield of the two steps was 79%;¹H NMR (400 MHz, CDCl₃) δ 7.26 (d, *J* = 15.7 Hz, 1H), 6.93 (s, 1H), 6.85 (t, *J* = 7.8 Hz, 2H), 4.65 (s, 2H), 3.92 - 3.79 (t,*J* = 4.7 Hz,4H), 3.25 - 3.08 (t, *J* = 4.7 Hz,4H).

Step 3: 3-hydroxymethylphenylmorpholine (0.2 g, 1.036 mmol) was dissolved in 10ml of dry tetrahydrofuran, and triphenylphosphine (543 mg, 2.07 mmol) and diethyl azodicarboxylate (326 µL, 2.07 mmol) were added and cooled sufficiently at 0 °C, diphenyl azidophosphate (446µL, 2.071 mmol) was added under nitrogen protection, raised to room temperature and reacted for 2h. Concentrated under reduced pressure, and 176 mg of 4-(3-azidomethylphenyl) morpholine was obtained as a colorless oil by separation on flash column chromatography, yield 78%;¹H NMR (400 MHz, CDCl₃) δ 7.29 (d, *J* = 7.8 Hz, 1H), 6.88 (dd, m, 1H), 6.83 (m, 2H), 4.30 (s, 2H), 3.89 - 3.84 (t, *J* = 4.8 Hz, 4H), 3.20 - 3.15 (t, *J* = 4.8 Hz ,4H).

Step 4: 4-(3-azidomethylphenyl) morpholine and intermediate 6 were used as raw materials, the preparation method was the same as that of synthetic route 1 and Example 1 and 17.5 mg of 3-(4-((1-(3-morpholinbenzyl)-1H-1, 2, 3-triazol-4-)methoxy)-1-oxoisoindolin-2-) piperidine-2, 6-dione was obtained as a white solid, yield 35 %; ¹H NMR (400 MHz, DMSO) δ 10.96 (s, 1H), 8.32 (s, 1H), 7.52 - 7.46 (m, 1H), 7.43 (d, *J* = 7.6 Hz, 1H), 7.33 (d, *J* = 7.2 Hz, 1H), 7.20 (t, *J* = 7.9 Hz, 1H), 6.94 (s, 1H), 6.89 (dd, *J* = 8.3, 2.1 Hz, 1H), 6.72 (d, *J* = 7.6 Hz, 1H), 5.52 (s, 2H), 5.29 (s, 2H), 5.09 (dd, *J* = 13.3, 5.1 Hz, 1H), 4.33 (d, *J* = 17.5 Hz, 1H), 4.17 (d, *J* = 17.5 Hz, 1H), 3.74 - 3.68 (t,*J* = 4.7 Hz, 4H), 3.09 - 3.04 (t,*J* = 4.7 Hz, 4H), 2.90 (ddd, *J* = 17.4, 13.6, 5.3 Hz, 1H), 2.59 - 2.53 (m, 1H), 2.40 (qd, *J* = 13.2, 4.3 Hz, 1H), 2.00 - 1.91 (m, 1H). UPLC-MS (ESI) calculated for C₂₇H₂₈N₆O₅ [M+H]⁺: 517.21, found 517.44.

### EXAMPLE 8: 3-(4-((1-(4-morpholinbenzyl)-1H-1, 2, 3-triazol-4-) methoxy)-1-oxoisoindolin-2-) piperidine-2, 6-dione (8)

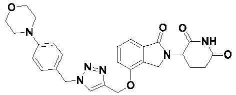

Step 1: The preparation method of 4-(4-azidomethylphenyl) morpholine was the same as that of 4-(3-azidomethylphenyl) morpholine, and 74mg of 4-(4-azidomethylphenyl) morpholine was obtained as a colorless oil, yield 33%; 1H NMR (400 MHz, CDC13) δ 7.23 (d, J = 8.6 Hz, 2H), 6.91 (d, J = 8.6 Hz, 2H), 4.25 (s, 2H), 3.89 - 3.83 (t, J = 4.8 Hz,4H), 3.21 - 3.15 (t, J = 4.8 Hz,4H).

Step 2: 4-(4-azidomethylphenyl) morpholine and intermediate 6 were used as raw materials, the preparation method was the same as that of synthetic route 1 and Example 1, and 25 mg of 3-(4-((1-(4-morpholinbenzyl)-1H-1, 2, 3-triazol-4-)methoxy)-1-oxoisoindolin-2-) piperidine-2, 6-dione as a white solid was obtained, yield 48 %; ¹H NMR (400 MHz, DMSO) δ 10.96 (s, 1H), 8.25 (s, 1H), 7.53 - 7.47 (m, 1H), 7.44 (d, *J* = 7.7 Hz, 1H), 7.33 (d, *J* = 7.2 Hz, 1H), 7.23 (d, *J* = 8.7 Hz, 2H), 6.92 (d, *J* = 8.7 Hz, 2H), 5.47 (s, 2H), 5.27 (s, 2H), 5.09 (dd, *J* = 13.4, 5.2 Hz, 1H), 4.32 (d, *J* = 17.4 Hz, 1H), 4.17 (d, *J* = 17.4 Hz, 1H), 3.77 -3.67 (m, 4H), 3.13 - 3.04 (m, 4H), 2.95 - 2.83 (m, 1H), 2.59 - 2.53 (m, 1H), 2.41 (ddd, *J* = 17.7, 13.5, 4.7 Hz, 1H), 1.98 - 1.90 (m, 1H). UPLC-MS (ESI) calculated for C₂₇H₂₈N₆O₅ [M+H]⁺: 517.21, found 517.44.

### EXAMPLE 9: 3-(4-((1-(3-dimethylamino) benzyl)-1H-1, 2, 3-triazol-4-(methoxy)-1-oxoisoindolin-2-)piperidine-2, 6-dione (9)

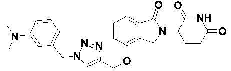

Step 1:154mg of 3-azidomethyl-N, N-dimethylaniline was obtained as a colorless oil with a yield of 74% according to above method for preparation method 5 of azides;1H NMR (400 MHz, CDC13) δ 7.40 (dd, J = 8.3, 7.5 Hz, 1H), 7.28 -7.23 (m, 1H), 6.71 (m, 1H), 6.66 (s, 1H), 4.29 (s, 2H), 2.97 (s, 6H).

Step 2:3-azidomethyl-N, N-dimethylaniline and intermediate 6 were used as raw materials, the preparation method was the same as that of synthetic route 1 and Example 1, and 11.3 mg of 3-(4-((1-(3-dimethylamino)benzyl)-1H-1, 2, 3-triazol-4-)methoxy)-1-oxoisoindolin-2-) piperidine-2, 6-dione was obtained as a white solid, yield 24 %; ¹H NMR (400 MHz, DMSO) δ 10.96 (s, 1H), 8.31 (s, 1H), 7.52 - 7.47 (m, 1H), 7.44 (d, *J* = 7.5 Hz, 1H), 7.33 (d, *J* = 7.2 Hz, 1H), 7.15 (t, *J* = 7.8 Hz, 1H), 6.70 - 6.64 (m, 2H), 6.55 (d, *J* = 7.4 Hz, 1H), 5.51 (s, 2H), 5.29 (s, 2H), 5.09 (dd, *J* = 13.4, 5.1 Hz, 1H), 4.33 (d, *J* = 17.5 Hz, 1H), 4.17 (d, *J* = 17.5 Hz, 1H), 2.95-2.88 (m, 1H), 2.86 (s, 6H), 2.57 (m, 1H), 2.40 (qd, *J* = 13.4, 4.5 Hz, 1H), 2.00 - 1.90 (m, 1H). UPLC-MS (ESI) calculated for C₂₅H₂₆N₆O₄ [M+H]⁺: 475.20, found 475.44.

### EXAMPLE 10: Methyl 3-((4-(((2-(2, 6-dioxopiperidin-3-)-1-oxoisoindolin-4-) oxo) methyl)-1H-1, 2, 3-triazol-1-)methyl) benzoate (10)

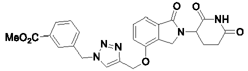

Step 1: 218.6mg of methyl 3- azide methyl benzoate was obtained as a colorless oil with a yield of 87% according to above method for preparation method 2 of azides;1H NMR (400 MHz, CDC13) δ 8.00 (d, J = 1.5 Hz, 2H), 7.49 (dt, J = 15.1, 7.6 Hz, 2H), 4.40 (s, 2H), 3.93 (s, 3H).

Step 2: methyl 3- azide methyl benzoate and intermediate 6 were used as raw materials, the preparation method was the same as that of synthetic route 1 and Example 1, and 22.8 mg of methyl 3-((4-(((2-(2, 6-dioxopiperidin-3-)-1-oxoisoindolin-4-) oxo) methyl)-1H-1, 2, 3-triazol-1-)methyl) benzoate was obtained as a white solid, yield 48 %; ¹H NMR (400 MHz, DMSO) δ 10.96 (s, 1H), 8.37 (s, 1H), 7.93 (d, *J* = 9.2 Hz, 2H), 7.61 (d, *J* = 7.8 Hz, 1H), 7.57-7.46 (m, 2H), 7.44 (d, *J* = 8.0 Hz, 1H), 7.34 (d, *J* = 7.2 Hz, 1H), 5.71 (s, 2H), 5.30 (s, 2H), 5.09 (dd, *J* = 13.3, 5.0 Hz, 1H), 4.34 (d, *J* = 17.4 Hz, 1H), 4.18 (d, *J* = 17.4 Hz, 1H), 3.85 (s, 3H), 2.96 - 2.84 (m, 1H), 2.59-2.54 (m, 1H), 2.41 (m, 1H), 2.01 - 1.91 (m, 1H). UPLC-MS (ESI) calculated for C₂₃H₂₈N₅O₆ [M+H]⁺: 490.16, found 490.40.

### EXAMPLE 11: 3-(1-oxo-4-((1-(3-(trifluoromethoxy) benzyl)-1H-1, 2, 3-triazol-4-(methoxy) isoindolin-2-) piperidine-2, 6-dione (11)

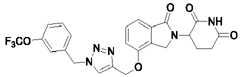

Step 1: azide was prepared as the preparation method 2 of azides

Step 2: 3-trifluoromethoxy benzyl azide and intermediate 6 were used as raw materials, the preparation method was the same as that of synthetic route 1 and Example 1, and 16.8 mg of 3-(1-oxo-4-((1-(3-(trifluoromethoxy) benzyl)-1*H*-1, 2, 3-triazol-4-(methoxy) isoindolin-2-) piperidine-2, 6-dione was obtained, yield 43%; ¹H NMR (400 MHz, DMSO) δ 10.96 (s, 1H), 8.38 (s, 1H), 7.48 (dt, *J* = 21.5, 8.4 Hz, 3H), 7.33 (dd, *J* = 8.6, 6.6 Hz, 4H), 5.69 (s, 2H), 5.31 (s, 2H), 5.10 (dd, *J* = 13.3, 5.0 Hz, 1H), 4.34 (d, *J* = 17.4 Hz, 1H), 4.18 (d, *J* = 17.4 Hz, 1H), 2.97-2.80 (m, 1H), 2.62 - 2.53 (m, 1H), 2.40 (qd, *J* = 13.3, 4.4 Hz, 1H), 1.97 (dd, *J* = 11.1, 5.6 Hz, 1H). UPLC-MS (ESI) calculated for C₂₄H₂₀F₃N₅O₅ [M+H]⁺: 516.14, found 516.32.

### EXAMPLE 12: 3-(4-((1-(4-(morpholinomethyl) benzyl)-1H-1, 2, 3-triazol-4-)methoxy)-1-oxoisoindolin-2-) piperidine-2, 6-dione (12)

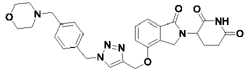

Step 1: 102.4 mg of 4-(4-azidomethylbenzyl) morpholine was obtained as a colorless oil with a yield of 92% according to the preparation method of 4-(3-azidomethylphenyl) morpholine (see Example 7);¹H NMR (400 MHz, CDCl₃) δ 7.35 (d, *J* = 8 Hz, 2H), 7.27 (d, *J* = 8 Hz, 2H), 4.32 (s, 2H), 3.76 - 3.66 (m, 4H), 3.50 (s, 2H), 2.49 - 2.39 (m, 4H).

Step 2: 4-(4-azidomethylbenzyl) morpholine and intermediate 6 were used as raw materials, the preparation method was the same as that of synthetic route 1 and Example 1, and 20.2 mg of 3-(4-((1-(4-morpholinmethyl)benzyl)-1H-1, 2, 3-triazol-4-)methoxy)-1-oxoisoindolin-2-) piperidine-2, 6-dione as a white solid was obtained, yield 28 %; ¹H NMR (400 MHz, DMSO) δ 10.97 (s, 1H), 8.31 (s, 1H), 7.52 - 7.47 (m, 1H), 7.44 (d, *J* = 7.6 Hz, 1H), 7.33 (d, *J* = 7.3 Hz, 1H), 7.29 (q, *J* = 8.4 Hz, 4H), 5.58 (s, 2H), 5.29 (s, 2H), 5.09 (dd, *J* = 13.3, 5.1 Hz, 1H), 4.33 (d, *J* = 17.5 Hz, 1H), 4.17 (d, *J* = 17.5 Hz, 1H), 3.57 - 3.52 (t,*J* =8.0 Hz, 4H), 3.43 (s, 2H), 2.95-2.83 (m, 1H), 2.56 (m, 1H), 2.47 - 2.35 (m, 1H), 2.31 (t,*J* =8.0 Hz, 4H), 1.99 - 1.91 (m, 1H). UPLC-MS (ESI) calculated for C₂₈H₃₀N₆O₅ [M+H]⁺: 531.23, found 531.58.

### EXAMPLE 13: 3-(4-(1-((1H-benzo [d]imidazol-5-) methyl)-1H-1, 2, 3-triazol-4-(methoxy)-1-oxoisoindolin-2-)piperidine-2, 6-dione (13)

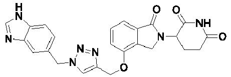

Step 1: compound 1*H*-benzimidazol-5-carboxylic acid was dissolved in 30ml of dry tetrahydrofuran and cooled to 0°C, lithium aluminum hydride (380mg, 10mmol) was added, and the reaction was raised to room temperature overnight. After the reaction was completed, the reaction solution was quenched with methanol, concentrated under reduced pressure to remove the solvent, 50mL of saturated sodium bicarbonate solution was added to the reaction mixture, extracted with ethyl acetate (3 × 80 mL), the organic layers were combined, washed with saturated sodium chloride, dried over anhydrous sodium sulfate, concentrated under reduced pressure, and subjected to silica gel column chromatography to obtain 94mg of (1*H*-benzo [*d*]imidazol-5-) methanol as a colorless liquid, yield 13%; ¹H NMR (400 MHz, DMSO) δ 12.36 (s, 1H), 8.15 (s, 1H), 7.57 - 7.44 (m, 2H), 7.14 (d, J = 9.3 Hz, 1H), 5.14 (t, J = 5.6 Hz, 1H), 4.58 (d, J = 5.6 Hz, 2H).

Step 2: (1*H*-benzo [*d*]imidazol-5-) methanol (87mg, 0.59 mmol) was dissolved in lOmL tetrahydrofuran, then diphenyl azidophosphate (140µL, 0.65 mmol) and DBU (98µL, 0.708 mmol) were added, and then heated and refluxed for 6h. After the reaction was completed, the solvent was removed under reduced pressure, the residue was diluted with ethyl acetate, washed with saturated sodium bicarbonate and saturated sodium chloride in turn, dried over anhydrous sodium sulfate, concentrated under reduced pressure, and subjected to silica gel column chromatography to obtain 6-azidomethyl-1*H*-benzo [*d*]imidazole 69.5 mg, yield 68%; ¹H NMR (400 MHz, DMSO) δ 12.54 (s, 1H), 8.25 (s, 1H), 7.61 (m, 2H), 7.20 (dd, J = 8.3, 1.3 Hz, 1H), 4.52 (s, 2H).

Step 3: 6-azidomethyl-1*H*-benzo [*d*]imidazole and intermediate 6 were used as raw materials, and the preparation method was the same as that of Synthetic Route 1 and Example 1, and 5.8 mg of 3-(4-(1-((1H-benzo [*d*] imidazol-5-) methyl)-1*H*-1, 2, 3-triazol-4-(methoxy)-1-oxoisoindolin-2-)piperidine-2, 6-dione was obtained, yield 13%; ¹H NMR (400 MHz, DMSO) δ 12.50 (s, 1H), 10.94 (s, 1H), 8.31 (s, 1H), 8.23 (s, 1H), 7.70 - 7.59 (m, 1H), 7.58 - 7.46 (m, 2H), 7.43 (d, *J* = 7.7 Hz, 1H), 7.33 (d, *J* = 7.1 Hz, 1H), 7.20 (ddd, *J* = 15.9, 6.7, 4.8 Hz, 1H), 5.69 (s, 2H), 5.27 (s, 2H), 5.08 (dd, *J* = 13.3, 5.1 Hz, 1H), 4.32 (d, *J =* 17.5 Hz, 1H), 4.16 (d, *J* = 17.4 Hz, 1H), 2.95 - 2.81 (m, 1H), 2.59 - 2.54 (m, 1H), 2.40 (ddd, *J* = 29.1, 14.3, 5.7 Hz, 1H), 1.98 - 1.91 (m, 1H). UPLC-MS (ESI) calculated for C₂₄H₂₁N₇O₄ [M+ H]⁺: 472.17, found 472.40.

### EXAMPLE 14: 3-(4-((1-(3-(1H-imidazol-1-) benzyl)-1H-1, 2, 3-triazol-4-) methoxy)-1-oxoisoindolin-2-)piperidine-2, 6-dione (14)

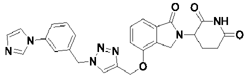

Step 1: 3-hydroxymethylphenylboronic acid (304mg, 2mmol), imidazole (163.39 mg, 2.4 mmol) and cuprous chloride (9.9 mg, 0.1 mmol) were added to a 25mL round bottom flask, lOmL methanol was added, the reaction system was heated to reflux for 5h, the solvent was concentrated under reduced pressure to be removed, and the residue was subjected to silica gel column chromatography to obtain 166mg of 1-(3-hydroxyniethylphenyl)-1H-imidazole as a colorless oil, yield 47.6%.

Step 2: 1-(3-hydroxymethylphenyl)-1H-imidazole was used as a raw material, and the preparation method was the same as that of synthesis method 2 of azides. 86mg of 1-(3-azidomethylphenyl)-1*H*-*imidazole* was obtained as a colorless oil with a yield of 90%; 1H NMR (400 MHz, CDC13) δ 7.89 (s, 1H), 7.51 (t, J = 7.9 Hz, 1H), 7.40 - 7.29 (m, 3H), 7.25-7.18 (m, 2H), 4.44 (s, 2H).

Step 3: 1-(3-azidomethylphenyl)-1*H*-imidazole and intermediate 6 were used as raw materials, the preparation method was the same as that of synthetic route 1 and Example 1 and 14 mg of 3-(4-((1-(3-(1*H*-imidazol-1-) benzyl)-1H-1, 2, 3-triazol-4-) methoxy)-1-oxoisoindolin-2-)piperidine-2, 6-dione was obtained as a white solid, yield 22 %; ¹H NMR (400 MHz, DMSO) δ 10.97 (s, 1H), 8.40 (s, 1H), 8.24 (s, 1H), 7.71 (d, *J* = 6.3 Hz, 2H), 7.63 (dd, *J* = 7.9, 1.5 Hz, 1H), 7.48 (dt, *J* = 18.9, 7.7 Hz, 3H), 7.33 (d, *J* = 7.2 Hz, 1H), 7.26 (d, *J* = 7.8 Hz, 1H), 7.12 (s, 1H), 5.68 (s, 2H), 5.30 (s, 2H), 5.09 (dd, *J* = 13.3, 5.1 Hz, 1H), 4.33 (d, *J* = 17.5 Hz, 1H), 4.18 (d, *J* = 17.5 Hz, 1H), 2.90 (ddd, *J* = 18.7, 13.6, 5.3 Hz, 1H), 2.58-2.4 (m, 1H), 2.41 (ddd, *J* = 26.9, 13.5, 4.6 Hz, 1H), 2.00 - 1.91 (m, 1H). UPLC-MS (ESI) calculated for C₂₆H₂₃N₇O₄ [M+H]⁺: 498.18, found 498.43.

### EXAMPLE 15: 3-(1-oxo-4-((1-phenyl-1H-1, 2, 3-triazol-4-)methoxy) isoindolin-2-) piperidine-2, 6-dione (15)

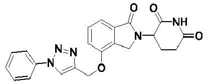

Azidobenzene and intermediate 6 were used as raw materials, the preparation method was the same as that of synthetic route 1 and Example 1 to obtain 2.4 mg of 3-(1-oxo-4-((1-phenyl-1H-1, 2, 3-triazol-4-)methoxy) isoindolin-2-) piperidine-2, 6-dione, yield 4 %; ¹H NMR (400 MHz, DMSO) δ 10.98 (s, 1H), 8.99 (s, 1H), 7.91 (d, *J* = 7.8 Hz, 2H), 7.62 (t, *J* = 7.8 Hz, 2H), 7.57 - 7.46 (m, 3H), 7.36 (d, *J* = 6.6 Hz, 1H), 5.41 (s, 2H), 5.11 (dd, *J* = 13.4, 5.1 Hz, 1H), 4.39 (d, *J* = 17.5 Hz, 1H), 4.23 (d, *J* = 17.5 Hz, 1H), 2.96 - 2.85 (m, 1H), 2.61-2.53 (m, 1H), 2.45 - 2.31 (m, 1H), 2.01 - 1.93 (m, 1H). UPLC-MS (ESI) calculated for C₂₂H₁₉N₅O₄ [M+H]⁺: 418.14, found 418.35.

### EXAMPLE 16: 3-(4-((1-(3-hydroxyphenyl)-1H-1,2,3-triazol-4-)methoxy)-1-oxoisoindolin-2-) piperidine-2, 6-dione (16)

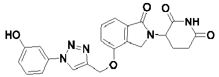

Step 1: 3-aminophenol was used as a raw material, and the preparation method was the same as that of synthesis method 1 of azides, and 210mg of 3-azidophenol was obtained as a brown oil with a yield of 85%; ¹H NMR (400 MHz, CDC13) δ 7.20 (t, J = 8.1 Hz, 1H), 6.61 (td, J = 7.8, 2.1 Hz, 2H), 6.50 (t, J = 2.2 Hz, 1H), 5.04 (s, 1H).

Step 2: 3-azidophenol and intermediate 6 were used as raw materials, the preparation method was the same as that of synthetic route 1 and Example 1, and 24.6 mg of 3-(4-((1-(3-hydroxyphenyl)-1*H*-1, 2, 3-triazol-4-)methoxy)-1-oxoisoindolin-2-) piperidine-2, 6-dione was obtained as a white solid, yield 42 %; ¹H NMR (400 MHz, DMSO) δ 10.98 (s, 1H), 10.08 (s, 1H), 8.92 (s, 1H), 7.56 - 7.47 (m, 2H), 7.41 - 7.34 (m, 2H), 7.32 - 7.28 (m, 2H), 6.89 (ddd, *J* = 8.1, 2.3, 1.1 Hz, 1H), 5.38 (s, 2H), 5.11 (dd, *J* = 13.3, 5.0 Hz, 1H), 4.39 (d, *J* = 17.5 Hz, 1H), 4.23 (d, *J* = 17.5 Hz, 1H), 2.97 - 2.84 (m, 1H), 2.61-2.53 (m, 1H), 2.42 (ddd, *J* = 17.5, 13.4, 4.7 Hz, 1H), 2.01 - 1.93 (m, 1H). UPLC-MS (ESI) calculated for C₂₂H₁₉N₅O₅ [M+H]⁺ : 434.14, found 434.26.

### EXAMPLE 17: 3-(1-oxo-4-((1-(4-(trifluoromethoxy) phenyl)-1H-1, 2, 3-triazol-4-(methoxy) isoindolin-2-) piperidine-2, 6-dione (17)

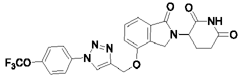

Step 1: 4- trifluoromethoxy aniline was used as a raw material, preparation method was the same as the preparation method 1 of azides, and 122mg of 4- trifluoromethoxy phenyl azide was obtained as a yellow oil with a yield of 53% ;1H NMR (400 MHz, CDCl₃) δ 7.21 (d, *J* = 8.8 Hz, 2H), 7.04 (d, *J* = 8.8 Hz, 2H).

Step 2: 4- trifluoromethoxyphenyl azide and intermediate 6 were used as raw materials, the preparation method was the same as that of synthetic route 1 and Example 1, and 16.8 mg of 3-(1-oxo-4-((1-(4-(trifluoromethoxy) phenyl)-1H-1, 2, 3-triazol-4-)methoxy) isoindolin-2-) piperidine-2, 6-dione was obtained as a white solid, yield 33 %; ¹H NMR (400 MHz, DMSO) δ 10.98 (s, 1H), 9.03 (s, 1H), 8.06 (d, *J* = 9.0, 2H), 7.64 (d, *J* = 9.0 Hz, 2H), 7.56-7.45 (m, 2H), 7.36 (d, *J* = 6.8 Hz, 1H), 5.42 (s, 2H), 5.12 (dd, *J* = 13.3, 5.0 Hz, 1H), 4.39 (d, *J* = 17.5Hz, 1H), 4.24 (d, *J* = 17.5 Hz, 1H), 2.97 - 2.85 (m, 1H), 2.61-2.53 (m, 1H), 2.42 (m, 1H), 2.01-1.93 (m, 1H). UPLC-MS (ESI) calculated for C₂₃H₁₈F₃N₅O₅ [M + H]⁺: 502.13, found 502.22.

### EXAMPLE 18: 3-(4-((1-(2, 3-dichlorophenyl)-1H-1, 2, 3-triazol-4-)methoxy)-1-oxoisoindolin-2-) piperidine-2, 6-dione (18)

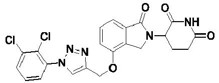

Step 1: 2,3-dichloroaniline was used as a raw material, the preparation method was the same as the preparation method 1 of azides, and 316mg of 2,3-dichlorophenyl azide was obtained as a yellow solid with a yield of 91%; 1H NMR (400 MHz, CDC13) δ 7.23 (m, 2H), 7.10 (dd, J = 7.3, 2.1 Hz, 1H).

Step 2: 2,3-dichlorophenyl azide and intermediate 6 were used as raw materials, the preparation method was the same as that of synthetic route 1 and Example 1, and 44.9 mg of 3-(4-((1-(2, 3-dichlorophenyl)-1*H*-1, 2, 3-triazol-4-)methoxy)-1-oxoisoindolin-2-) piperidine-2, 6-dione was obtained as a white solid, yield 55 %; ¹H NMR (400 MHz, DMSO) δ 10.99 (s, 1H), 8.79 (s, 1H), 7.93 (dd, *J* = 8.1, 1.1 Hz, 1H), 7.72 (dd, *J* = 7.9, 1.1 Hz, 1H), 7.62 (t, *J* = 8.1 Hz, 1H), 7.52 (m, 2H), 7.36 (d, *J* = 6.6 Hz, 1H), 5.12 (dd, *J* = 13.3, 5.0 Hz, 1H), 4.39 (d, *J* = 17.5 Hz, 1H), 4.23 (d, *J* = 17.5 Hz, 1H), 2.97-2.84 (m, 1H), 2.62-2.53 (m, 1H), 2.49-2.37 (m, 1H), 2.02-1.92 (m, 1H). UPLC-MS (ESI) calculated for C₂₂H₁₇Cl₂N₅O₄ [M + H]⁺: 486.07, found 486.16.

### EXAMPLE 19: 3-(4-((1-(4-morpholinophenyl)-1H-1, 2, 3-triazol-4-)methoxy)-1-oxoisoindolin-2-) piperidine-2, 6-dione (19)

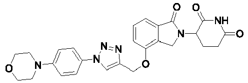

Step 1: the preparation method was the same as that of synthesis method 1 of azides, and 101mg of 4-(4-azidophenyl)morpholine was obtained, yield 44.2%; ¹H NMR (400 MHz, CDCl₃) δ 6.95 (d, *J* = 9.0 Hz, 2H), 6.90 (d, *J* = 9.0 Hz, 2H), 3.89 - 3.82 (t,*J*=4.8Hz,4H), 3.15 - 3.08 (t,*J*=4.8Hz,4H).

Step 2: 4-(4-azidophenyl) morpholine and intermediate 6 were used as raw materials, the preparation method was the same as that of synthetic route 1 and Example 1, and 47.7 mg of white solid was obtained, yield 57 %; ¹H NMR (400 MHz, DMSO) δ 10.98 (s, 1H), 8.84 (s, 1H), 7.72 (d, *J* = 9.0 Hz, 2H), 7.57 ― 7.45 (m, 2H), 7.35 (d, *J* = 6.9 Hz, 1H), 7.11 (d, *J* = 9.0 Hz, 2H), 5.37 (s, 2H), 5.11 (dd, *J* = 13.3, 5.0 Hz, 1H), 4.38 (d, *J* = 17.5 Hz, 1H), 4.22 (d, *J* = 17.5 Hz, 1H), 3.81 - 3.68 (m, 4H), 3.24 - 3.13 (m, 4H), 2.97 - 2.83 (m, 1H), 2.62-2.53(m, 1H), 2.48 -2.35 (m, 1H), 2.02-1.92 (m, 1H). UPLC-MS (ESI) calculated for C₂₆H₂₆N₆O₅ [M + H]⁺: 503.20, found 503.30.

### EXAMPLE 20: 3-(1-oxo-4-((1-(3-(trifluoromethoxy) phenyl)-1H-1, 2, 3-triazol-4-)methoxy) isoindolin-2-) piperidine-2, 6-dione (20)

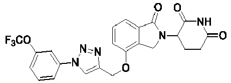

Step 1: the preparation method was the same as that of synthetic method 1 of azides, 74 mg of 3-trifluoromethoxyphenyl azide was obtained as a yellow oil, yield 32%; ¹H NMR (400 MHz, CDCl₃)δ 7.38 (t, *J* = 8.2 Hz, 1H), 6.99 (td, *J* = 8.4, 1.4 Hz, 2H), 6.87 (s, 1H).

Step 2: 3- trifluoromethoxyphenyl azide and intermediate were used as raw materials, the preparation method was the same as that of synthetic route 1 and Example 1, and 52.2 mg of 3-(1-oxo-4-((1-(3-(trifluoromethoxy) phenyl)-1H-1, 2, 3-triazol-4-)methoxy) isoindolin-2-) piperidine-2, 6-dione was obtained as a white powder, yield 62 %; ¹H NMR (400 MHz, DMSO) δ 10.98 (s, 1H), 9.09 (s, 1H), 8.04 - 7.98 (m, 2H), 7.76 (t, *J* = 8.4 Hz, 1H), 7.58 - 7.47 (m, 3H), 7.36 (dd, *J* = 7.1, 0.9 Hz, 1H), 5.42 (s, 2H), 5.11 (dd, *J* = 13.4, 5.1 Hz, 1H), 4.39 (d, *J* = 17.5 Hz, 1H), 4.23 (d, *J* = 17.5 Hz, 1H), 2.97 - 2.84 (m, 1H), 2.61 - 2.53 (m, 1H), 2.48-2.35 (m, 1H), 2.02 - 1.94 (m, 1H). UPLC-MS (ESI) calculated for C₂₃H₁₈F₃N₅O₅ [M + H]⁺: 502.13, found 502.22.

### EXAMPLE 21: 3-(4-((1-(4-hydroxyphenyl)-1H-1, 2, 3-triazol-4-)methoxy)-1-oxoisoindolin-2-) piperidine-2, 6-dione (21)

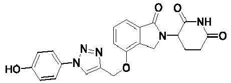

Step 1: the preparation method was the same as that of synthesis method 1 of azides, and 162mg of 4-azidophenol was obtained as a red brown solid with a yield of 66%; ¹H NMR (400 MHz, CDCl₃)δ 6.91 (d, *J* = 8.8 Hz, 2H), 6.82 (d, *J* = 8.8 Hz, 2H), 4.79 (s, 1H).

Step 2: 4-azidophenol and intermediate 6 were used as raw materials, the preparation method was the same as that of synthetic route 1 and Example 1, and 42.6 mg of 3-(4-((1-(4-hydroxyphenyl)-1H-1, 2, 3-triazol-4-)methoxy)-1-oxoisoindolin-2-) piperidine-2, 6-dione was obtained as a white solid, yield 59 %; ¹H NMR (400 MHz, DMSO) δ 10.98 (s, 1H), 9.99 (s, 1H), 8.79 (s, 1H), 7.69 - 7.63 (m, 2H), 7.55 - 7.46 (m, 2H), 7.37 - 7.33 (m, 1H), 6.97 - 6.91 (m, 2H), 5.37 (s, 2H), 5.11 (dd, *J* = 13.3, 5.1 Hz, 1H), 4.38 (d, *J* = 17.5 Hz, 1H), 4.22 (d, *J* = 17.5 Hz, 1H), 2.91 (ddd, *J* = 17.6, 13.7, 5.4 Hz, 1H), 2.61 - 2.54 (m, 1H), 2.42 (ddd, *J* = 26.0, 13.0, 4.2 Hz, 1H), 2.02-1.94 (m, 1H). UPLC-MS (ESI) calculated for C₂₂H₁₉N₅O₅ [M + H]⁺ : 434.14, found 434.26.

### EXAMPLE 22: 3-(4-((1-(3-morpholinophenyl)-1H-1, 2, 3-triazol-4-)methoxy)-1-oxoisoindolin-2-) piperidine-2, 6-dione (22)

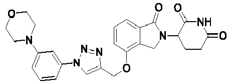

Step 1: the preparation method was the same as that of synthesis method 1 of azides, and 69mg of 4-(3-azidophenyl)morpholine was obtained as a yellow oil, yield 30%; ¹H NMR (400 MHz, CDCl₃) δ 7.18 (t, *J* = 8.1 Hz, 1H), 6.87 (m, 1H), 6.84 (d, *J* = 7.8 Hz, 1H), 6.78 (dd, *J* = 8.4, 2.3 Hz, 1H), 3.88 - 3.82 (t,*J*=4.7Hz, 4H), 3.16 (t,*J*=4.7Hz, 4H).

Step 2: 4-(3-azidophenyl) morpholine and intermediate 6 were used as raw materials, the preparation method was the same as that of Example 1, and 33.3 mg of 3-(4-((1-(3-morpholinphenyl)-1H-1, 2, 3-triazol-4-)methoxy)-1-oxoisoindolin-2-) piperidine-2, 6-dione was obtained as a white solid, yield 40 %; ¹H NMR (400 MHz, DMSO) δ 10.98 (s, 1H), 8.98 (s, 1H), 7.56 - 7.46 (m, 2H), 7.39 (m, 3H), 7.29 (dd, *J* = 7.8, 1.2 Hz, 1H), 7.06 (dd, *J* = 8.4, 2.0 Hz, 1H), 5.39 (s, 2H), 5.11 (dd, *J* = 13.3, 5.0 Hz, 1H), 4.38 (d, *J* = 17.5 Hz, 1H), 4.23 (d, *J* = 17.5 Hz, 1H), 3.80 - 3.70 (t, *J* = 4.6 Hz,4H), 3.28 - 3.16 (t, *J* = 4.6 Hz, 4H), 2.97 - 2.83 (m, 1H), 2.61-2.54 (m, 1H), 2.48-2.35 (m, 1H), 2.01-1.92 (m, 1H). UPLC-MS (ESI) calculated for C₂₆H₂₆N₆O₅ [M + H]⁺: 503.20, found 503.30.

### EXAMPLE 23: 3-(4-((1-(benzo [d] thiazol-6)-1H-1, 2, 3-triazol-4-(methoxy)-1-oxoisoindolin-2-) piperidine-2, 6-dione (23)

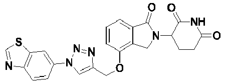

Step 1: the preparation method was the same as that of synthesis method 1, and 191mg of 6-azidobenzo[d]thiazole was obtained as a yellow solid with a yield of 81.5%; ¹H NMR (400 MHz, CDCl₃) δ 8.93 (s, 1H), 8.10 (d, *J* = 8.7 Hz, 1H), 7.60 (d, *J* = 2.2 Hz, 1H), 7.20 (dd, *J* = 8.7, 2.2 Hz, 1H)

Step 2: 6-azidobenzo[d]thiazole and intermediate 6 were used as raw materials, the preparation method was the same as that of Synthetic Route 1 and Example 1, and 3-(4-((1-(benzo [*d*] thiazol-6)-1*H*-1, 2, 3-triazol-4-(methoxy)-1-oxoisoindolin-2-) piperidine-2, 6-dione was obtained; ¹H NMR (400 MHz, DMSO) δ 10.97 (s, 1H), 9.52 (s, 1H), 9.04 (s, 1H), 8.79 (d, *J* = 2.2 Hz, 1H), 8.30 (d, *J* = 8.8 Hz, 1H), 8.09 (dd, *J* = 8.8, 2.2 Hz, 1H), 7.57 - 7.48 (m, 2H), 7.36 (dd, *J* = 6.8, 1.1 Hz, 1H), 5.44 (s, 2H), 5.11 (dd, *J* = 13.4, 4.9 Hz, 1H), 4.40 (d, *J* = 17.5 Hz, 1H), 4.25 (d, *J* = 17.5 Hz, 1H), 2.96 - 2.85 (m, 1H), 2.62 - 2.53 (m, 1H), 2.42 (ddd, *J* = 26.7, 13.7, 4.7 Hz, 1H), 2.03 - 1.93 (m, 1H). UPLC-MS (ESI) calculated for C₂₃H₁₈N₆O₄S [M + H]⁺: 475.11, found 475.17.

### EXAMPLE 24: 3-(1-oxo-4-((1-(4-(((R)-tetrahydrofuran-3-)oxy)phenyl)-1H-1,2,3-triazol-4-)methoxy)isoindolin-2-)piperidine-2,6-dione (24)

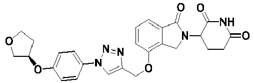

**Step 1:** diisopropyl azodicarboxylate (233ul, 1.18mmol) was added to the solution of 4-azidophenol (80mg, 0.59mmol), (S)-(+)-3-hydroxytetrahydrofuran (104mg, 1.184mmol) and triphenylphosphine (310mg, 1.18mmol) in tetrahydrofuran solution (6mL) under ice bath and nitrogen protection, the reaction solution was raised to room temperature and reacted overnight. After the reaction was completed, the solvent was removed under reduced pressure, and the residue was subjected to silica gel column chromatography to obtain 41mg of (*S*)-3-(4-azidophenoxy) tetrahydrofuran as a brown solid, yield 33.7%; ¹H NMR (400 MHz, CDCl₃)δ 6.98 - 6.92 (m, 2H), 6.88 - 6.82 (m, 2H), 4.89 (ddt, *J* = 6.1, 4.3, 2.2 Hz, 1H), 4.03 - 3.87 (m, 4H), 2.25 - 2.09 (m, 2H).

Step 2: (S)-3-(4-azidophenoxy)tetrahydrofuran and intermediate 6 were used as raw materials, the preparation method was the same as that of synthetic route 1 and Example 1, and 30mg of 3-(1-oxo-4-((1-(4-(((R)-tetrahydrofuran-3-)oxy)phenyl)-1H- 1,2,3-triazol-4-)methoxy)isoindolin-2-)piperidine-2,6-dione was obtained, yield 44%; ¹H NMR (400 MHz, DMSO), δ 10.97 (s, 1H), 8.87 (s, 1H), 7.80 (d, *J* = 8.9 Hz, 2H),7.56-7.46 (m, 2H), 7.35 (d, *J* = 7.1 Hz, 1H), 7.13 (d, *J* = 9.0 Hz, 2H), 5.39 (s, 2H), 5.13-5.09 (m, 2H), 4.38 (d, *J* = 17.5 Hz, 1H), 4.23 (d, *J* = 17.5 Hz, 1H), 3.91-3.73 (m, 4H), 2.97-2.83 (m, 1H), 2.63 - 2.54 (m, 1H), 2.48-2.35 (m, 1H), 2.33 - 2.20 (m, 1H), 2.01 - 1.92 (m, 2H). UPLC-MS (ESI) calculated for C₂₆H₂₅N₅O₆ [M + H]⁺: 504.18, found 504.28.

### EXAMPLE 25: 3-(1-oxo-4-((1-(4-(((S)-tetrahydrofuran-3-)oxy)phenyl)-1H-1,2,3-triazol-4-)methoxy)isoindolin-2-)piperidine-2,6-dione (25)

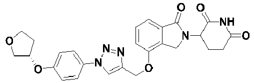

Step 1: The preparation method was the same as (S)-3-(4-azidophenoxy) tetrahydrofuran, and 41mg of (*R*)-3-(4-azidophenoxy) tetrahydrofuran was obtained as a red brown solid, yield 33.7%; ¹H NMR (400 MHz, CDCl₃)δ 6.98 - 6.92 (m, 2H), 6.88 - 6.82 (m, 2H), 4.89 (ddt, *J* = 6.2, 4.3, 2.2 Hz, 1H), 4.04 - 3.84 (m, 4H), 2.27 - 2.05 (m, 2H).

Step 2: (R)-3-(4-azidophenoxy)tetrahydrofuran and intermediate 6 were used as raw materials, the preparation method was the same as that of synthetic route 1 and Example 1, and 51mg of 3-(1-oxo-4-((1-(4-(((S)-tetrahydrofuran-3-)oxy)phenyl)-1H-1,2,3-triazol-4-)methoxy)isoindolin-2-)piperidine-2,6-dione was obtained, yield 75 %; ¹H NMR (400 MHz, DMSO) δ 10.96 (s, 1H), 8.87 (s, 1H), 7.80 (d, *J* = 9.0 Hz, 2H), 7.57 ― 7.45 (m, 2H), 7.36 (d, *J* = 6.6 Hz, 1H), 7.13 (d, *J =* 9.0 Hz, 2H), 5.39 (s, 2H), 5.15 - 5.05 (m, 2H), 4.38 (d, *J* = 17.5 Hz, 1H), 4.23 (d, *J* = 17.5 Hz, 1H), 3.95 - 3.73 (m, 4H), 2.97-2.83 (m, 1H), 2.63 - 2.54 (m, 1H), 2.48-2.35(m, 1H), 2.33 - 2.20 (m, 1H), 2.03 - 1.92 (m, 2H). UPLC-MS (ESI) calculated for C₂₆H₂₅N₅O₆ [M + H]⁺: 504.18, found 504.24.

### EXAMPLE 26: 3-(4-((1-(1H-indol-5-)-1H-1,2,3-triazol-4-)methoxy)-1-oxoisoindolin-2-) piperidine-2,6-dione (26)

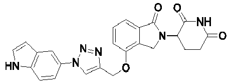

Step 1: the preparation method was the same as that of synthesis method 1, and 601mg of 5-azido-1H-indole was obtained as a yellow solid with a yield of 85.2%; ¹H NMR (400 MHz, CDCl₃)δ 8.16 (s, 1H), 7.36 (d, *J* = 8.6 Hz, 1H), 7.31 (d, *J* = 2.0 Hz, 1H), 7.25 (t, *J* = 2.8 Hz, 1H), 6.89 (dd, *J* = 8.6, 2.2 Hz, 1H), 6.52 (t, *J* = 2.1 Hz, 1H).

Step 2: 5-azido-1H-indole and intermediate 6 were used as raw materials, the preparation method was the same as that of Example 1 and 37 mg of 3-(4-((1-(1H-indol-5-)-1H-1,2,3-triazol-4-)methoxy)-1-oxoisoindolin-2-) piperidine-2,6-dione was obtained, yield 61 %; ¹H NMR (400 MHz, DMSO) δ 11.45 (s, 1H), 10.98 (s, 1H), 8.88 (s, 1H), 8.01 (s, 1H), 7.60 - 7.48 (m, 5H), 7.36 (dd, *J* = 6.7, 1.2 Hz, 1H), 6.61 - 6.51 (m, 1H), 5.40 (s, 2H), 5.11 (dd, *J* = 13.3, 5.0 Hz, 1H), 4.40 (d, *J* = 17.5 Hz, 1H), 4.24 (d, *J* = 17.5 Hz, 1H), 2.97 - 2.83 (m, 1H), 2.61-2.54 (m, 1H), 2.47 - 2.35 (m, 1H), 2.03 - 1.93 (m, 1H). UPLC-MS (ESI) calculated for C₂₄H₂₀N₆O₄ [M + H]⁺: 457.15, found 457.25.

### EXAMPLE 27: 3-(4-((1-(1-(2-(dimethylamino)ethyl)-1H-indol-5-)-1H-1,2,3-triazol-4-)methoxy )-1-oxoisoindoli n-2-)piperidine-2,6-dione (27)

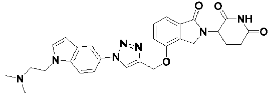

Step 1: 5-azido-1-(2-methoxyethyl)-1*H*-indole (100mg, 0.63 mmol), dimethylaminochloroethane hydrochloride (118.4 mg, 0.822 mmol) and potassium carbonate (262mg, 1.9 mmol) were dissolved in 5ml of DMF, and the reaction solution was heated to 80°C and reacted overnight. After the reaction was completed, the reaction solution was diluted with ethyl acetate, washed with water and saturated sodium chloride solution in turn, the organic phase was dried over anhydrous sodium sulfate, filtered, the solvent was removed under reduced pressure, and the residue was subjected to silica gel column chromatography to obtain 61.7 mg of 2-(5-azide-1H-indol-1-)-N,N-dimethylethyl-1-amine as a red-brown oil, yield 43%; ¹H NMR (400 MHz, CDCl₃)δ 7.31 (d, *J* = 8.7 Hz, 1H), 7.28 (d, *J* = 2.0 Hz, 1H), 7.17 (d, *J* = 3.1 Hz, 1H), 6.89 (dd, *J* = 8.7, 2.2 Hz, 1H), 6.44 (d, *J* = 3.1 Hz, 1H), 4.21 (t, *J* = 7.1 Hz, 2H), 2.69 (t, *J* = 7.1 Hz, 2H), 2.29 (s, 6H). UPLC―MS (ESI) calculated for C₂₈H₂₉N₇O₄ [M + H]⁺: 528.23, found 528.73.

Step 2: 2-(5-azide-1H-indol-1-)-N,N-dimethylethyl-1-amine and intermediate 6 were used as raw materials, the preparation method was the same as that of Synthetic Route 1 and Example 1 and 41mg of 3-(4-((1-(1-(2-(dimethylamino)ethyl)-1H-indol-5-)-1H-1,2,3-triazol-4-)methoxy )-1-oxoisoindoli n-2-)piperidine-2,6-dione was obtained, yield 58 %; ¹H NMR (400 MHz, DMSO) δ 10.98 (s, 1H), 8.90 (s, 1H), 8.01 (d, *J* = 2.0 Hz, 1H), 7.71 (d, *J* = 8.9 Hz, 1H), 7.62 (dd, *J* = 8.8, 2.1 Hz, 1H), 7.56 (d, *J* = 3.1 Hz, 1H), 7.52 (q, *J* = 6.8 Hz, 2H), 7.36 (dd, *J* = 6.6, 1.4 Hz, 1H), 6.57 (d, *J* = 3.1 Hz, 1H), 5.40 (s, 2H), 5.12 (dd, *J* = 13.3, 5.1 Hz, 1H), 4.40 (d, *J* = 17.5 Hz, 1H), 4.35 (t, *J* = 6.5 Hz, 2H), 4.24 (d, *J* = 17.5 Hz, 1H), 2.98 - 2.83 (m, 1H), 2.70 (t, *J* = 6.5 Hz, 2H), 2.61-2.54 (m, 1H), 2.40-2.45 (m, 1H), 2.23 (s, 6H), 2.03-1.93 (m, 1H).

### EXAMPLE 28: 3-(4-(1-(1-(2-methoxyethyl)-1H-indol-5-)-1H-1, 2, 3-triazol-4-)methoxy)-1-oxoisoindolin-2-) piperidine-2, 6-dione (28)

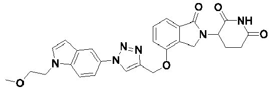

Step 1: 5-azido-1H-indole (100mg, 0.632 mmol) was dissolved in 5ml of dry DMF solution under ice bath, sodium hydride (38mg, 0.95 mmol) was added, the reaction solution was raised to room temperature and continued to stir and react for 30min, 2-bromoethyl methyl ether (71.3 ul, 0.759 mmol) was added, then the temperature was raised to 60°C and reacted overnight. After the reaction was completed, the reaction solution was quenched with water, extracted with ethyl acetate, the organic layer was washed with water and saturated sodium chloride respectively, dried over anhydrous sodium sulfate, the solvent was removed under reduced pressure, and 124.5 mg of 5-azido-1-(2-methoxyethyl)-1H-indole was obtained as a yellow oil by silica gel column chromatography, yield 93%; ¹H NMR (400 MHz, CDCl₃)δ 7.32 (d, *J* = 8.7 Hz, 1H), 7.29 (d, *J* = 2.1 Hz, 1H), 7.19 (d, *J* = 3.1 Hz, 1H), 6.89 (dd, *J* = 8.7, 2.2 Hz, 1H), 6.44 (d, *J* = 3.1 Hz, 1H), 4.27 (t, *J* = 5.5 Hz, 2H), 3.70 (t, *J* = 5.5 Hz, 2H), 3.31 (s, 3H).

Step 2: 5-azido-1-(2-methoxyethyl)-1H-indole and intermediate 6 were used as raw materials, the preparation method was the same as that of Synthetic Route 1 and Example 1, and 39mg of 3-(4-(1-(1-(2-methoxyethyl)-1*H*-indol-5-)-1*H*-1, 2, 3-triazol-4-)methoxy)-1-oxoisoindolin-2-) piperidine-2, 6-dione was obtained, yield 56 %; ¹H NMR (400 MHz, DMSO) δ 10.98 (s, 1H), 8.90 (s, 1H), 8.02 (d, *J* = 1.9 Hz, 1H), 7.71 (d, *J* = 8.8 Hz, 1H), 7.62 (dd, *J* = 8.8, 2.0 Hz, 1H), 7.57 ― 7.48 (m, 3H), 7.39 ― 7.24 (m, 2H), 6.58 (d, *J* = 3.1 Hz, 1H), 5.40 (s, 2H), 5.12 (dd, *J* = 13.3, 5.0 Hz, 1H), 4.46 - 4.35 (m, 3H), 4.24 (d, *J* = 17.5 Hz, 1H), 3.67 (t, *J* = 5.2 Hz, 2H), 3.22 (s, 3H), 2.97-2.85 (m, 1H), 2.61-2.53 (m, 1H), 2.42-2.35 (m, 1H), 2.02 - 1.92 (m, 1H). UPLC-MS (ESI) calculated for C₂₇H₂₆N₆O₅ [M + H]⁺: 515.20, found 515.27.

### EXAMPLE 29: 3-(1-oxo-4-((1-(4-((2H-tetrahydropyran-4-)methoxy)phenyl)-1H-1,2,3-triazol-4- )methoxy)isoin dolin-2-)piperidine-2,6-dione (29)

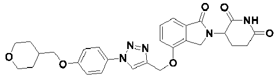

Step 1: The preparation method was the same as (*S*)-3-(4-azidophenoxy) tetrahydrofuran, and 81mg of 4-((4-azidophenoxy)methyl)tetrahydro-2H-pyran was obtained as a red brown oil, yield 59%; ¹H NMR (400 MHz, CDC13) δ 6.97 - 6.92 (m, 2H), 6.89 - 6.85 (m, 2H), 4.02 (dd, J = 10.8, 3.7 Hz, 2H), 3.78 (d, J = 6.4 Hz, 2H), 3.45 (td, J = 11.9, 2.1 Hz, 2H), 2.12 - 1.99 (m, 1H), 1.75 (dd, J = 13.0, 1.8 Hz, 2H), 1.46 (ddd, J = 25.3, 12.1, 4.5 Hz, 2H).

Step 2: 4-((4-azidophenoxy)methyl)tetrahydro-2H-pyran and intermediate 6 were used as raw materials, the preparation method was the same as that of synthetic route 1 and Example 1, and 37mg of 3-(1-oxo-4-((1-(4-((2H-tetrahydropyran-4-)methoxy)phenyl)-1H-1,2,3-triazol-4-)methoxy)isoind olin-2-)piperidine-2,6-dione was obtained, yield 52%; ¹H NMR (400 MHz, DMSO) δ 10.98 (s, 1H), 8.87 (s, 1H), 7.79 (d, *J* = 8.9 Hz, 2H), 7.57 ― 7.45 (m, 2H), 7.35 (d, *J* = 7.0 Hz, 1H), 7.14 (d, *J* = 8.9 Hz, 2H), 5.38 (s, 2H), 5.11 (dd, *J* = 13.2, 4.9 Hz, 1H), 4.38 (d, *J* = 17.5 Hz, 1H), 4.23 (d, *J* = 17.4 Hz, 1H), 3.89 (dd, *J* = 15.2, 4.7 Hz, 4H), 3.40 - 3.25 (m, 2H), 2.99 - 2.83 (m, 1H), 2.62 - 2.53 (m, 1H), 2.42 (m, 1H), 2.10 - 1.89 (m, 2H), 1.72-1.63 (m, 2H), 1.34 (m, 2H). UPLC-MS (ESI) calculated for C₂₈H₂₉N₅O₆ [M + H]⁺: 532.21, found 532.26.

### EXAMPLE 30: 3-(1-oxo-4-((1-(4-((2H-tetrahydropyran-4-)oxy)phenyl)-1H-1,2,3-triazol-4-)methoxy)isoindolin-2-)piperidine-2,6-dione (30)

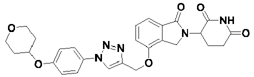

Step 1: the preparation method was the same as (S)-3-(4-azidophenoxy) tetrahydrofuran, and 82mg of 4-((4-azidophenoxy)tetrahydro-2H-pyran was obtained as a red brown oil, yield 63%; ¹H NMR (400 MHz, CDCl₃)δ 6.97 - 6.93 (m, 2H), 6.93 - 6.88 (m, 2H), 4.43 (tt, *J* = 7.8, 3.8 Hz, 1H), 4.02 - 3.92 (m, 2H), 3.57 (ddd, *J* = 11.6, 8.3, 3.2 Hz, 2H), 2.05 - 1.95 (m, 2H), 1.77 (dtd, *J* = 12.4, 8.2, 3.8 Hz, 2H).

Step 2: 4-((4-azidophenoxy)tetrahydro-2H-pyran and intermediate 6 were used as raw materials, the preparation method was the same as that of synthetic route 1 and Example 1, and 16mg of 3-(1-oxo-4-((1-(4-((2H-tetrahydropyran-4-)oxy)phenyl)-1H-1,2,3-triazol-4-)methoxy)isoindolin-2-)piperidine-2,6-dione was obtained, yield 18 %; ¹H NMR (400 MHz, DMSO) δ 10.97 (s, 1H), 8.87 (s, 1H), 7.78 (d, *J* = 8.9 Hz, 2H), 7.57 ― 7.45 (m, 2H), 7.35 (d, *J* = 6.9 Hz, 1H), 7.19 (d, *J =* 9.0 Hz, 2H), 5.38 (s, 2H), 5.11 (dd, *J* = 13.3, 5.0 Hz, 1H), 4.73 - 4.62 (m, 1H), 4.38 (d, *J* = 17.5 Hz, 1H), 4.23 (d, *J* = 17.5 Hz, 1H), 3.86 (dt, *J* = 11.1, 4.2 Hz, 2H), 3.55 - 3.44 (m, 2H), 2.99 - 2.83 (m, 1H), 2.57 (dd, *J* = 17.9, 1.8 Hz, 1H), 2.42 (ddd, *J* = 26.3, 13.3, 4.4 Hz, 1H), 2.00 (dd, *J* = 12.5, 3.9 Hz, 3H), 1.67 - 1.53 (m, 2H). UPLC-MS (ESI) calculated for C₂₇H₂₇N₅O₆ [M + H]⁺: 518.20, found 518.23.

### EXAMPLE 31: 3-(4-((1-(4-(epoxy propanoxy-3-oxy)phenyl)-1H-1,2,3-triazol-4-)methoxy)-1-oxoisoindolin-2-)piperidine-2,6-dione (31)

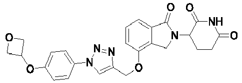

Step 1: Sodium hydride (80mg, 2.01 mmol) was added to the solution (6mL) of oxetan-3-ol (99mg, 1.34 mmol) in DMF under the condition of ice bath cooling. The reaction was continued with stirring for 30min under the condition of ice bath cooling, and then p-fluoronitrobenzene (170 ul, 1.60 mmol) was added, and the reaction system was raised to room temperature and reacted overnight. After the reaction was completed, the reaction system was quenched with water, extracted with ethyl acetate, the organic layer was washed with saturated sodium chloride, dried, the solvent was removed under reduced pressure, and the residue was subjected to silica gel column chromatography to obtain 207mg of 3-(4-nitrophenoxy) oxetane as a yellow solid, yield 79%; ¹H NMR (400 MHz, CDCl₃) δ 8.24 - 8.17 (m, 2H), 6.81 - 6.73 (m, 2H), 5.34 - 5.25 (m, 1H), 5.02 (t, *J* = 7.1 Hz, 2H), 4.81 - 4.73 (m, 2H).

Step 2: 3-(4-nitrophenoxy) oxetane (196mg, 1mmol) was dissolved in 10ml of methanol, ammonium chloride (267mg, 5mmol), Zinc powder (327mg, 5mmol) and a small amount of acetic acid were added sequentially to the reaction solution. The reaction solution reacted for 1h at room temperature, filtered by diatomite, the solvent was removed under reduced pressure, diluted with ethyl acetate, the organic layer was washed with saturated sodium bicarbonate and saturated sodium chloride in turn, dried over anhydrous sodium sulfate, filtered, concentrated under reduced pressure, and 133mg of 3-(4-aminophenoxy) oxetane was obtained by silica gel column chromatography as a yellow solid, yield 68%; ¹H NMR (400 MHz, CDCl₃)δ 6.62 (d, *J* = 8.7 Hz, 2H), 6.54 (d, *J* = 8.8 Hz, 2H), 5.14 - 5.06 (m, 1H), 4.92 (t, *J* = 6.7 Hz, 2H), 4.74 (dd, *J* = 7.0, 5.6 Hz, 2H).

Step 3: the preparation method was the same as that of synthesis method 1, and 108.4mg of 3-(4-azidophenoxy)oxetane was obtained as a yellow solid with a yield of 74%; ¹H NMR (400 MHz, CDCl₃)δ 6.98 - 6.91 (m, 2H), 6.72 - 6.66 (m, 2H), 5.22 - 5.11 (m, 1H), 4.96 (t, *J* = 6.8 Hz, 2H), 4.75 (dd, *J* = 7.2, 5.5 Hz, 2H).

Step 4: 3-(4-azidophenoxy)oxetane and intermediate 6 were used as raw materials, the preparation method was the same as that of synthetic route 1 and Example 1, and 33mg of 3-(4-((1-(4-(epoxy propanoxy-3-oxy)phenyl)-1H-1,2,3-triazol-4-)methoxy)-1-oxoisoindolin-2-)piperidine-2,6-dione was obtained, yield 55%; ¹H NMR (400 MHz, DMSO) δ 10.97 (s, 1H), 8.87 (s, 1H), 7.80 (d, *J* = 12.3 Hz, 2H), 7.58 ― 7.45 (m, 2H), 7.35 (d, *J* = 6.9 Hz, 1H), 7.01 (d, *J* = 9.0 Hz, 2H), 5.41 - 5.33 (m, 3H), 5.11 (dd, *J* = 13.3, 5.0 Hz, 1H), 4.96 (t, *J* = 6.7 Hz, 2H), 4.57 (dd, *J* = 7.3, 5.0 Hz, 2H), 4.38 (d, *J* = 17.5 Hz, 1H), 4.22 (d, *J* = 17.5 Hz, 1H), 2.97 - 2.81 (m, 1H), 2.56 (dd, *J* = 10.9, 9.1 Hz, 1H), 2.46 - 2.31 (m, 1H), 2.04 - 1.91 (m, 1H). UPLC-MS (ESI) calculated for C₂₅H₂₃N₅O₆ [M + H]⁺: 490.16, found 490.21.

### EXAMPLE 32: 3-(4-((1-(4-cyclopropoxyphenyl)-1H-1, 2, 3-triazol-4-(methoxy)-1-oxoisoindolin-2-) piperidine-2, 6-dione (32)

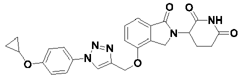

Step 1: NaH (60% dispersed in mineral oil, 103mg, 2.58mmol) was added to the solution (6mL) of cyclopropanol (100mg, 1.72mmol) in DMF under the condition of ice bath cooling. The reaction was continued for 30 min under the condition of ice bath cooling, and then p-fluoronitrobenzene (219ul, 2.07mmol) was added. The reaction solution was raised to room temperature and reacted overnight. After the reaction was completed, water was added to quench and extracted with ethyl acetate. The organic phase was washed with water and saturated sodium chloride solution in turn, dried over anhydrous sodium sulfate, filtered, and the solvent was removed under reduced pressure and 148mg of 1-cyclopropoxy-4-nitrobenzene was obtained as a yellow oil by silica gel column chromatography, yield 48%; ¹H NMR (400 MHz, CDCl₃) δ 8.24 - 8.17 (m, 2H), 7.15 ― 7.08 (m, 2H), 3.86-3.80 (m, 1H), 0.91 - 0.78 (m, 4H).

Step 2: 1-cyclopropoxy-4-nitrobenzene (145mg, 0.81 mmol) was dissolved in 6mL methanol, zinc powder (265mg, 4.05 mmol) and ammonium chloride (217mg, 4.05 mmol) were added sequentially, and the reaction was stirred at room temperature for 2 hours. After the reaction was completed, the reaction solution was filtered through diatomite, the solvent was removed under reduced pressure, and the residue was subjected to silica gel column chromatography to obtain 84mg of 1-cyclopropoxy-4-aniline as a yellow oil with a yield of 69%.

Step 3: the preparation method was the same as that in the example, and 24mg of 1-cyclopropoxy-4-phenylazide was obtained as a red brown oil with a yield of 25%; ¹H NMR (400 MHz, CDCl₃) δ 7.06 ― 7.00 (m, 2H), 6.98 - 6.92 (m, 2H), 3.75 - 3.66 (m, 1H), 0.77 (ddd, *J* = 6.4, 3.9, 2.5 Hz, 4H).

Step 4: 1-cyclopropoxy-4-phenylazide and intermediate 6 were used as raw materials, the preparation method was the same as that of synthetic route 1 and Example 1, and 39mg of 3-(4-((1-(4-cyclopropoxyphenyl)-1*H*-1, 2, 3-triazol-4-(methoxy)-1-oxoisoindolin-2-) piperidine-2, 6-dione was obtained as a white solid, yield 70%; ¹H NMR (400 MHz, DMSO) δ 10.97 (s, 1H), 8.86 (s, 1H), 7.81 (d, *J* = 9.0 Hz, 2H), 7.57 - 7.45 (m, 2H), 7.36 (d, *J* = 6.3 Hz, 1H), 7.25 (d, *J* = 9.0 Hz, 2H), 5.39 (s, 2H), 5.11 (dd, *J* = 13.3, 5.1 Hz, 1H), 4.39 (d, *J* = 17.5 Hz, 1H), 4.23 (d, *J* = 17.5 Hz, 1H), 3.93 (tt, *J* = 6.0, 2.9 Hz, 1H), 2.97 - 2.84 (m, 1H), 2.62 - 2.53 (m, 1H), 2.42 (ddd, *J* = 26.4, 13.3, 4.4 Hz, 1H), 2.01-1.92 (m, 1H), 0.85-0.79 (m, 2H), 0.72-0.67 (m, 2H). UPLC-MS (ESI) calculated for C₂₅H₂₃N₅O₅ [M + H]⁺: 474.17, found 474.27.

### EXAMPLE 33: 3-(4-((1-(4-(2-hydroxyethyl) phenyl)-1H-1, 2, 3-triazol-4-(methoxy)-1-oxoisoindolin-2-) piperidine-2, 6-dione (33)

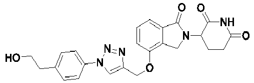

Step 1: the preparation method was the same as that of synthesis method 1 of azides, and 228mg of 2-(4-azidophenyl)-1-ethanol was obtained as a yellow oil, yield 96%; ¹H NMR (400 MHz, CDC13) δ 8.93 (s, 1H), 8.10 (d, J = 8.7 Hz, 1H), 7.60 (d, J = 2.2 Hz, 1H), 7.20 (dd, J = 8.7, 2.2 Hz, 1H),

Step 2: 2-(4-azidophenyl)-1-ethanol and intermediate 6 were used as raw materials, the preparation method was the same as that of synthetic route 1 and Example 1, and 35mg of 3-(4-((1-(4-(2-hydroxyethyl) phenyl)-1*H*-1, 2, 3-triazol-4-(methoxy)-1-oxoisoindolin-2-) piperidine-2, 6-dione was obtained as a white solid, yield 57%; ¹H NMR (400 MHz, DMSO) δ 10.97 (s, 1H), 8.93 (s, 1H), 7.80 (d, *J* = 8.4 Hz, 2H), 7.56 - 7.51 (m, 1H), 7.50 (d, *J* = 7.4 Hz, 1H), 7.45 (d, *J* = 8.4 Hz, 2H), 7.36 (d, *J* = 7.0 Hz, 1H), 5.40 (s, 2H), 5.11 (dd, *J* = 13.3, 5.0 Hz, 1H), 4.70 (t, *J* = 5.1 Hz, 1H), 4.39 (d, *J* = 17.5 Hz, 1H), 4.24 (d, *J* = 17.5 Hz, 1H), 3.66 (dd, *J* = 12.0, 6.7 Hz, 2H), 2.97 - 2.85 (m, 1H), 2.81 (t, *J* = 6.8 Hz, 2H), 2.64-2.52 (m, 1H), 2.49 - 2.36 (m, 1H), 2.03 - 1.94 (m, 1H). UPLC-MS (ESI) calculated for C₂₄H₂₃N₅O₅ [M + H]⁺: 462.17, found 462.27.

### EXAMPLE 34: (S)-3-methyl-3-(1-oxo-4-((1-(4-(trifluoromethoxyphenyl)-1H-1,2,3-triazol-4-) methoxy)isoindolin-2-)piperidine-2,6-dione (34)

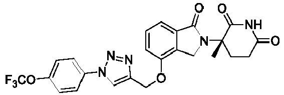

4- trifluoromethoxyphenyl azide and intermediate 8 were used as raw materials, the preparation method was the same as that of synthetic route 1 and Example 1, and 38mg of (S)-3-methyl-3-(1-oxo-4-((1-(4-(trifluoromethoxyphenyl)-1H-1,2,3-triazol-4-) methoxy)isoindolin-2-)piperidine-2,6-dione was obtained as a white solid, yield 77%; ¹H NMR (400 MHz, DMSO) δ 10.86 (s, 1H), 9.04 (s, 1H), 8.06 (d, *J* = 9.1 Hz, 2H), 7.65 (d, *J* = 8.6 Hz, 2H), 7.54 - 7.47 (m, 2H), 7.27 (dt, *J* = 6.2, 2.9 Hz, 1H), 5.42 (s, 2H), 4.65 (d, *J* = 17.6 Hz, 1H), 4.53 (d, *J* = 17.6 Hz, 1H), 2.79 - 2.57 (m, 3H), 1.87 (dt, *J* = 9.2, 4.3 Hz, 1H), 1.67 (s, 3H). UPLC-MS (ESI) calculated for C₂₄H₂₀F₃N₅O₅ [M + H]⁺: 516.14, found 516.28.

### EXAMPLE 35: (S)-2-(3-methyl-2, 6-dioxopiperidine-3-)-4-((1-(4-trifluoromethoxyphenyl)-1H-1, 2, 3-triazol-4-(methoxy) isoindolin-1, 3-dione (35)

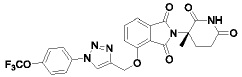

4- trifluoromethoxyphenyl azide and intermediate 12 were used as raw materials, the preparation method was the same as that of synthetic route 1 and Example 1, and 16mg of (S)-2-(3-methyl-2, 6-dioxopiperidine-3-)-4-((1-(4-trifluoromethoxyphenyl)-1*H*-1, 2, 3-triazol-4-(methoxy) isoindolin-1, 3-dione was obtained as a white solid, yield 22%; ¹H NMR (400 MHz, DMSO) δ 10.97 (s, 1H), 9.03 (s, 1H), 8.07 (d, *J* = 9.0 Hz, 2H), 7.87 - 7.80 (m, 1H), 7.74 (d, *J* = 8.5 Hz, 1H), 7.65 (d, *J* = 8.5 Hz, 2H), 7.43 (d, *J* = 7.1 Hz, 1H), 5.49 (s, 2H), 2.73 - 2.52 (m, 3H), 2.04 - 1.97 (m, 1H), 1.85 (s, 3H). UPLC-MS (ESI) calculated for C₂₄H₁₈F₃N₅O₆ [M + H]⁺: 530.12, found 530.22.

### EXAMPLE 36: (S)-3-(4-((1-(1-(2-methoxyethyl)-1H-indol-5-)-1H-1, 2, 3-triazol-4-) methoxy)-1-oxoisoindolin-2-)-3-methylpiperidine-2, 6-dione (36)

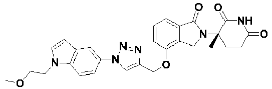

The preparation method was the same as that of Synthetic Route 1 and Example 1, and 24 mg of (*S*)-3-(4-((1-(1-(2-methoxyethyl)-1*H*-indol-5-)-1*H*-1, 2, 3-triazol-4-) methoxy)-1-oxoisoindolin-2-)-3-methylpiperidine-2, 6-dione was obtained as a white solid, yield 32%; ¹H NMR (400 MHz, DMSO) δ 10.86 (s, 1H), 8.91 (s, 1H), 8.02 (d, *J* = 1.9 Hz, 1H), 7.72 (d, *J* = 8.9 Hz, 1H), 7.62 (dd, *J* = 8.8, 2.1 Hz, 1H), 7.55 ― 7.47 (m, 3H),7.28-7.24(m, 1H), 6.58 (d, *J* = 3.1 Hz, 1H), 5.41 (s, 2H), 4.66 (d, *J* = 17.7 Hz, 1H), 4.54 (d, *J* = 17.6 Hz, 1H), 4.41 (t, *J* = 5.2 Hz, 2H), 3.68 (t, *J* = 5.2 Hz, 2H), 3.22 (s, 3H), 2.68 (dtd, *J* = 16.7, 12.2, 5.0 Hz, 3H), 1.87 (dt, *J* = 12.8, 4.5 Hz, 1H), 1.67 (s, 3H). UPLC-MS (ESI) calculated for C₂₈H₂₈N₆O₅ [M + H]⁺: 529.21, found 529.33.

### EXAMPLE 37: (S)-4-((1-(1-(2-methoxyethyl)-1H-indole-5-)-1H-1, 2, 3-triazol-4-)methoxy)-2-(3-methyl-2, 6-dioxopiperidine-3-) isoindolin-1, 3-dione (37)

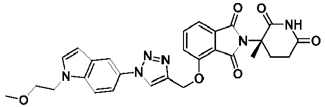

5-azido-1-(2-methoxyethyl)-1H-indole and intermediate 12 were used as raw materials, the preparation method was the same as that of Synthetic Route 1 and Example 1, and 14mg of product was obtained, yield 28%; ¹H NMR (400 MHz, DMSO) δ 10.97 (s, 1H), 8.89 (s, 1H), 8.03 (d, *J* = 1.9 Hz, 1H), 7.87 - 7.81 (m, 1H), 7.76 (d, *J* = 8.5 Hz, 1H), 7.71 (d, *J* = 8.9 Hz, 1H), 7.62 (dd, *J* = 8.8, 2.0 Hz, 1H), 7.52 (d, *J* = 3.1 Hz, 1H), 7.42 (d, *J* = 7.1 Hz, 1H), 6.58 (d, *J* = 3.0 Hz, 1H), 5.47 (s, 2H), 4.41 (t, *J* = 5.2 Hz, 2H), 3.68 (t, *J* = 5.2 Hz, 2H), 3.22 (s, 3H), 2.73 - 2.52 (m, 3H), 2.06 - 1.95 (m, 1H), 1.86 (s, 3H). UPLC-MS (ESI) calculated for C₂₈H₂₆N₆O₆ [M + H]⁺: 543.19, found 543.32.

### EXAMPLE 38: 2-(2, 6-dioxopiperidine-3-)-4-((1-(4-trifluoromethoxyphenyl)-1H-1, 2, 3-triazol-4-) methoxy) isoindolin-1, 3-dione (38)

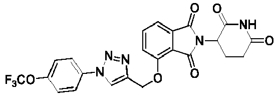

4- trifluoromethoxyphenyl azide and intermediate were used as raw materials, the preparation method was the same as that of Example 1, and 63.2mg of 2-(2, 6-dioxopiperidine-3-)-4-((1-(4-trifluoromethoxyphenyl)-1*H*-1, 2, 3-triazol-4-) methoxy) isoindolin-1, 3-dione was obtained as a white solid, yield 42%; ¹H NMR (400 MHz, DMSO) δ 11.13 (s, 1H), 9.05 (s, 1H), 8.07 (d, *J* = 9.0 Hz, 2H), 7.89 ― 7.83 (m, 1H), 7.78 (d, *J* = 8.6 Hz, 1H), 7.64 (d, *J* = 8.9 Hz, 2H), 7.50 (d, *J* = 7.2 Hz, 1H), 5.53 (s, 2H), 5.08 (dd, *J* = 12.8, 5.4 Hz, 1H), 2.88 (ddd, *J* = 16.9, 13.9, 5.3 Hz, 1H), 2.62 ― 2.53 (m, 1H), 2.49-2.40 (m, 1H), 2.05-1.98(m, 1H). UPLC-MS (ESI) calculated for C₂₃H₁₆F₃N₅O₆ [M + H]⁺: 516.11, found 516.17.

### Synthetic route 2:

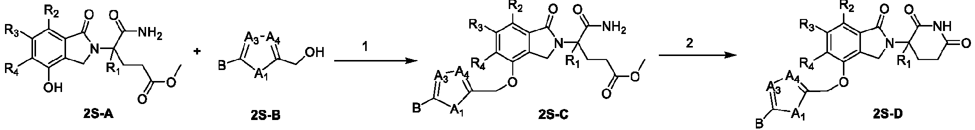

wherein R₁, R₂, R₃, R₄, A₁, A₃, A₄ and B have the same definitions as above;

Step 1: Methyl 5-amino-4-(4-hydroxy-1-oxoisoindolin-2-)-5-oxopentanoate (1 equivalent), alcohol derivative (2 equivalents), and triphenylphosphine (2 equivalents) were dissolved in dry tetrahydrofuran, DIAD (2 equivalents) was added dropwise under the condition of nitrogen protection, and reacted overnight at room temperature. After the reaction was completed, concentrated under reduced pressure, and purified by separation on flash column chromatography to obtain 2S-C.

Step 2: 1C (1 equivalent) obtained in the previous step was dissolved in dry tetrahydrofuran, and cooled sufficiently at 0 °C, potassium tert-butoxide (1.05 eq.) was addedand, reacted for 15 minutes at 0 °C, quenched with 1N HCl, diluted with water, extracted with ethyl acetate, the organic layer was washed with water and saturated sodium chloride sequentially, dried over anhydrous sodium sulfate, the solvent was removed under reduced pressure, and the residue was purified by HPLC to obtain the product **2S-D**.

### EXAMPLE 39: 3-(1-oxo-4-((5-(4-trifluoromethoxyphenyl) thiazol-2-) methoxy) isoindolin-2-) piperidine-2, 6-dione (39)

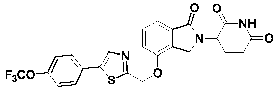

Step 1: 4-bromotrifluoromethoxybenzene (850mg, 3.53 mmol), thiazole (200mg, 2.35 mmol), palladium acetate (26mg, 0.118 mmol) and tetrabutylammonium acetate (1.42 g, 4.7 mmol)was dissolved in 20ml DMA, heated to 70°C under nitrogen protection and reacted for 24h.After the reaction was completed, the reaction solution was cooled to room temperature, diluted with ethyl acetate, filtered with diatomite, the filtrate was concentrated under reduced pressure, and 230mg of 5-(4-trifluoromethoxyphenyl) thiazole was obtained by column chromatography with a yield of 40%; ¹H NMR (400 MHz, CDCl₃) δ 8.79 (s, 1H), 8.07 (s, 1H), 7.61 (d, *J* = 8.8 Hz, 2H), 7.28 (s, 2H).

Step 2: 5-(4-trifluoromethoxyphenyl) thiazole (137mg, 0.56 mmol) was dissolved in 20ml of dry tetrahydrofuran under the protection of nitrogen, and the reaction solution was cooled to -78°C, n-butyl lithium (2.5 mol/L, 0.25 mL, 0.62 mmol) was added dropwise. The reaction was continued with stirring for 30min, DMF (48ul, 0.62 mmol) was added to the reaction solution, and the reaction solution was continued to react for 1h at -78°C, then raised to room temperature and reacted for 2h. After the reaction was completed, the reaction solution was adjusted to pH5 with 1N HCl, extracted with ethyl acetate, the organic phase was washed with saturated sodium chloride, dried over anhydrous sodium sulfate, filtered, and concentrated under reduced pressure to obtain 139mg of the product with a yield of 91%. ¹H NMR (400 MHz, CDCl₃)δ 9.97 (s, 1H), 8.25 (s, 1H), 7.69 (d, *J* = 8.8 Hz, 2H), 7.32 (d, *J* = 8.1 Hz, 2H).

Step 3: 5-(4-trifluoromethoxyphenyl) thiazole-2-carboxaldehyde (132mg, 0.48 mmol) was dissolved in a mixed solution of 6mL methanol and 6mL tetrahydrofuran, sodium borohydride (18mg, 0.48 mmol) was added under ice bath cooling, and the reaction solution was raised to room temperature and reacted for 1h. After the reaction was completed, the reaction solution was quenched with water, the solvent was removed under reduced pressure, diluted with ethyl acetate, washed with water and saturated sodium chloride in turn, dried over anhydrous sodium sulfate, filtered, concentrated under reduced pressure, and purified by column chromatography to obtain 103mg of 5-(4-trifluoromethoxyphenyl) thiazole-2-methanol with a yield of 77%; ¹H NMR (400 MHz, DMSO) δ 8.15 (s, 1H), 7.79 (d, *J* = 8.8 Hz, 2H), 7.44 (d, *J* = 8.1 Hz, 2H), 6.17 (t, *J* = 5.9 Hz, 1H), 4.74 (d, *J* = 5.9 Hz, 2H).

Step 4: 5-(4-trifluoromethoxyphenyl) thiazole-2-methanol and intermediate 4 were used as raw materials, the preparation method was the same as that of synthetic route 2, and 24mg of 3-(1-oxo-4-((5-(4-trifluoromethoxyphenyl) thiazol-2-) methoxy) isoindolin-2-) piperidine-2, 6-dione was obtained as a white solid, yield 42%; ¹H NMR (400 MHz, DMSO) δ 11.01 (s, 1H), 8.29 (s, 1H), 7.83 (d, *J* = 8.8 Hz, 2H), 7.54 (t, *J* = 7.8 Hz, 1H), 7.49 - 7.38 (m, 4H), 5.63 (s, 2H), 5.14 (dd, *J* = 13.3, 5.1 Hz, 1H), 4.47 (d, *J* = 17.5 Hz, 1H), 4.32 (d, *J* = 17.5 Hz, 1H), 2.98 - 2.87 (m, 1H), 2.63 - 2.55 (m, 1H), 2.48-2.42 (m, 1H), 2.07 - 1.95 (m, 1H). UPLC - MS (ESI) calculated for C₂₄H₁₈F₃N₃O₅S [M + H]⁺: 518.09, found: 518.08.

### EXAMPLE 40: 3-(1-oxo-4-((5-(4-trifluoromethoxyphenyl) oxazol-2-) methoxy) isoindolin-2-) piperidine-2, 6-dione (40)

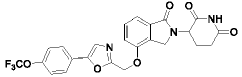

Step 1: 4-trifluoromethoxybenzaldehyde (800mg, 4.21 mmol) was dissolved in 20mL of methanol, 4-methylbenzenesulfonyl methyl isonitrile (904mg, 4.63 mmol) was added under stirring conditions and heated to reflux for 1h. After the reaction was completed, concentrated under reduced pressure to remove the solvent, saturated sodium bicarbonate aqueous solution was added to the residue, extracted with dichloromethane, the organic layer was washed with water and saturated sodium chloride successively, dried, filtered, the solvent was removed under reduced pressure, and the residue was subjected to column chromatography to obtain 887mg of 4-trifluoromethoxyphenyl oxazole as a yellow solid with a yield of 82%; ¹H NMR (400 MHz, CDCl₃)δ 7.93 (s, 1H), 7.69 (d, *J* = 8.9 Hz, 2H), 7.36 (s, 1H), 7.28 (d, *J* = 8.2 Hz, 2H).

Step 2: 4-trifluoromethoxyphenyl oxazole (879mg, 3.84mmol) was dissolved in 30ml of dry THF under the protection of nitrogen, and the reaction solution was cooled to -78°C, n-butyl lithium (2.5 mol/L, 0.25 mL, 4.22mmol) was added dropwise. The reaction was continued for 30min, DMF (325ul, 4.22mmol) was added to the reaction solution, and the reaction solution was continued to react for 1h at -78°C, then raised to room temperature and reacted for 2h. After the reaction was completed, the reaction solution was adjusted to pH5 with 1N HCl, extracted with ethyl acetate, the organic phase was washed with saturated sodium chloride, dried over anhydrous sodium sulfate, filtered, and concentrated under reduced pressure to obtain the crude product, which was directly used in the next step.

Step 3: the crude product of the previous step was dissolved in a mixed solution of lOmL methanol and lOmL THF, sodium borohydride (145mg, 3.84 mmol) was added under ice bath cooling, the reaction solution was raised to room temperature and reacted for 1h. After the reaction was completed, water was added to quench, the solvent was removed under reduced pressure, the residue was diluted with ethyl acetate, washed with water and saturated sodium chloride in turn, dried over anhydrous sodium sulfate, filtered, concentrated under reduced pressure and subjected to silica gel column chromatography to obtain 450mg of 5-(4-trifluoromethoxyphenyl) oxazol-2- methanol with a total yield of 45% for two steps; ¹H NMR (400 MHz, DMSO) δ 7.83 (d, *J* = 8.8 Hz, 2H), 7.69 (s, 1H), 7.49 (d, *J* = 8.2 Hz, 2H), 5.75 (t, *J* = 6.2 Hz, 1H), 4.56 (d, *J* = 6.2 Hz, 2H).

Step 4: 5-(4-trifluoromethoxyphenyl) oxazol-2-methanol and intermediate 4 were used as raw materials, the preparation method was the same as that of synthetic route 2, and 15mg of 3-(1-oxo-4-((5-(4-trifluoromethoxyphenyl) oxazol-2-) methoxy) isoindolin-2-) piperidine-2, 6-dione was obtained, yield 30%; ¹H NMR (400 MHz, DMSO) δ 10.98 (s, 1H), 7.89 - 7.79 (m, 3H), 7.56 - 7.43 (m, 4H), 7.38 (d, *J* = 7.3 Hz, 1H), 5.47 (s, 2H), 5.11 (dd, *J* = 13.3, 5.0 Hz, 1H), 4.42 (d, *J* = 17.5 Hz, 1H), 4.26 (d, *J* = 17.5 Hz, 1H), 2.94 - 2.80 (m, 1H), 2.59-2.52 (m, 1H), 2.47 - 2.36 (m, 1H), 2.02 - 1.93 (m, 1H). UPLC - MS (ESI) calculated for C₂₄H₁₈F₃N₃O₆ [M + H]⁺: 502.11, found: 502.22.

### EXAMPLE 41: 3-(1-oxo-4-((5-(4-trifluoromethoxyphenyl)-1, 3, 4-thiadiazol-2-) methoxy) isoindolin-2-) piperidine-2, 6-dione (41)

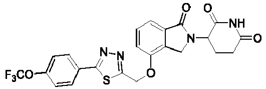

Step 1: ethyl 2-oxo-2-(2-(4-trifluoromethoxybenzoyl hydrazide)) acetate (300mg, 1.03 mmol) was dispersed in 30ml of dry toluene, phosphorus pentasulfide (522mg, 2.73 mmol) was added to the reaction solution, and the reaction was refluxed for 1.5 h. After the reaction was completed, the reaction solution was cooled to room temperature, diluted with ethyl acetate, the organic phase was washed with water, saturated sodium bicarbonate and saturated sodium chloride solution in turn, dried over anhydrous sodium sulfate, filtered, concentrated under reduced pressure, and subjected to silica gel column chromatography to obtain 200mg of a white solid with a yield of 61%. ¹H NMR (400 MHz, CDCl₃)δ 8.08 (d, *J* = 8.9 Hz, 2H), 7.37 (d, *J* = 8.1 Hz, 2H), 4.55 (q, *J* = 7.1 Hz, 2H), 1.48 (t, *J* = 7.1 Hz, 3H).

Step 2: ethyl 5-(4-trifluoromethoxyphenyl)-1,3,4-thiadiazol-2-carboxylate (200mg, 0.63mmol) was dissolved in a mixed solution of (15mL) methanol and THF (15mL), sodium borohydride (71mg, 1.885mmol) was added under ice bath cooling, the reaction solution was raised to room temperature and reacted for overnight. After the reaction was completed, water was added to quench, the solvent was removed under reduced pressure, the residue was dissolved with ethyl acetate, washed with water and saturated sodium chloride in turn, dried over anhydrous sodium sulfate, filtered, concentrated under reduced pressure and subjected to silica gel column chromatography to obtain 145mg of 5-(4-trifluoromethoxyphenyl)-1,3,4-thiadiazol-2-methanol as a white solid, yield 84%; ¹H NMR (400 MHz, CDCl₃) δ 8.08 (d, *J* = 8.9 Hz, 2H), 7.37 (d, *J* = 8.1 Hz, 2H), 4.55 (q, *J* = 7.1 Hz, 2H), 1.48 (t, *J* = 7.1 Hz, 3H).

Step 3: the preparation method was the same as the synthesis route 2, 8mg, yield 56%; ¹H NMR (400 MHz, DMSO) δ 10.99 (s, 1H), 8.15 (d, *J* = 8.9 Hz, 2H), 7.60 - 7.51 (m, 3H), 7.47 (d, *J* = 7.7 Hz, 1H), 7.41 (d, *J* = 7.0 Hz, 1H), 5.81 (s, 2H), 5.13 (dd, *J* = 13.2, 5.0 Hz, 1H), 4.45 (d, *J* = 17.5 Hz, 1H), 4.30 (d, *J* = 17.5 Hz, 1H), 2.97 - 2.86 (m, 1H), 2.62 - 2.55 (m, 1H), 2.48 - 2.38 (m, 1H), 2.05 - 1.96 (m, 1H). UPLC - MS (ESI) calculated for C₂₃H₁₇F₃N₄O₅S [M + H]⁺: 519.09, found: 519.26.

### EXAMPLE 42: 3-(1-oxo-4-((5-(4-trifluoromethoxyphenyl) furan-2-) methoxy) isoindolin-2-) piperidine-2, 6-dione (42)

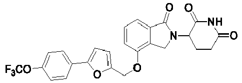

Step 1: p-4-trifluoromethoxyphenylboronic acid (500 mg, 2.43 mmol), 5-bromofuran-2-carboxaldehyde (425 mg, 2.43 mmol), Pd (dppf) Cl₂ (35.6 mg, 0.049 mmol), and sodium carbonate (773 mg, 7.29 mmol) were added into a 50 mL two-necked flask. After replacing the gas three times, 15 mL of toluene, 3.5 mL of ethanol, 3.5 mL of water were added, replaced gas once. The reaction system was refluxed overnight under the protection of nitrogen, dried under reduced pressure, diluted with ethyl acetate, washed the organic phase with water, extracted the aqueous layer with ethyl acetate once again, combined the organic layers, washed with saturated sodium chloride once, dried over anhydrous sodium sulfate, filtered, concentrated under reduced pressure, and chromatographed on silica gel column to obtain 5-(4-trifluoromethoxyphenyl) furan-2-formaldehyde as a light yellow solid 474 mg, with a yield of 76%; ¹H NMR (400 MHz, CDCl₃) δ 9.67 (s, 1H), 7.88 - 7.82 (m, 2H), 7.33 (d, *J* = 3.7 Hz, 1H), 7.30 (d, *J* = 8.1 Hz, 2H), 6.85 (d, *J* = 3.7 Hz, 1H).

Step 2: 5-(4-trifluoromethoxyphenyl) furan-2-formaldehyde (469 mg, 1.83 mmol) was dissolved in 20 mL of methanol and NaBH4 (41.613 mg, 1.1 mmol) was added under stirring, reacted at room temperature for 2h, concentrated under reduced pressure, the residue was dissolved in ethyl acetate, washed once with 1N HCl, the aqueous layer was extracted once with ethyl acetate, the organic layers are combined, washed once with water, saturated sodium bicarbonate and saturated sodium chloride, dried over anhydrous sodium sulfate, filtered, and concentrated under reduced pressure to give 5-(4-trifluoromethoxyphenyl) furan-2-methanol 472mg, yield 100%; ¹H NMR (400 MHz, CDCl₃) δ 7.71 ― 7.63 (m, 2H), 7.23 (d, *J* = 8.7 Hz, 2H), 6.60 (d, *J* = 3.3 Hz, 1H), 6.39 (d, *J* = 3.3 Hz, 1H), 4.67 (d, *J* = 4.3 Hz, 2H).

Step 3: 5-(4-trifluoromethoxyphenyl) furan-2-methanol and intermediate 4 were used as raw materials, the preparation method was the same as that of synthetic route 2, and 10.2 mg of 3-(1-oxo-4-((5-(4-trifluoromethoxyphenyl) furan-2-) methoxy) isoindolin-2-) piperidine-2, 6-dione was obtained, yield 22.6 %; ¹H NMR (400 MHz, DMSO) δ 10.94 (s, 1H), 7.85 ― 7.80 (m, 2H), 7.56 - 7.50 (m, 1H), 7.45 (m, , 3H), 7.36 (d, *J* = 6.7 Hz, 1H), 7.03 (d, *J* = 3.4 Hz, 1H), 6.78 (d, *J* = 3.4 Hz, 1H), 5.30 (s, 2H), 5.09 (dd, *J* = 13.3, 5.2 Hz, 1H), 4.37 (d, *J* = 17.5 Hz, 1H), 4.21 (d, *J* = 17.5 Hz, 1H), 2.89 (ddd, *J* = 17.5, 13.5, 5.4 Hz, 1H), 2.59 - 2.52 (m, 1H), 2.42 (ddd, *J* = 18.1, 13.6, 4.9 Hz, 1H), 1.99 - 1.90 (m, 1H). UPLC - MS (ESI) calculated for C₂₅H₁₉F₃N₂O₆ [M + H]⁺: 501.12, found: 501.24.

### EXAMPLE 43: 3-(1-oxo-4-((5-(4-trifluoromethoxyphenyl)-1, 3, 4-oxadiazol-2-) methoxy) isoindolin-2-) piperidine-2, 6-dione (43)

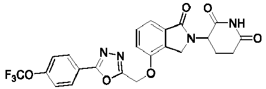

Step 1: A solution of methyl 4-trifluoromethoxybenzoate (3.7 g, 16.8 mmol) and 85% hydrazine hydrate (3.96 g, 67.23 mmol) in methanol was heated to reflux overnight, cooled, concentrated under reduced pressure, and the resulting solid was washed with a small amount of diethyl ether to give analytically pure 4-trifluoromethoxybenzoyl hydrazine (3.3 g, yield 88%); ¹H NMR (400 MHz, DMSO) δ 9.90 (s, 1H), 8.01 - 7.88 (m, 2H), 7.45 (d, *J* = 8.1 Hz, 2H), 4.54 (s, 2H).

Step 2: at 0°C, ethyl oxalyl chloride (1.65 mL, 14.80 mmol) was added dropwise to 4-trifluoromethoxybenzoyl hydrazide (3.26 g, 14.80 mmol) in dichloromethane (55 mL) suspension under N2 protection conditions, continued to react at 0°C for 0.5 h, then raised to room temperature for 1 h. The obtained reaction solution was washed with saturated sodium bicarbonate, the organic phase was dried with anhydrous sodium sulfate, filtered and concentrated under reduced pressure to obtain 3.6 g of yellow solid, which was directly used in the next step.

Step 3: at 0°C, pyridine (614 µL, 7.5 mmol) was added to the toluene (50 ml) suspension of the product (2.0 g, 6.25 mmol) obtained in step 2, and then SOCl₂ (1.36 mL, 18.74 mmol) was added dropwise. After the addition, the reaction solution was heated to reflux overnight, concentrated under reduced pressure to remove the solvent, the obtained solid residue was dissolved in dichloromethane, the organic phase was washed with saturated sodium bicarbonate, dried over anhydrous sodium sulfate, filtered, concentrated under reduced pressure, and the obtained residue was subjected to silica gel column chromatography to obtain ethyl 2-(4-trifluoromethoxyphenyl)-5-carboxylate 1.48 g, yield 78%; ¹H NMR (400 MHz, CDCl₃)δ 8.36 - 8.09 (m, 2H), 7.39 (d, *J* = 8.2 Hz, 2H), 4.56 (q, *J* = 7.1 Hz, 2H), 1.49 (t, *J* = 7.1 Hz, 3H).

**Step 4**: At 0°C, sodium borohydride (156mg, 4.13mmol) was added to ethyl 2-(4-trifluoromethoxyphenyl)-5-carboxylate (500mg, 1.65mmol) in the mixed solution of methanol (8ml) and tetrahydrofuran (8ml), stirred and reacted for 10min, warmed to room temperature and reacted overnight. After the reaction was completed, quenched with water, concentrated under reduced pressure, diluted with ethyl acetate, washed with water and saturated sodium chloride solution in turn, and removed the solvent under reduced pressure to obtain 430mg of white solid by fast silica gel column chromatography, yield 100%.

Step 4: 2-(4-trifluoromethoxyphenyl) 5-methanol and intermediate 4 were used as raw materials, the preparation method was the same as that of synthetic route 2, and 32.9 mg of 3-(1-oxo-4-((5-(4-trifluoromethoxyphenyl)1,3,4-oxadiazol-2-) methoxy) isoindolin-2-) piperidine-2, 6-dione was obtained, yield 46 %; ¹H NMR (400 MHz, DMSO) δ 10.97 (s, 1H), 8.18 - 8.12 (m, 2H), 7.62 (d, *J* = 8.2 Hz, 2H), 7.58 ― 7.52 (m, 1H), 7.49 (d, *J* = 7.6 Hz, 1H), 7.41 (d, *J* = 7.3 Hz, 1H), 5.67 (s, 2H), 5.11 (dd, *J* = 13.3, 5.1 Hz, 1H), 4.44 (d, *J* = 17.5 Hz, 1H), 4.29 (d, *J* = 17.5 Hz, 1H), 2.91 (ddd, *J* = 17.5, 13.6, 5.3 Hz, 1H), 2.60-2.54 (m, 1H), 2.47 - 2.35 (m, 1H), 2.03 - 1.93 (m, 1H). UPLC - MS (ESI) calculated for C₂₃H₁₇F₃N₄O₆ [M + H]⁺: 503.11, found:503.75.

### EXAMPLE 44: 3-(1-oxo-4-((2-(4-trifluoromethoxyphenyl) thiazol-5-) methoxy) isoindolin-2-) piperidine-2, 6-dione (44)

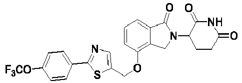

Step 1: Ethyl 2-bromothiazol-5-carboxylate(500mg, 2.12mmol), 4-trifluoromethoxyphenylboronic acid (665 mg, 3.18 mmol), sodium carbonate (450 mg, 4.24 mmol), tetrakis(triphenylphosphine) palladium (245 mg, 0.212 mmol) was added to a 100 ml two-necked flask, toluene (30 ml) and water (5 ml) were added, and refluxed overnight under the protection of N2. After the reaction was completed, diluted with water, extracted with ethyl acetate, the aqueous layer was extracted with ethyl acetate once again, combined the organic layers, washed with saturated NaCl, dried over anhydrous sodium sulfate, filtered, concentrated under reduced pressure, and chromatography on silica gel column to obtain the product ethyl 2-(4-trifluoromethoxyphenyl) thiazol-5-carboxylate (white solid, 335mg, yield 69%); ¹H NMR (400 MHz, CDCl₃)δ 8.42 (s, 1H), 8.03 (d, *J* = 8.8 Hz, 2H), 7.32 (d, *J* = 8.8 Hz, 2H), 4.40 (q, *J* = 7.1 Hz, 2H), 1.41 (t, *J* = 7.1 Hz, 3H).

Step 2: at 0°C, LiAlH₄ (2.2 mL, 2.2 mmol) was added in portions to a THF solution of 2-(4-trifluoromethoxyphenyl)thiazole-5-carboxylic acid ethyl ester (460 mg, 1.45 mmol). After 15min, warmed to room temperature and reacted for 1.5h, quenched by adding water, filtered, spin- dried, column chromatography. 399mg of yellow solid was obtained, yield 100%; ¹H NMR (400 MHz, CDCl₃)δ 7.99 ― 7.92 (m, 2H), 7.72 (s, 1H), 7.29 (d, *J* = 8.1 Hz, 2H), 4.91 (d, *J* = 5.0 Hz, 2H), 2.01 (t, *J* = 5.0Hz, 1H).

Step 3: 2-(4-trifluoromethoxyphenyl) thiazole-5-methanol and intermediate 6 were used as raw materials, and the preparation method was the same as the synthesis route 2 to obtain 67.7 mg of 3-(1-oxo-4-((2-(4-trifluoromethoxyphenyl) thiazol-5-) methoxy) isoindolin-2-) piperidine-2, 6-dione as a white solid, yield 52%; 1H NMR (400 MHz, DMSO) δ 10.97 (s, 1H),8.08-8.04 (m, 3H), 7.55-7.49 (m, 3H), 7.45 (d, J = 7.8 Hz, 1H), 7.37 (d, J = 7.3 Hz, 1H), 5.58 (s, 2H), 5.11 (dd, J = 13.3, 5.0 Hz, 1H), 4.39 (d, J = 17.4 Hz, 1H), 4.24 (d, J = 17.4 Hz, 1H), 2.96 - 2.83 (m, 1H), 2.60-2.54(m, 1H), 2.47 - 2.37 (m, 1H), 2.03 - 1.92 (m, 1H). UPLC - MS (ESI) calculated for C₂₄H₁₈F₃N₃O₅S [M + H]⁺: 518.09, found: 518.08.

### EXAMPLE 45: 3-(1-oxo-4-((2-(4-trifluoromethoxyphenyl) oxazol-5-) methoxy) isoindolin-2-) piperidine-2, 6-dione (45)

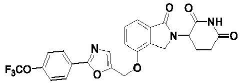

Step 1: a solution of LHMDS (1mol/L, 7.44 ml, 7.44 mmol) in tetrahydrofuran was added to a solution of ethyl oxazole-5-carboxylate (1g, 7.09 mmol) in tetrahydrofuran (25 mL) dropwise at -78°C. After 1h, a solution of diiodoethane (2.31 g, 8.184 mmol) in tetrahydrofuran (10 ml) was added dropwise, reacted at the same temperature for 1h, warmed to room temperature for reaction, monitored by TLC, after the reaction was completed, 100ml of cold ether and saturated sodium thiosulfate were added, extracted and separated, washed the organic layer once with saturated sodium chloride, spin-dried, and column chromatography. Ethyl 2-iodinoxole-5-carboxylate was obtained (white solid, 1.5 g, yield 50%). ¹H NMR (400 MHz, CDCl₃)δ 7.65 (s, 1H), 4.39 (q, *J* = 7.1 Hz, 2H), 1.38 (t, *J* = 7.1 Hz, 3H).

Step 2: Ethyl 2-iodooxazol-5-carboxylate (800 mg, 3 mmol), 4-trifluoromethoxyphenylboronic acid (618 mg, 4.5 mmol), potassium carbonate (1.24 mg, 9 mmol), Pd( PPh3)4 (347 mg, 0.3 mmol) were added to a 100 ml two-necked flask, dioxane (20 mL) and water (3 mL) were added, refluxed overnight under N2 protection, diluted with water, extracted with ethyl acetate (EA), and the water layer was extracted with EA once, combined the organic layers, washed with saturated NaCl, dried, spin-dried, column chromatography. 2-(4-trifluoromethoxyphenyl)-oxazoe-5-carboxylic acid (476mg) was obtained as a hydrolysate; ¹H NMR (400 MHz, DMSO) δ 13.79 (s, 1H), 8.17 (d, *J* = 8.8 Hz, 2H), 8.06 (s, 1H), 7.59 (d, *J* = 8.3 Hz, 2H).

Step 3: At 0°C, a THF solution of borane (1M/L, 5.2mL, 5.2mmol) was added dropwise to 2-(4-trifluoromethoxyphenyl)-oxazole-5-carboxylic acid (474mg, 1.735 mmol) in THF (10 mL) solution, warmed to room temperature and reacted for 2h. After the reaction was completed, the excess borane was quenched with methanol, and spin-dried under reduced pressure, subjected to silica gel column chromatography to obtain 250 mg of white solid with a yield of 56%; ¹H NMR (400 MHz, DMSO) δ 8.13 - 8.02 (d, *J* = 8.1 Hz, 2H), 7.54 (d, *J* = 8.1 Hz, 2H), 7.22 (s, 1H), 5.49 (t, *J* = 5.8 Hz, 1H), 4.55 (d, *J* = 5.7 Hz, 2H).

Step 4: the preparation method was the same as the synthesis route 2 and Example 40, 32.6 mg of white solid was obtained, yield 27.8 %; ¹H NMR (400 MHz, DMSO) δ 10.95 (s, 1H), 8.15 - 8.07 (m, 2H), 7.56-7.52 (m, 4H), 7.48 (d, *J* = 7.5 Hz, 1H), 7.40 - 7.35 (m, 1H), 5.42 (s, 2H), 5.09 (dd, *J* = 13.3, 5.1 Hz, 1H), 4.39 (d, *J* = 17.5 Hz, 1H), 4.23 (d, *J* = 17.5 Hz, 1H), 2.95 - 2.83 (m, 1H), 2.61 ― 2.52 (m, 1H), 2.42 (ddd, *J* = 26.1, 13.2, 4.4 Hz, 1H), 2.00 ― 1.91 (m, 1H). UPLC - MS (ESI) calculated for C₂₄H₁₈F₃N₃O₆ [M + H]⁺: 502.11, found:502.25.

### EXAMPLE 46: 3-(4-(2-(benzo[d]thiazol-2-) thiazol-5-) methoxy)-1-oxoisoindolin-2-) piperidine-2, 6-dione (46)

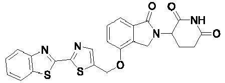

Step 1: A solution of benzothiazole (135.19 mg, 1mmol), 5-hydroxymethylthiazole (115.15 mg, 1mmol), copper acetate (218mg, 1.2 mmol) in DMSO (8ml) was heated to 130 °C under nitrogen protection and reacted for 16h, cooled to room temperature, diluted with ethyl acetate, filtered with diatomite, washed with water and saturated sodium chloride in turn, dried over anhydrous sodium sulfate, the solvent was removed under reduced pressure, 68mg of (2-(benzo[d] thiazol-2-) thiazol-5-) methanol was obtained by separation on flash column chromatography, yellow solid, yield 27%; ¹H NMR (400 MHz, DMSO) δ 8.19 (d, *J* = 7.8 Hz, 1H), 8.11 (d, *J* = 7.7 Hz, 1H), 7.96 ― 7.87 (m, 1H), 7.63 ― 7.49 (m, 2H), 5.81 (t, *J* = 5.7 Hz, 1H), 4.79 (d, *J* = 5.7 Hz, 2H).

Step 2: (2-(benzo [d] thiazol-2-) thiazol-5-) methanol and intermediate 4 were used as raw materials, the preparation method was the same as that of synthetic route 2 and 23 mg of 3-(4-(2-(benzo[*d*] thiazol-2-) thiazol-5-) methoxy)-1-oxoisoindolin-2-) piperidine-2, 6-dione was obtained, yield 32%; ¹H NMR (400 MHz, DMSO) δ 10.97 (s, 1H), 8.20 (d, *J* = 7.9 Hz, 2H), 8.11 (d, *J* = 7.9 Hz, 1H), 7.60 (dd, *J* = 11.1, 4.1 Hz, 1H), 7.54 (t, *J* = 7.6 Hz, 2H), 7.45 (d, *J* = 8.0 Hz, 1H), 7.38 (d, *J* = 7.4 Hz, 1H), 5.64 (s, 2H), 5.11 (dd, *J* = 13.3, 5.1 Hz, 1H), 4.43 (d, *J* = 17.5 Hz, 1H), 4.28 (d, *J* = 17.5 Hz, 1H), 2.97 - 2.84 (m, 1H), 2.63 - 2.54 (m, 1H), 2.48 - 2.39 (m, 1H), 2.04 - 1.91 (m, 1H). UPLC - MS (ESI) calculated for C₂₄H₁₈N₄O₄S₂ [M + H]⁺: 491.08, found:491.15.

### EXAMPLE 47: 3-(1-oxo-4-((5'-trifluoromethoxy-[2,2'-bithiazole]-5-) methoxy) isoindolin-2-) piperidine-2, 6-dione (47)

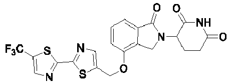

Step 1: 2-bromothiazole (1g, 6.10 mmol), palladium acetate (137mg, 0.61 mmol), tetrabutylammonium bromide (983mg, 3.05 mmol) and N, N-diisopropylethylamine (1ml, 6.10 mmol) were suspended in 15ml of toluene and heated to 105°C under nitrogen protection, stirred and reacted for 18h. After TLC monitored the reaction was completed, poured the reaction solution into water, extracted with ethyl acetate which was dried over anhydrous sodium sulfate, filtered, the solvent was removed under reduced pressure, and 385mg of bithiazole was obtained by separation on fast column chromatography, yellow solid, yield 37.5%; 1H NMR (400 MHz, CDCl3): δ 7.90 (d, 2H, J = 2.8 Hz), 7.45 (d, 2H, J = 2.8 Hz).

Step 2: 2, 2 '-bithiazole (375mg, 2.23 mmol) and NBS (1.59 g, 8.92 mmol) were dissolved in DMF (15ml) and heated to 60°C and reacted overnight. After the reaction was completed, the reaction solution was diluted with ethyl acetate, washed with water and saturated sodium chloride in turn, the solvent was removed under reduced pressure, and 612mg of 5, 5 '-dibromo-2, 2'-bithiazole was obtained by separation on flash column chromatography as a white solid, yield 84%;¹H NMR (400 MHz, CDCl₃) δ 7.75 (s, 2H).

Step 3: Under the condition of -78°C, n-butyl lithium (356ul, 0.889mmol) was added to 5,5' -dibromo-2,2' -bithiazole (276mg, 0.85mmol) in dry THF solution (25mL) dropwise under the protection of nitrogen. After reacting for 1h at -78°C, DMF (69ul, 0.89 mmol) was added. After TLC monitored that the reaction was completed, the reaction solution was quenched with 1N hydrochloric acid, extracted with ethyl acetate (50mL), the organic layer was washed with water and saturated sodium chloride in turn, dried over anhydrous sodium sulfate, filtered, the solvent was removed under reduced pressure, and 147mg of 5'-bromo-[2, 2'-bithiazole]-5-formaldehyde was obtained by silica gel column chromatography, a yellow solid, yield 63%;¹H NMR (400 MHz, CDCl₃) δ 10.09 (s, 1H), 8.42 (s, 1H), 7.87 (s, 1H).

Step 4: 5 '-bromo-[2, 2'-bithiazole]-5-formaldehyde (140mg, 0.51 mmol) was dissolved in 10ml DMF, methyl fluorosulfonyl difluoroacetate (227ul, 1.79 mmol) and cuprous iodide (29mg, 0.153 mmol) were added, and heated to 85°C and reacted for 18h, water (20mL) was added and extracted with ethyl acetate (60mL). The organic layer was washed with water and saturated sodium chloride in turn, dried over anhydrous sodium sulfate, filtered, and the solvent was removed under reduced pressure to obtain 95mg of yellow solid. The crude product was directly used in the next step.

Step 5: the crude product obtained in the previous step was dissolved in a mixed solution of 5mL tetrahydrofuran and 5mL methanol, sodium borohydride (19mg, 0.51 mmol) was added under the condition of ice bath cooling, the reaction solution was raised to room temperature for 1h, the reaction was completed, quenched with water, and then extracted with ethyl acetate (50ml). The ethyl acetate layer was washed with water and saturated sodium chloride solution in turn, dried over anhydrous sodium sulfate, filtered, the solvent was removed under reduced pressure, and purified by HPLC to obtain 28mg of white solid, with a two-step yield of 21%; ¹H NMR (400 MHz, CDCl₃) δ 8.13 (d, *J* = 1.0 Hz, 1H), 7.80 (s, 1H), 4.96 (dd, *J* =6.0, 1.0Hz, 2H), 2.05 (t, *J* = 6.0 Hz, 1H).

Step 6: (5' -trifluoromethyl)-[2,2' -bithiazole]-5-) methanol and intermediate 4 were used as raw materials, the preparation method was the same as that of synthetic route 2, and 29mg of 3-(1-oxo-4-((5 '-trifluoromethoxy-[2, 2'-bithiazole]-5-) methoxy) isoindolin-2-) piperidine-2, 6-dione was obtained as a white solid, yield 74%; ¹H NMR (400 MHz, DMSO) δ 10.96 (s, 1H), 8.63 (d, *J* = 1.1 Hz, 1H), 8.21 (s, 1H), 7.53 (t, *J* = 7.8 Hz, 1H), 7.44 (d, *J* = 8.0 Hz, 1H), 7.37 (d, *J* = 7.3 Hz, 1H), 5.63 (s, 2H), 5.10 (dd, *J* = 13.3, 5.1 Hz, 1H), 4.41 (d, *J* = 17.5 Hz, 1H), 4.26 (d, *J* = 17.5 Hz, 1H), 2.96 - 2.83 (m, 1H), 2.61 - 2.53 (m, 1H), 2.48 - 2.39 (m, 1H), 2.03 - 1.93 (m, 1H). UPLC - MS (ESI) calculated for C₂₁H₁₅F₃N₄O₄S₂ [M + H]⁺: 509.05, found:509.19.

### EXAMPLE 48: 3-(1-oxo-4-((1-((tetrahydro-2H-pyran-4-) methyl)-1H-1, 2, 3-triazol-4-) methoxy) isoindolin-2-) piperidine-2, 6-dione (48)

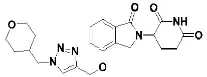

Step 1: sodium azide (218mg, 3.35 mmol) was added to a solution of 4-bromomethyltetrahydropyran (0.3 g, 1.68 mmol) in DMF (8mL) and reacted overnight at room temperature. After the reaction was completed, the reaction solution was diluted with ethyl acetate (50mL), the organic phase was washed with water and saturated aqueous sodium chloride solution sequentially, dried over anhydrous sodium sulfate, filtered, the solvent was removed under reduced pressure, and 227mg of 2*H*-tetrahydropyran-4-methylazide was obtained as a colorless oil, yield 96%; ¹H NMR (400 MHz, CDCl₃)δ 3.98 (dd, J = 11.4, 4.4 Hz, 2H), 3.38 (td, J = 11.9, 1.9 Hz, 2H), 3.18 (d, J = 6.8 Hz, 2H), 1.86 - 1.73 (m, 1H), 1.65 (dd, J = 13.0, 1.7 Hz, 2H), 1.34 (ddd, J = 25.1, 12.2, 4.5 Hz, 2H).

Step 2: 2*H*-tetrahydropyran-4-methylazide and intermediate 6 were used as raw materials, and the preparation method was the same as that of Synthetic Route 1 and Example 1, and 6 mg of 3-(1-oxo-4-((1-((tetrahydro-2*H*-pyran-4-) methyl)-1H-1, 2, 3-triazol-4-) methoxy) isoindolin-2-) piperidine-2, 6-dione was obtained as a white solid, yield 14%; ¹H NMR (400 MHz, DMSO) δ 10.95 (s, 1H), 8.24 (s, 1H), 7.53 - 7.47 (m, 1H), 7.44 (dd, *J* = 8.1, 0.7 Hz, 1H), 7.34 (dd, *J* = 7.3, 0.7 Hz, 1H), 5.30 (s, 2H), 5.10 (dd, *J* = 13.3, 5.1 Hz, 1H), 4.34 (d, *J* = 17.5 Hz, 1H), 4.28 (d, *J* = 7.2 Hz, 2H), 4.18 (d, *J* = 17.5 Hz, 1H), 3.82 (dd, *J* = 11.2, 3.0 Hz, 2H), 3.23 (td, *J* = 11.6, 2.1 Hz, 2H), 2.90 (ddd, *J* = 17.5, 13.5, 5.3 Hz, 1H), 2.61 - 2.54 (m, 1H), 2.42 (ddd, *J* = 26.2, 13.1, 4.3 Hz, 1H), 2.13 - 2.01 (m, 1H), 2.00 - 1.93 (m, 1H), 1.41 - 1.31 (m, 2H), 1.29 - 1.17 (m, 2H). UPLC - MS (ESI) calculated for C₂₂H₂₅N₅O₅ [M + H]⁺: 440.19, found:440.44.

### EXAMPLE 49: 3-(1-oxo-4-(1-phenethyl-1H-1, 2, 3-triazol-4-(methoxy) isoindolin-2-) piperidine-2, 6-dione (49)

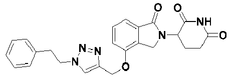

Step 1: The preparation method of 2-phenylethyl azide was the same as that of synthetic method 3 of azides, and 228 mg was obtained as a colorless oil with a yield of 77.5%; ¹H NMR (400 MHz, CDCl₃) δ 7.33 (m, 2H), 7.27 (d, *J* = 1.4 Hz, 1H), 7.23 (m, 2H), 3.51 (t, *J* = 7.3 Hz, 2H), 2.90 (t, *J* = 7.3 Hz, 2H).

Step 2: 2-phenylethyl azide and intermediate 6 were used as raw materials, the preparation method was the same as that of synthetic route 1 and Example 1, and 11.2 mg of 3-(1-oxo-4-((1-phenylethyl-1H-1, 2, 3-triazol-4-)methoxy) isoindolin-2-) piperidine-2, 6-dione was obtained, yield 25 %; ¹H NMR (400 MHz, DMSO) δ 10.77 (s, 1H), 8.16 (s, 1H), 7.50 (t, *J* = 7.8 Hz, 1H), 7.41 (d, *J* = 8.0 Hz, 1H), 7.34 (d, *J* = 7.4 Hz, 1H), 7.28 ― 7.13 (m, 5H), 5.28 (s, 2H), 5.11 (dd, *J* = 13.3, 5.0 Hz, 1H), 4.62 (t, *J* = 7.3 Hz, 2H), 4.33 (d, *J* = 17.5 Hz, 1H), 4.17 (d, *J* = 17.5 Hz, 1H), 3.15 (t, *J* = 7.3 Hz, 2H), 2.97 - 2.82 (m, 1H), 2.61-2.54 (m, 1H), 2.47-2.36 (m, 1H), 2.02 - 1.91 (m, 1H). UPLC - MS (ESI) calculated for C₂₄H₂₃N₅O₄ [M + H]⁺: 446.18, found:446.41.

### EXAMPLE 50: 3-(1-oxo-4-((1-((R)-1-phenethyl)-1H-1, 2, 3-triazol-4-(methoxy) isoindolin-2-) piperidine-2, 6-dione (50)

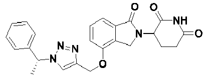

Step 1: (*R*)-1-phenylethylamine was used as a raw material, the preparation method was the same as that of method 6, and 110mg of (*R*)-1-phenylethyl azide was obtained, yield 75%; ¹H NMR (400 MHz, CDC13) δ 7.43 - 7.37 (m, 2H), 7.37 - 7.30 (m, 3H), 4.63 (q, J = 6.8 Hz, 1H), 1.55 (d, J = 6.8 Hz, 3H).

Step 2: (*R*)-1-phenylethylazide and intermediate 6 were used as raw materials, and the preparation method was the same as that of Synthetic Route 1 and Example 1, and 39.6 mg of 3-(1-oxo-4-((1-((*R*)-1-phenethyl)-1*H*-1, 2, 3-triazol-4-(methoxy) isoindolin-2-) piperidine-2, 6-dione was obtained, yield 32%; ¹H NMR (400 MHz, DMSO) δ 10.96 (s, 1H), 8.39 (s, 1H), 7.54 - 7.48 (m, 1H), 7.44 (d, *J* = 7.8 Hz, 1H), 7.41 - 7.24 (m, 7H), 5.95 (m, 1H), 5.28 (s, 2H), 5.09 (dd, *J* = 13.3, 5.1 Hz, 1H), 4.34 (d, *J* = 17.5 Hz, 1H), 4.18 (d, *J* = 17.5 Hz, 1H), 2.96 - 2.82 (m, 1H), 2.61 - 2.53 (m, 1H), 2.41 (qd, *J* = 13.3, 4.4 Hz, 1H), 1.98 - 1.92 (m, 1H), 1.89 (d, *J* = 7.1 Hz, 3H). UPLC - MS (ESI) calculated for C₂₄H₂₃N₅O₄ [M + H]⁺: 446.18, found:446.41.

### EXAMPLE 51: 3-(1-oxo-4-((1-((S)-1-phenethyl)-1H-1, 2, 3-triazol-4-(methoxy) isoindolin-2-) piperidine-2, 6-dione (51)

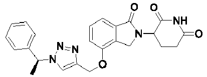

Step 1: (*S*)-1-phenylethylamine was used as a raw material, the preparation method was the same as that of method 6, and 110mg of (*S*)-1-phenylethyl azide was obtained, yield 75%; ¹H NMR (400 MHz, CDCl₃) δ 7.43 - 7.37 (m, 2H), 7.37 - 7.30 (m, 3H), 4.63 (q, *J* = 6.8 Hz, 1H), 1.55 (d, *J* = 6.8 Hz, 3H).

Step 2: (*S*)-1-phenylethylazide and intermediate 6 were used as raw materials, and the preparation method was the same as that of Synthetic Route 1 and Example 1, and 31.5 mg of 3-(1-oxo-4-((1-((*S*)-1-phenethyl)-1*H*-1, 2, 3-triazol-4-(methoxy) isoindolin-2-) piperidine-2, 6-dione was obtained, yield 52%; ¹H NMR (400 MHz, DMSO) δ 10.97 (s, 1H), 8.39 (s, 1H), 7.54 ― 7.41 (m, 2H), 7.39-7.30 (m, 6H), 5.96 (q, *J* = 7.0 Hz, 1H), 5.28 (s, 2H), 5.10 (dd, *J* = 13.0, 4.4 Hz, 1H), 4.34 (d, *J* = 17.5 Hz, 1H), 4.18 (d, *J* = 17.5 Hz, 1H), 2.97 - 2.82 (m, 1H),2.59-2.52 (m, 1H), 2.47-2.35 (m, 1H), 2.02 - 1.92 (m, 1H), 1.89 (d, *J* = 6.9 Hz, 3H). UPLC - MS (ESI) calculated for C₂₄H₂₃N₅O₄ [M + H]⁺: 446.18, found:446.37.

### EXAMPLE 52: 3-(4-((1-(R)-1-methoxy-3-phenylpropyl-2-)-1H-1, 2, 3-triazol-4-)methoxy)-1-oxoisoindolin-2-) piperidine-2, 6-dione (52)

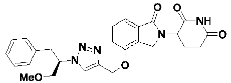

Step 1: azide compound was prepared as preparation method 6 of azides, and 170mg of (R)-(2-azido-3-methoxypropyl) benzene was obtained as a yellow oil, yield 89%.

Step 2: (*R*)-(2-azido-3-methoxypropyl) benzene and intermediate 6 were used as raw materials, and the preparation method was the same as that of Synthetic Route 1 and Example 1, and 47.7 mg of 3-(4-(1-((*R*)-1-methoxy-3-phenylpropyl-2-)-1*H*-1, 2, 3-triazol-4-)methoxy)-1-oxoisoindolin-2-) piperidine-2, 6-dione was obtained as a white solid, yield 58%; ¹H NMR (400 MHz, DMSO) δ 10.99 (s, 1H), 8.22 (s, 1H), 7.49 (t, *J* = 7.8 Hz, 1H), 7.41 (s, 1H), 7.35 (s, 1H), 7.18 ― 7.11 (m, 3H),7.05-7.02(m,2H), 5.25 (s, 2H), 5.11 (dd, *J* = 13.3, 4.9 Hz, 1H), 5.03 (m, 1H), 4.34 (dd, *J* = 17.4, 2.4 Hz, 1H), 4.18 (d, *J* = 17.5 Hz, 1H), 3.79 (dd, *J* = 10.3, 8.0 Hz, 1H), 3.69 (dd, *J* = 10.4, 4.1 Hz, 1H), 3.21 (d, *J* = 6.2 Hz, 3H), 3.19 - 3.08 (m, 2H), 2.91 (ddd, *J* = 17.5, 13.7, 5.4 Hz, 1H), 2.60-2.54 (m, 1H), 2.47-2.53 (m, 1H), 2.02 -1.92 (m, 1H). UPLC - MS (ESI) calculated for C₂₆H₂₇N₅O₅ [M + H]⁺: 490.20, found:490.29. **EXAMPLE 53:** 3-(4-((1-(*R*)-1-hydroxy-3-phenylpropyl-2-)-1*H*-1, 2, 3-triazol-4-(methoxy)-1-oxoisoindolin-2-) piperidine-2, 6-dione (**53**)

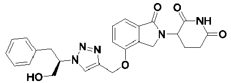

Step 1: preparation method was same as that of method 6, and 177mg of (*R*)-2-azido-3-phenyl-1-propanol was obtained, yield 100%; ¹H NMR (400 MHz, CDCl3) δ 7.33 (m, 2H), 7.26 (m, 3H), 3.72 (m, 2H), 3.57 (m, 1H), 2.93 - 2.80 (m, 2H), 1.79 (s, 1H).

Step 2: (*R*)-(2-azido-3-phenyl)-1-propanol and intermediate 6 were used as raw materials, and the preparation method was the same as that of Synthetic Route 1 and Example 1, and 38 mg of 3-(4-((1-(*R*)-1-hydroxy-3-phenylpropyl-2-)-1*H*-1, 2, 3-triazol-4-(methoxy)-1-oxoisoindolin-2-) piperidine-2, 6-dione was obtained as a white solid, yield 48 %; ¹H NMR (400 MHz, DMSO) δ 10.98 (s, 1H), 8.23 (s, 1H), 7.50 (t, *J* = 7.8 Hz, 1H), 7.42 (d, *J* = 7.8 Hz, 1H), 7.34 (d, *J* = 7.3 Hz, 1H), 7.14 (m, 3H), 7.04 (m, 2H), 5.25 (s, 2H), 5.11 (dd, *J* = 13.3, 5.0 Hz, 1H), 4.87 - 4.76 (m, 1H), 4.34 (d, *J* = 17.5 Hz, 1H), 4.18 (d, *J* = 17.5 Hz, 1H), 3.84 ― 3.73 (m, 2H), 3.21 (dd, *J* = 14.0, 5.5 Hz, 1H), 3.11 (dd, *J* = 14.0, 9.5 Hz, 1H), 2.97 - 2.84 (m, 1H), 2.60-2.54(m, 1H), 2.47-2.35 (m, 1H), 2.02-1.92 (m, 1H). UPLC - MS (ESI) calculated for C₂₅H₂₅N₅O₅ [M + H]⁺: 476.19, found:476.26.

### EXAMPLE 54: 3-(4-((1-(S)-1-hydroxy-3-phenylpropyl-2-)-1H-1, 2, 3-triazol-4-(methoxy)-1-oxoisoindolin-2-) piperidine-2, 6-dione (54)

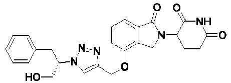

Step 1: the preparation method was the same as that of method 6, and 177mg of (*S*)-2-azido-3-phenyl-1-propanol was obtained, yield 99%.

Step 2: (*S*)-(2-azido-3-phenyl)-1-propanol and intermediate 6 were used as raw materials, and the preparation method was the same as that of Synthetic Route 1 and Example 1, and 26.6 mg of 3-(4-((1-(S)-1-hydroxy-3-phenylpropyl-2-)-1H-1, 2, 3-triazol-4-(methoxy)-1-oxoisoindolin-2-) piperidine-2, 6-dione was obtained, yield 33 %; ¹H NMR (400 MHz, DMSO) δ 10.99 (s, 1H), 8.24 (s, 1H), 7.50 (t, *J* = 7.8 Hz, 1H), 7.42 (d, *J* = 8.0 Hz, 1H), 7.34 (d, *J* = 7.3 Hz, 1H), 7.18 - 7.09 (m, 3H), 7.04 (d, *J* = 7.5 Hz, 2H), 5.25 (s, 2H), 5.11 (dd, *J* = 13.2, 5.0 Hz, 1H), 4.87 - 4.78 (m, 1H), 4.34 (dd, *J* = 17.4, 3.2 Hz, 1H), 4.19 (dd, *J* = 17.5, 1.5 Hz, 1H), 3.83 - 3.72 (m, 2H), 3.21 (dd, *J* = 14.0, 5.6 Hz, 1H), 3.11 (dd, *J* = 14.0, 9.6 Hz, 1H), 2.89 (d, *J* = 12.4 Hz, 1H), 2.60-2.54 (m, 1H), 2.47-2.35 (m, 1H),2.03-1.90 (m, 1H). UPLC - MS (ESI) calculated for C₂₅H₂₅N₅O₅ [M + H]⁺: 476.19, found:476.26.

### EXAMPLE 55: 3-(4-((1-(S)-1-methoxy-3-phenylpropyl-2-)-1H-1, 2, 3-triazol-4-(methoxy)-1-oxoisoindolin-2-) piperidine-2, 6-dione (55)

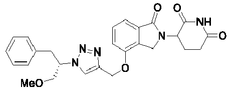

Step 1: the preparation method was the same as method 6, and 146mg of (S)-(2-azido-3-methoxypropyl) benzene was obtained, yield 76%; ¹H NMR (400 MHz, CDCl₃) δ 7.35 - 7.30 (m, 2H), 7.28 - 7.20 (m, 3H), 3.76-3.67 (m, 1H), 3.49 (dd, *J* = 10.0, 3.9 Hz, 1H), 3.42- 3.37 (m, 4H), 2.84 (ddd, *J* = 21.6, 13.8, 7.1 Hz, 2H).

Step 2: (S)-(2-azido-3-methoxypropyl) benzene and intermediate 6 were used as raw materials, and the preparation method was the same as that of Synthetic Route 1 and Example 1, and 54.8 mg of 3-(4-(1-((*S*)-1-methoxy-3-phenylpropyl-2-)-1*H*-1, 2, 3-triazol-4-)methoxy)-1-oxoisoindolin-2-) piperidine-2, 6-dione was obtained as a white solid, yield 67 %; ¹H NMR (400 MHz, DMSO) δ 11.01 (s, 1H), 8.23 (s, 1H), 7.49 (t, *J* = 7.8 Hz, 1H), 7.40 (d, *J* = 7.9 Hz, 1H), 7.34 (d, *J* = 7.2 Hz, 1H), 7.17 ― 7.11 (m, 3H), 7.03 (m, 2H), 5.25 (s, 2H), 5.11 (dd, *J* = 13.3, 5.1 Hz, 1H), 5.08 ― 4.98 (m, 1H), 4.34 (dd, *J* = 17.4, 2.5 Hz, 1H), 4.18 (d, *J* = 17.5 Hz, 1H), 3.79 (dd, *J* = 10.2, 8.0 Hz, 1H), 3.69 (dd, *J* = 10.4, 4.0 Hz, 1H), 3.20 (s, 3H), 3.19 - 3.08 (m, 2H), 2.91 (ddd, *J* = 17.6, 13.8, 5.3 Hz, 1H), 2.60-2.52(m, 1H), 2.42 (m, 1H), 2.01 - 1.92 (m, 1H), UPLC - MS (ESI) calculated for C₂₆H₂₇N₅O₅ [M + H]⁺: 490.20, found:490.33.

### EXAMPLE 56: 3-(4-((1-((S)-1-(dimethylamino)-3-phenylpropyl-2-)-1H-1, 2, 3-triazol-4-(methoxy)-1-oxoisoindolin-2-) piperidine-2, 6-dione (56)

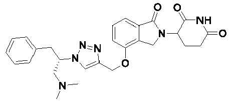

Step 1: the preparation method was the same as the synthesis method 6 of azides, and 75mg of (S)-2-azido-N, N-dimethyl-3-phenyl-1-propylamine was obtained as a yellow oil, yield 37%.

Step 2: ((S) -2-azo-N, N-dimethyl-3-phenyl-1-propylamine and intermediate 6 were used as raw materials, and the preparation method was the same as that of Synthetic Route 1 and Example 1, and 41.1 mg of 3-(4-((1-((*S*)-1-(dimethylamino)-3-phenylpropyl-2-)-1*H*-1, 2, 3-triazol-4-)methoxy)-1-oxoisoindolin-2-) piperidine-2, 6-dione was obtained as a white solid, yield 49 %; ¹H NMR (400 MHz, DMSO) δ 11.02 (s, 1H), 8.19 (d, *J* = 1.4 Hz, 1H), 7.49 (t, *J* = 7.8 Hz, 1H), 7.38 (d, *J* = 8.2 Hz, 1H), 7.34 (d, *J* = 7.4 Hz, 1H), 7.13-7.09 (m, 3H), 6.99 - 6.92 (m, 2H), 5.24 (s, 2H), 5.12 (dd, *J* = 13.3, 5.0 Hz, 1H), 5.00-4.90 (m, 1H), 4.33 (dd, *J* = 17.5, 3.0 Hz, 1H), 4.18 (d, *J* = 17.4 Hz, 1H), 3.19 (dd, *J* = 14.0, 4.9 Hz, 1H), 3.06 (dd, *J* = 14.0, 9.8 Hz, 1H), 2.96-2.87(m, 2H), 2.64-2.54 (m, 2H), 2.48 - 2.35 (m, 1H), 2.11 (s, 6H), 2.02 - 1.90 (m, 1H). UPLC - MS (ESI) calculated for C₂₇H₃₀N₆O₄ [M + H]+: 503.23, found:503.30.

### EXAMPLE 57: 3-(4-((1-(R)-1-(dimethylamino)-3-phenylpropyl-2-)-1H-1, 2, 3-triazol-4-(methoxy)-1-oxoisoindolin-2-) piperidine-2, 6-dione (57)

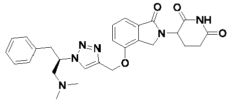

Step 1: the preparation method of azide compound was the same as the synthesis method 6 of azides, and 133mg of (R) -2-azido-N, N-dimethyl-3-phenyl-1-propylamine was obtained, yield 65%; ¹H NMR (400 MHz, CDCl3) δ 7.36 - 7.27 (m, 3H), 7.26-7.20 (m, 2H), 3.74 - 3.62 (m, 1H), 2.87 (dd, J = 13.9, 5.0 Hz, 1H), 2.75 (dd, J = 13.9, 8.1 Hz, 1H), 2.47 (dd, J = 12.8, 8.7 Hz, 1H), 2.32 (dd, J = 12.8, 4.6 Hz, 1H), 2.28 (s, 6H).

Step 2: ((R) -2-azido-N, N-dimethyl-3-phenyl-1-propylamine and intermediate 6 were used as raw materials, and the preparation method was the same as that of Synthetic Route 1 and Example 1, and 51.1 mg of 3-(4-((1-((R)-1-(dimethylamino)-3-phenylpropyl-2-)-1H-1, 2, 3-triazol-4-(methoxy)-1-oxoisoindolin-2-) piperidine-2, 6-dione was obtained as a white solid, yield 61 %; ¹H NMR (400 MHz, DMSO) δ 11.02 (s, 1H), 8.19 (d, *J* = 1.5 Hz, 1H), 7.49 (t, *J* = 7.8 Hz, 1H), 7.38 (d, *J* = 8.1 Hz, 1H), 7.34 (d, *J* = 7.4 Hz, 1H), 7.11 (m, 3H), 6.99 ― 6.92 (m, 2H), 5.25 (s, 2H), 5.12 (dd, *J* = 13.3, 5.0 Hz, 1H),5.01-4.91 (m, 1H), 4.33 (dd, *J* = 17.5, 2.8 Hz, 1H), 4.18 (d, *J* = 17.4 Hz, 1H), 3.19 (dd, *J* = 14.1, 5.0 Hz, 1H), 3.06 (dd, *J* = 13.9, 9.7 Hz, 1H), 2.98 - 2.85 (m, 2H), 2.69 - 2.54 (m, 2H), 2.48-2.35 (m, 1H), 2.13 (s, 6H), 2.02-1.90 (m, 1H). UPLC - MS (ESI) calculated for C₂₇H₃₀N₆O₄ [M + H]+: 503.23, found:503.34.

### EXAMPLE 58: 3-(1-oxo-4-((1-((R)-1-phenylpropyl-2-)-1H-1, 2, 3-triazol-4-)methoxy) isoindolin-2-) piperidine-2, 6-dione (58)

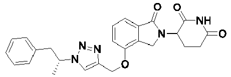

Step 1: the preparation metod was the same as intermediate (*S*)-1-phenyl-2-propylazide, 76mg of (R)-1-phenyl-2-propylazide was obtained as a yellow oil, yield 47%.

Step 2: (R) -1-phenyl-2-propyl azide and intermediate 6 were used as raw materials, and the preparation method was the same as that of Synthetic Route 1 and Example 1, and 41.5 mg of 3-(1-oxo-4-((1-((*R*)-1-phenylpropyl-2-)-1*H*-1, 2, 3-triazol-4-(methoxy) isoindolin-2-) piperidine-2, 6-dione was obtained as a white solid, yield 54 %; ¹H NMR (400 MHz, DMSO) δ 11.02 (s, 1H), 8.23 (s, 1H), 7.51 (t, *J* = 7.8 Hz, 1H), 7.41 (d, *J* = 8.1 Hz, 1H), 7.36 ― 7.33 (m, 1H), 7.19 ― 7.13 (m, 3H), 7.03-7.01(m, 2H), 5.26 (s, 2H), 5.12 (dd, *J* = 13.3, 5.0 Hz, 1H), 5.00 - 4.90 (m, 1H), 3.14 (d, *J* = 7.5 Hz, 2H), 2.92 (ddd, *J* = 17.8, 13.8, 5.3 Hz, 1H), 2.62 - 2.55 (m, 1H), 2.43 (ddd, *J* = 26.5, 13.3, 4.4 Hz, 1H), 2.02 - 1.92 (m, 1H), 1.51 (d, *J* = 6.7 Hz, 3H). UPLC - MS (ESI) calculated for C₂₅H₂₅N₅O₄ [M + H]⁺: 460.19, found:460.32.

### EXAMPLE 59: 3-(1-oxo-4-((1-((S)-1-phenylpropyl-2-)-1H-1, 2, 3-triazol-4-)methoxy) isoindolin-2-) piperidine-2, 6-dione (59)

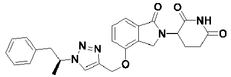

Step 1: iodine (2.18 g, 8.60 mmol) was added to a dichloromethane solution (30mL) containing triphenylphosphine (2.26 g, 8.60 mmol) and imidazole (585mg, 8.60 mmol) at 0°C, and reacted for 10min, (*R*)-N-Boc-1-hydroxy-3-phenyl-2-propylamine (1.66 g, 6.61 mmol) in dichloromethane (10mL) was added to the reaction solution and the reaction solution was raised to room temperature and reacted for 2h. After the reaction was completed, the reaction solution was washed with water and saturated sodium chloride solution successively, dried over anhydrous sodium sulfate, filtered, concentrated under reduced pressure, and the residue was subjected to silica gel column chromatography to obtain 1.62 g of (*R*)-N-Boc-1-iodo-3-phenyl-2-propylamine as a white solid, yield 68%; ¹H NMR (400 MHz, CDCl₃) δ 7.34 ― 7.25 (m, 5H), 4.69 (d, J = 7.1 Hz, 1H), 3.59 (m, 1H), 3.40 (dd, J = 10.0, 4.4 Hz, 1H), 3.16 (dd, J = 10.2, 3.7 Hz, 1H), 2.91 (dd, J = 13.5, 5.8 Hz, 1H), 2.76 (dd, J = 13.6, 8.3 Hz, 1H), 1.43 (s, 9H).

Step 2: (*R*)-N-Boc-1-iodo-3-phenyl-2-propylamine (1.62 g, 4.48 mmol) was dissolved in 30 ml of methanol, triethylamine (3.12 ml, 22.4 mmol) and 10% Pd/C (162mg) were added, and reacted in H₂(1 atm) for 5h. After the reaction was completed, the reaction solution was filtered by diatomite, concentrated under reduced pressure, and the residue was sujected to silica gel column chromatography to obtain 824mg of (*S*)-N-Boc-1-phenyl-2-propylamine as a light yellow solid, yield 78%;¹H NMR (400 MHz, CDCl₃) δ 7.32-7.27 (m, 2H), 7.23-7.16(m, 3H), 4.44-4.31 (m, 1H), 3.97-3.85 (m, 1H), 2.84 (dd, J = 12.9, 5.0 Hz, 1H), 2.68-2.62 (m, 1H), 1.42 (s, 9H), 1.08 (d, J = 6.7 Hz, 3H).

Step 3: (*S*)-N-Boc-1-phenyl-2-propylamine (824mg, 3.94 mmol) was dissolved in 20ml of dioxane hydrochloride and reacted overnight at room temperature. After the reaction was completed, the solvent was removed under reduced pressure, water was added to the reaction system, pH was adjusted to be alkaline with saturated sodium bicarbonate solution, extracted with ethyl acetate twice, combined the organic layers, washed with saturated sodium chloride, dried over anhydrous sodium sulfate, and the solvent was removed under reduced pressure to obtain 414mg of (*S*)-1-phenyl-2-propylamine as a colorless oil, yield 74%.

Step 4: The preparation method was the same as method 6, and 65mg of (*S*)-1-phenyl-2-propylazide was obtained, yield 40%; ¹H NMR (400 MHz, CDCl₃) δ 7.36 ― 7.18 (m, 5H), 3.76 - 3.62 (m, 1H), 2.84 (dd, J = 13.6, 7.3 Hz, 1H), 2.73 (dd, J = 13.6, 6.5 Hz, 1H), 1.27 (d, J = 6.5 Hz, 3H).

Step 5: (S) -1-phenyl-2-propyl azide and intermediate 6 were used as raw materials, and the preparation method was the same as that of Synthetic Route 1 and Example 1, and 35.8 mg of 3-(1-oxo-4-((1-((S)-1-phenylpropyl-2-)-1H-1, 2, 3-triazol-4-)methoxy) isoindolin-2-) piperidine-2, 6-dione was obtained as a white solid, yield 47 %; ¹H NMR (400 MHz, DMSO) δ 11.01 (s, 1H), 8.23 (s, 1H), 7.50 (t, *J* = 7.8 Hz, 1H), 7.40 (d, *J* = 8.1 Hz, 1H), 7.34 (d, *J* = 7.4 Hz, 1H), 7.18 ― 7.10 (m, 3H),7.03-7.01 (m, 2H), 5.25 (s, 2H), 5.12 (dd, *J* = 13.2, 5.0 Hz, 1H), 5.01 - 4.87 (m, 1H), 4.33 (dd, *J* = 17.4, 2.4 Hz, 1H), 4.18 (d, *J* = 17.2 Hz, 1H), 3.14 (d, *J* = 7.4 Hz, 2H), 2.91 (ddd, *J* = 17.7, 14.0, 5.5 Hz, 1H), 2.62 - 2.55 (m, 1H), 2.42 (ddd, *J* = 26.0, 12.9, 4.2 Hz, 1H), 2.02 - 1.92 (m, 1H), 1.50 (d, *J* = 6.7 Hz, 3H). UPLC - MS (ESI) calculated for C₂₅H₂₅N₅O₄ [M + H]+: 460.19, found:460.32.

### EXAMPLE 60: 3-(1-oxo-4-(2-(1-phenyl-1H-1, 2, 3-triazol-4-) ethoxy) isoindolin-2-) piperidine-2, 6-dione (60)

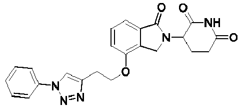

Azido benzene and intermediate 8 were used as raw materials, the preparation method was the same as that of synthetic route 1 and Example 1, and 35mg of 3-(1-oxo-4-(2-(1-phenyl-1H-1, 2, 3-triazol-4-)ethoxy) isoindolin-2-) piperidine-2, 6-dione was obtained, yield 55 %; ¹H NMR (400 MHz, DMSO) δ 10.96 (s, 1H), 8.69 (s, 1H), 7.87 (d, *J* = 8.2 Hz, 2H), 7.59 (t, *J* = 7.9 Hz, 2H), 7.48 (q, *J* = 7.7 Hz, 2H), 7.32 (dd, *J* = 7.8, 4.8 Hz, 2H), 5.10 (dd, *J* = 13.3, 5.1 Hz, 1H), 4.44 (t, *J* = 6.5 Hz, 2H), 4.34 (d, *J* = 17.4 Hz, 1H), 4.21 (d, *J* = 17.4 Hz, 1H), 3.23 (t, *J* = 6.5 Hz, 2H), 2.97 - 2.83 (m, 1H), 2.62 - 2.53 (m, 1H), 2.38 (ddd, *J* = 26.3, 13.3, 4.4 Hz, 1H), 2.02 - 1.82 (m, 1H). UPLC - MS (ESI) calculated for C₂₃H₂₁N₅O₄ [M + H]+: 432.16, found:432.23.

### EXAMPLE 61: 3-(1-oxo-4-(2-(1-(4-(trifluoromethoxy) phenyl)-1H-1, 2, 3-triazol-4-) ethoxy) isoindoline-2-) piperidine-2, 6-dione (61)

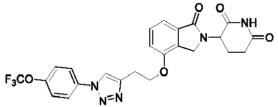

4- trifluoromethoxyphenyl azide and intermediate 8 were used as raw materials, the preparation method was the same as that of synthetic route 1 and Example 1, and 40.1mg of 3-(1-oxo-4-(2-(1-(4-(trifluoromethoxy) phenyl)-1*H*-1, 2, 3-triazol-4-) ethoxy) isoindoline-2-) piperidine-2, 6-dione was obtained as a white solid, yield 54%; ¹H NMR (400 MHz, DMSO) δ 10.98 (s, 1H), 8.73 (s, 1H), 8.05 ― 7.98 (m, 2H), 7.62 (d, *J* = 8.7 Hz, 2H), 7.49 (t, *J* = 7.8 Hz, 1H), 7.32 (t, *J* = 7.4 Hz, 2H), 5.10 (dd, *J* = 13.3, 5.1 Hz, 1H), 4.45 (t, *J* = 6.4 Hz, 2H), 4.34 (d, *J* = 17.4 Hz, 1H), 4.21 (d, *J* = 17.4 Hz, 1H), 3.23 (t, *J* = 6.4 Hz, 2H), 2.98 - 2.84 (m, 1H), 2.63 - 2.52 (m, 1H), 2.40 (qd, *J* = 13.3, 4.3 Hz, 1H), 2.01-1.92 (m, 1H). UPLC ―MS (ESI) calculated for C₂₄H₂₀F₃N₅O₅ [M + H]+: 516.14, found:516.17. 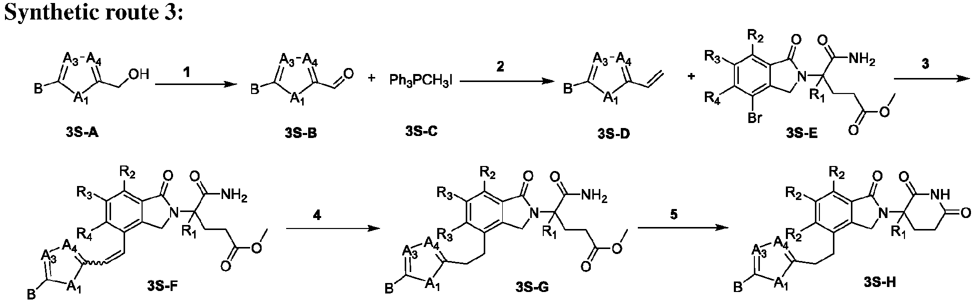 wherein R₁, R₂, R₃, R₄, A₁, A₃, A₄ and B have the same definitions as above.

### EXAMPLE 62:3-(1-oxo-4-(2-(2-(4-(trifluoromethoxy) phenyl) oxazol-5-yl) ethyl) isoindolin-2-yl) piperidine-2, 6-dione (62)

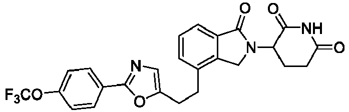

The synthetic route of EXAMPLE **62** referred to synthetic route 3. Step 1: (2-(4-(trifluoromethoxy) phenyl) oxazol-5-yl) methanol (170mg, 0.66 mmol) was dissolved in dry dichloromethane, manganese dioxide (570mg, 6.56 mmol) was added under stirring conditions, the reaction system reacted overnight at room temperature, TLC monitored that the reaction was completed, filtrated with diatomite, the filtrate was concentrated under reduced pressure, and flash column chromatography was used to give 164mg of white solid, yield 98%. ¹H NMR (400 MHz, CDCl₃) δ 9.83 (s, 1H), 8.26 - 8.21 (m, 2H), 7.95 (s, 1H), 7.36 (d, *J* = 8.2 Hz, 2H).

Step 2: potassium tert-butoxide (160mg, 1.39 mmol) was added to PPh3⁺CH3I⁻(562mg, 1.39 mmol) in a dry tetrahydrofuran solution at 0°C, reacted for 45min under nitrogen protection at the same temperature. A solution of 2-(4-(trifluoromethoxy) phenyl) oxazol-5-formaldehyde (143mg, 0.56 mmol) in tetrahydrofuran (5ml) was added dropwise to the reaction system. After dropwise addition, it was raised to room temperature and reacted for 2h. After LC-MSS monitored the completion of the reaction, it was quenched with ice water, then extracted with ethyl acetate (2 × 40 ml), combined the organic layers, washed the organic layers with saturated sodium chloride solution, dried, and subjected to flash column chromatography to obtain 123mg of light yellow oil with a yield of 87%. ¹H NMR (500 MHz, CDCl3) δ 8.09 (d, *J* = 8.8 Hz, 2H), 7.30 (d, *J* = 8.5 Hz, 2H), 7.08 (s, 1H), 6.57 (dd, *J* = 17.5, 11.3 Hz, 1H), 5.83 (d, *J* = 17.5 Hz, 1H), 5.37 (d, *J* = 11.4 Hz, 1H).

Step 3: the solution of 2-(4-(trifluoromethoxy) phenyl)-5-vinyloxinazole (120mg, 0.47 mmol), methyl 5-amino-4-(4-bromo-1-oxoisoindolin-2-yl)-5-oxopentanoate(167mg,. 47mmol), palladium acetate (11mg, 0.047 mmol), tris (o-methylphenyl) phosphorus (23mg, 0.075 mmol), N, N-diisopropylethylamine (117µL, 0.71 mmol) in acetonitrile was replaced with nitrogen three times, reacted overnight at 90°C under the condition of nitrogen protection . LC-MSS was used to monitor that the reaction was completed, concentrated under reduced pressure, diluted with ethyl acetate, washed with saturated sodium chloride solution, the organic layer was dried over anhydrous sodium sulfate, and subjected to rapid column chromatography to obtain 67mg of a white solid.

Step 4: (Z)-5-amino-5-oxo-4-(1-oxo-4-(2-(2-(4-(trifluoromethoxy) phenyl) oxazol-5-yl) vinyl) isoindolin-2-yl) valeric acid (67mg, 0.126 mmol) was dissolved in 5ml of methanol, 7mg of 10% Pd/C was added, and reacted overnight at room temperature under hydrogen of normal pressure.LC-MSS was used to monitor that the reaction was completed, filtered, spin-dried, and directly used in the next step.

Step 5: the crude methyl 5-amino-5-oxo-4-(1-oxo-4-(2-(2-(4-(trifluoromethoxy) phenyl) oxazol-5-yl) ethyl) isoindolin-2-yl) valerate (67mg. 127mmol) obtained in the previous step was dissolved in 3ml of dry tetrahydrofuran, potassium tert-butoxide (16mg, 0.14 mmol) was added at 0°C, and reacted at the same temperature for half an hour. After the reaction was completed monitoring by LC-MSS, formic acid was added to quench, concentrated under reduced pressure, and purified by HPLC to obtain 20mg of white solid with a yield of 31.5%. ¹H NMR (400 MHz, DMSO) δ 10.99 (s, 1H), 8.03 (d, *J* = 8.8 Hz, 2H), 7.60 (d, *J* = 7.2 Hz, 1H), 7.55 - 7.43 (m, 4H), 7.04 (s, 1H), 5.12 (dd, *J* = 13.3, 5.1 Hz, 1H), 4.44 (d, *J* = 17.2 Hz, 1H), 4.32 (d, *J* = 17.2 Hz, 1H), 3.18 - 3.10 (m, 2H), 3.10 - 3.03 (m, 2H), 2.96 - 2.84 (m, 1H), 2.60-2.52 (m, 1H), 2.33 (qd, *J =* 13.3, 4.6 Hz, 1H), 1.97 - 1.88 (m, 1H). UPLC - MS (ESI) calculated for C₂₅H₂₀F₃N₃O₅ [M + H]+: 500.14, found:500.30.

### EXAMPLE 63:3-(1-oxo-4-(3-(2-(4-(trifluoromethoxy) phenyl) oxazol-5-yl) propyl) isoindolin-2-yl) piperidine-2, 6-dione (63)

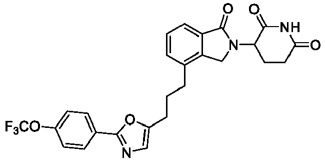

The synthetic route of EXAMPLE **63** referred to synthetic route 3. 4.5mg of white solid was obtained, yield 14%. ¹H NMR (400 MHz, DMSO) δ 10.99 (s, 1H), 8.04 (d, *J* = 8.8 Hz, 2H), 7.57 (d, *J* = 6.7 Hz, 1H), 7.54 ― 7.43 (m, 4H), 7.10 (s, 1H), 5.12 (dd, *J* = 13.3, 5.1 Hz, 1H), 4.47 (d, *J* = 17.2 Hz, 1H), 4.32 (d, *J* = 17.2 Hz, 1H), 2.97 - 2.86 (m,1H), 2.85 - 2.72 (m, 4H), 2.58 (d, *J* = 17.2 Hz, 1H), 2.37 (dt, *J* = 13.0, 8.8 Hz, 1H), 2.06-1.97 (m, 3H). UPLC - MS (ESI) calculated for C₂₆H₂₂F₃N₃O₅ [M + H]+: 514.15, found:514.37.

### EXAMPLE 64: 3-(1-oxo-4-((1-(4-trifluoromethoxyphenyl) azetidin-3-) methoxy) isoindolin-2-) piperidine-2, 6-dione (64)

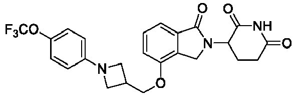

Step 1: 1-iodo-4-trifluoromethoxybenzene (288mg, 1.00 mmol), 3-methoxyazetidine hydrochloride (82mg, 0.66 mmol), cuprous iodide (25mg, 0.13 mmol), L-proline (30.4 mg, 0.26 mmol) and cesium carbonate (538mg, 1.65 mmol) were dissolved in 10ml of DMSO and heated to 90°C and reacted for 18h under nitrogen protection. After the reaction was completed, the reaction solution was cooled to room temperature, diluted with ethyl acetate, washed with water and saturated sodium chloride aqueous solution in turn, dried over anhydrous sodium sulfate, filtered, concentrated under reduced pressure, and then subjected to silica gel column chromatography to obtain 71mg of 1-(4-trifluoromethoxyphenyl) azetidine-3-methanol as a brown solid, yield 44%; 1H NMR (400 MHz, DMSO) δ 7.13 (d, J = 8.8 Hz, 2H), 6.43 (d, J = 8.9 Hz, 2H), 4.75 (t, J = 5.3 Hz, 1H), 3.83 (t, J = 7.6 Hz, 2H), 3.60 - 3.51 (m, 4H), 2.80-2.72 (m, 1H).

Step 2: 1-(4-trifluoromethoxyphenyl) azetidine-3-methanol and intermediate 4 were used as raw materials, and the preparation method was the same as the synthesis route 2, and 3-(1-oxo-4-((1-(4-trifluoromethoxyphenyl) azetidin-3-) methoxy) isoindolin-2-) piperidine-2, 6-dione was obtained as a white solid.

Step 3: the preparation method was the same as the synthesis route 2, 51mg of product was obtained, yield 65%; ¹H NMR (400 MHz, DMSO) δ 10.97 (s, 1H), 7.49 (t, *J* = 7.8 Hz, 1H), 7.31 (dd, *J* = 13.1, 7.7 Hz, 2H), 7.15 (d, *J* = 8.3 Hz, 2H), 6.53 - 6.45 (m, 2H), 5.10 (dd, *J* = 13.3, 5.1 Hz, 1H), 4.35 (d, *J* = 6.6 Hz, 2H), 4.26 (d, *J* = 17.4 Hz, 1H), 4.13 (d, *J* = 17.4 Hz, 1H), 3.99 (t, *J* = 7.7 Hz, 2H), 3.74 - 3.68 (m, 2H), 3.20 - 3.08 (m, 1H), 2.97 - 2.85 (m, 1H), 2.62-2.55 (m, 1H),2.48-2.30 (m, 1H), 2.02 - 1.92 (m, 1H). UPLC - MS (ESI) calculated for C₂₄H₂₂F₃N₃O₅ [M + H]+: 490.15, found:490.25.

### EXAMPLE 65: 3-(1-oxo-4-(((S)-1-(quinolin-4-) pyrrolin-3-) methoxy) isoindolin-2-) piperidine-2, 6-dione (65)

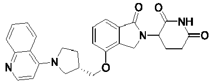

Step 1: (S)-pyrrolidine-3-methanol (100mg, 0.99 mmol), 4-chloroquinoline (485mg, 2.97 mmol, 3.0 eq), potassium carbonate (410mg, 2.97 mmol, 3eq) were dissolved in 10ml DMF, reacted at 120 °C for 24h. After completion of the reaction, the reaction solution was diluted with ethyl acetate, washed with saturated sodium chloride and purified by column chromatography to obtain 123mg (S)-(1-(quinolin-4-) pyrrolin-3-) methanol as a yellow oil, yield 54%; ¹H NMR (400 MHz, CDCl₃) δ 8.35 (d, *J* = 5.5 Hz, 1H), 8.15 (d, *J* = 8.5 Hz, 1H), 7.91 (d, *J* = 8.3 Hz, 1H), 7.53 (dd, *J* = 11.2, 4.0 Hz, 1H), 7.32 - 7.23 (m, 1H), 6.31 (d, *J* = 5.5 Hz, 1H), 3.82 - 3.56 (m, 6H), 2.65 ― 2.51 (m, 1H), 2.13 (dq, *J* = 12.1, 6.1 Hz, 1H), 1.85 (dq, *J* = 12.4, 7.7 Hz, 1H).

Step 2: methyl 5-amino-4-(4-hydroxy-1-oxoisoindolin-2-)-5-oxopentanoate (50mg, 0.17 mmol, 1.0 eq), (*S*)-(1-(quinolin-4-) pyrrolin-3-) methanol (82mg, 0.34 mmol, 2.0 eq) were dissolved in 20ml of tetrahydrofuran, triphenylphosphine (89mg, 0.34 mmol, 2.0 eq) was added until completely dissolved, azobisisobutyronitrile (67ul, 0.34 mmol, 2.0 eq) was added, and reacted at room temperature for 2 hours. After completion of the reaction, the solvent was removed and purified by TLC to give 60mg of methyl 5-amino-5-oxo-4-(1-oxo-4-(((*S*)-1-(quinolin-4-) pyrrolin-3-) methoxy) isoindolin-2-) oxopentanoate as a white solid, yield 69%; ¹H NMR (400 MHz, CDCl₃) δ 8.53 (d, *J* = 5.5 Hz, 1H), 8.21 (d, *J* = 8.3 Hz, 1H), 8.01 (d, *J* = 8.3 Hz, 1H), 7.61 (t, *J* = 7.2 Hz, 1H), 7.47 - 7.36 (m, 3H), 7.02 (dd, *J* = 7.0, 1.7 Hz, 1H), 6.53 (d, *J* = 5.6 Hz, 1H), 6.38 (s, 1H), 5.53 (s, 1H), 4.91 (dd, *J* = 8.7, 6.3 Hz, 1H), 4.42 (q, *J* = 17.5 Hz, 2H), 4.15 (d, *J* = 6.6 Hz, 2H), 3.93 (dd, *J* = 9.8, 7.1 Hz, 1H), 3.82 (dd, *J* = 9.6, 5.0 Hz, 2H), 3.68 (dd, *J* = 9.9, 6.9 Hz, 1H), 3.64 - 3.61 (m, 3H), 2.97 - 2.87 (m, 1H), 2.47 - 2.28 (m, 4H), 2.24 ― 2.15 (m, 1H), 2.04 (dd, *J* = 12.4, 7.7 Hz, 1H).

Step 3: methyl 5-amino-5-oxo-4-(1-oxo-4-(((S)-1-(quinolin-4-)pyrrolin)-3-)methoxy)isoindolin-2-)oxopentanoa te (30mg, 0.06mmol, 1.0eq) was dissolved in 10ml of dry tetrahydrofuran, potassium tert-butoxide (7mg, 0.06mmol, 1eq) was added under ice bath condition, and the detection of the reaction was started 10 min later. After completion of the reaction, 5ul of formic acid was added to quench the reaction, the solvent was rotated away and purified by HPLC to give 11mg of 3-(1-oxo-4-(((*S*)-1-(quinolin-4-) pyrrolin-3-) methoxy) isoindolin-2-) piperidine-2, 6-dione as a white solid, yield 39%; ¹H NMR (400 MHz, DMSO) δ 10.94 (s, 1H), 8.60 (d, *J* = 8.5 Hz, 1H), 8.46 (s, 1H), 7.91 (dt, *J* = 8.5, 7.3 Hz, 2H), 7.63 (t, *J* = 7.7 Hz, 1H), 7.49 (t, *J* = 7.7 Hz, 1H), 7.33 (d, *J* = 7.5 Hz, 1H), 7.27 (d, *J* = 8.2 Hz, 1H), 6.80 (d, *J* = 7.3 Hz, 1H), 5.11 (dd, *J* = 13.2, 5.1 Hz, 1H), 4.47-3.76(m, 8H), 2.98-2.86 (m, 2H), 2.65 - 2.54 (m, 1H), 2.38-2.25 (m, 2H), 2.15-2.04 (m, 1H), 2.02-1.92 (m, 1H). UPLC - MS (ESI) calculated for C₂₇H₂₆N₄O₄ [M + H]+: 471.20, found:471.39.

### EXAMPLE 66: 3-(1-oxo-4-(((R)-1-(quinolin-4-) pyrrolin-3-) methoxy) isoindolin-2-) piperidine-2, 6-dione (66)

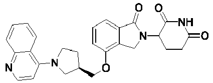

Step 1: (*R*)-pyrrolidine-3-methanol (50mg, 0.49 mmol, 1.5 eq), 4-chloroquinoline (54mg, 0.33 mmol, 1eq), potassium carbonate (138mg, 0.99 mmol, 3eq) were dissolved in 5ml of DMF, and reacted at 120 °C for 24h. After the reaction was completed, diluted with ethyl acetate, washed with saturated sodium chloride, and purified by column chromatography to obtain 273mg of (*R*)-(1-(quinolin-4-) pyrrolin-3-) methanol as a yellow oil, yield 49%; ¹H NMR (400 MHz, CDCl3) δ 8.47 (d, *J* = 5.4 Hz, 1H), 8.20 (d, *J* = 8.6 Hz, 1H), 7.96 (dd, *J* = 8.6 Hz, 0.8 Hz, 1H), 7.61 - 7.54 (m, 1H), 7.36 - 7.30 (m, 1H), 6.44 (d, *J* = 5.5 Hz, 1H), 3.84 - 3.68 (m, 5H), 3.60 (dd, *J* = 9.8, 7.0 Hz, 1H), 2.60 (dt, *J* = 14.0, 6.8 Hz, 1H), 2.18 (td, *J* = 12.1, 6.0 Hz, 1H), 1.88 (ddd, *J* = 15.8, 12.3, 7.9 Hz, 2H), 1.71 (s, 1H).

Step 2: methyl 5-amino-4-(4-hydroxy-1-oxoisoindolin-2-)-5-oxopentanoate (50mg, 0.17 mmol, 1.0 eq), (*R*)-pyrrolidin-3-methanol (82mg, 0.34 mmol, 2.0 eq) were dissolved in 20ml of tetrahydrofuran, triphenylphosphine (89mg, 0.34 mmol, 2.0 eq) was added until completely dissolved, diisopropyl azodicarboxylate (67ul, 0.34 mmol, 2.0 eq) was added, and reacted at room temperature for 2 hours. After the reaction was completed, the solvent was spun off, and the product was purified by TLC to obtain 60 mg of white solid with a yield of 69%; ¹H NMR (400 MHz, CDCl₃) δ 8.53 (d, *J* = 5.5 Hz, 1H), 8.21 (d, *J* = 8.3 Hz, 1H), 8.01 (d, *J* = 8.3 Hz, 1H), 7.61 (t, *J* = 7.2 Hz, 1H), 7.47 - 7.36 (m, 3H), 7.02 (dd, *J* = 7.0, 1.7 Hz, 1H), 6.53 (d, *J* = 5.6 Hz, 1H), 6.38 (s, 1H), 5.53 (s, 1H), 4.91 (dd, *J* = 8.7, 6.3 Hz, 1H), 4.42 (q, *J* = 17.5 Hz, 2H), 4.15 (d, *J* = 6.6 Hz, 2H), 3.93 (dd, *J* = 9.8, 7.1 Hz, 1H), 3.82 (dd, *J* = 9.6, 5.0 Hz, 2H), 3.68 (dd, *J* = 9.9, 6.9 Hz, 1H), 3.64 - 3.61 (m, 3H), 2.97 - 2.87 (m, 1H), 2.47 - 2.28 (m, 4H), 2.24 - 2.15 (m, 1H), 2.04 (dd, *J* = 12.4, 7.7 Hz, 1H).

Step 3: methyl 5-amino-5-oxo-4-(1-oxo-4-(((R)-1-(quinolin-4-)pyrrolin)-3-)methoxy)isoindolin-2-)oxopentanoa te (20mg, 0.04mmol, 1.0eq) was dissolved in 10ml of dry tetrahydrofuran, potassium tert-butoxide (4.5mg, 0.06mmol, 1eq) was added under ice bath condition, and the detection of the reaction wsa started 10 min later. After completion of the reaction, 5ul of formic acid was added to quench the reaction, the solvent was rotated away and purified by HPLC to give 8.4mg of 3-(1-oxo-4-(((R)-1-(quinolin-4-) pyrrolin-3-) methoxy) isoindolin-2-) piperidine-2, 6-dione as a white solid, yield 45%; ¹H NMR (400 MHz, DMSO) δ 10.99 (s, 1H), 8.60 (d, *J* = 8.6 Hz, 1H), 8.49 - 8.42 (t, *J* = 8.6 Hz, 1H), 7.96 ― 7.86 (m, 2H), 7.63 (ddd, *J* = 8.5, 6.6, 1.7 Hz, 1H), 7.50 (t, *J* = 7.8 Hz, 1H), 7.33 (d, *J* = 7.4 Hz, 1H), 7.27 (d, *J* = 8.1 Hz, 1H), 6.81 (d, *J* = 7.2 Hz, 1H), 5.10 (dd, *J* = 13.5, 5.1 Hz, 1H), 4.37-3.58(m, 8H)2.98 - 2.83 (m, 2H), 2.67 - 2.54 (m, 1H), 2.35-2.27 (m, 2H), 2.15 - 2.03 (m, 1H), 1.99-1.89 (m, 2H). UPLC - MS (ESI) calculated for C₂₇H₂₆N₄O₄ [M + H]+: 471.20, found:471.39.

### EXAMPLE 67: 3-(1-oxo-4-((1-(quinolin-4-) piperidin-4-) methoxy) isoindolin-2-) piperidin-2, 6-dione (67)

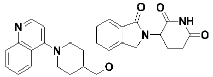

The preparation method was the same as that of the example **66,** 29.1 mg of example compound **67** was obtained, yield 50%; ¹H NMR (400 MHz, DMSO) δ 10.99 (s, 1H), 8.66 (d, *J* = 7.0 Hz, 1H), 8.15 (d, *J* = 8.5 Hz, 1H), 8.00 ― 7.94 (m, 2H), 7.70 (ddd, *J* = 8.4, 6.8, 4.2 Hz, 1H), 7.50 (t, *J* = 7.8 Hz, 1H), 7.33 (d, *J* = 7.4 Hz, 1H), 7.29 (d, *J* = 8.1 Hz, 1H), 7.22 (d, *J* = 7.1 Hz, 1H), 5.13 (dd, *J* = 13.3, 5.0 Hz, 1H), 4.40 (d, *J* = 17.3 Hz, 1H), 4.24 (dd, *J* = 15.2, 10.9 Hz, 3H), 4.15 - 4.06 (m, 2H), 3.50 (t, *J* = 12.9 Hz, 2H), 2.92 (ddd, *J* = 17.8, 13.4, 5.1 Hz, 1H), 2.58 (ddd, *J* = 5.1, 4.2, 1.8 Hz, 1H), 2.48 - 2.37 (m, 1H), 2.34 - 2.22 (m, 1H), 2.10 - 1.94 (m, 3H), 1.64 (dd, *J* = 24.2, 12.2 Hz, 2H). UPLC - MS (ESI) calculated for C₂₈H₂₈N₄O₄ [M + H]+: 485.21, found:485.38.

### EXAMPLE 68: 3-(1-oxo-4-(2-(1-(quinolin-4-) piperidin-4-) ethoxy) isoindolin-2-) piperidin-2, 6-dione (68)

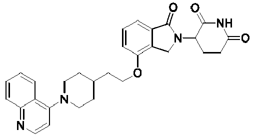

The preparation method was the same as that of the example 69, 26.5mg of example compound 68 was obtained, yield 55 %; ¹H NMR (400 MHz, DMSO) δ 10.99 (s, 1H), 8.65 (d, *J* = 7.0 Hz, 1H), 8.14 (d, *J* = 8.5 Hz, 1H), 8.04 ― 7.93 (m, 2H), 7.70 (ddd, *J* = 8.3, 5.8, 2.4 Hz, 1H), 7.50 (t, *J* = 7.8 Hz, 1H), 7.31 (dd, *J* = 14.3, 7.8 Hz, 2H), 7.20 (d, *J* = 7.1 Hz, 1H), 5.13 (dd, *J* = 13.3, 5.1 Hz, 1H), 4.40 (d, *J* = 17.4 Hz, 1H), 4.34 ― 4.02 (m, 5H), 3.46 (t, *J* = 12.6 Hz, 2H), 3.01 - 2.85 (m, 1H), 2.60 (d, *J* = 17.6 Hz, 1H), 2.49 - 2.38 (m, 1H), 2.04-1.95 (m, 4H), 1.82 (dd, *J* = 11.9, 5.6 Hz, 2H), 1.54 (dd, *J* = 22.8, 11.3 Hz, 2H). UPLC - MS (ESI) calculated for C₂₉H₃₀N₄O₄ [M + H]⁺: 499.23, found:499.84.

### EXAMPLE 69: 3-(1-oxo-4-(2-(1-(quinolin-4-) azetidin-3-) ethoxy) isoindolin-2-) piperidine-2, 6-dione (69)

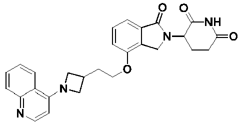

Step 1: 2-(1-(tert-butoxycarbonyl) azetidin-3-) acetic acid (200mg, 0.93 mmol, 1.0 eq) was dissolved in 5ml of DMF and methyl iodide (70ul, 1.11 mmol, 1.2 eq) was added to react overnight at room temperature. After the reaction was completed, diluted with ethyl acetate, washed three times with saturated sodium chloride, dried, and the solvent was rotated away without further purification to obtain 213mg (100%) of colorless oil. ¹H NMR (400 MHz, CDCl₃) δ 4.09 (t, *J* = 8.6 Hz, 1H), 3.68 (s, 3H), 3.60 (dd, *J* = 8.8, 5.5 Hz, 1H), 2.91 - 2.84 (m, OH), 2.63 (d, *J* = 7.9 Hz, 1H), 1.43 (s, 3H).

Step 2: Methyl 2-(1-(tert-butoxycarbonyl) azetidine-3-) acetate (213mg, 0.93 mmol, 1.0 eq) was dissolved in 5ml of dichloromethane, 5ml of trifluoroacetic acid was added, and reacted at room temperature for 30 minutes. After the reaction was completed, the solvent was rotated away. A yellow oil was obtained. The yellow oil was dissolved in 10ml of DMF, 4-chloro-quinoline (304mg, 1.86 mmol, 2.0 eq) and anhydrous potassium carbonate (524mg, 3.72 mmol, 4.0 eq) were added, reacted at 120°C overnight. After the reaction was completed, diluted with ethyl acetate, washed with saturated sodium chloride, and purified by column chromatography to obtain 134mg of methyl 2-(1-(qinolin-4 -) azetidin-3-) acetate as a yellow oil, yield 56%; ¹H NMR (400 MHz, CDCl₃) δ 8.54 (d, *J* = 5.3 Hz, 1H), 8.01 (d, *J* = 8.6 Hz, 1H), 7.93 (d, *J* = 8.5 Hz, 1H), 7.63 (s, 1H), 7.38 (d, *J* = 7.1 Hz, 1H), 6.20 (d, *J* = 5.3 Hz, 1H), 4.60 (t, *J* = 8.1 Hz, 2H), 4.09 (dd, *J* = 8.1, 5.6 Hz, 2H), 3.75 (s, 3H), 3.23 (ddd, *J* = 13.3, 7.9, 5.4 Hz, 2H), 2.82 (d, *J* = 7.8 Hz, 2H).

Step 3: Methyl 2-(1-(quinolin-4-) azetidin-3-) acetate (152mg, 0.59 mmol, 1.0 eq) was dissolved in 10ml of tetrahydrofuran, and DIBAL-H (1M, 1.25 ml, 2.1 eq) was added under ice bath conditions. After the reaction was completed, the solvent was rotated away, and 94mg of 2-(1-(quinoline-4-) azetidin-3-) ethanol was obtained by purification on column chromatography, yellow oil, yield 70%; ¹H NMR (400 MHz, CDCl₃) δ 8.48 (d, *J* = 5.3 Hz, 1H), 7.97 (d, *J* = 7.9 Hz, 1H), 7.92 (d, *J* = 7.8 Hz, 1H), 7.59 (ddd, *J* = 8.3, 6.9, 1.3 Hz, 1H), 7.34 (ddd, *J* = 8.2, 6.9, 1.2 Hz, 1H), 6.15 (d, *J* = 5.3 Hz, 1H), 4.53 (t, *J* = 8.1 Hz, 2H), 4.06 (dd, *J* = 7.9, 5.8 Hz, 2H), 3.76 (t, *J* = 6.2 Hz, 2H), 3.00 (ddd, *J* = 13.6, 7.7, 5.8 Hz, 1H), 1.99 (dd, *J* = 13.6, 6.3 Hz, 3H).

Step 4: methyl 5-amino-4-(4-hydroxy-1-oxoisoindolin-2-)-5-oxopentanoate (50mg, 0.17 mmol, 1.0 eq), 2-(1-(quinolin-4-) azetidin-3-) ethanol (78mg, 0.34 mmol, 2.0 eq) were dissolved in 20ml of tetrahydrofuran, triphenylphosphine (89mg, 0.34 mmol, 2.0 eq) was added until completely dissolved, azobisisobutyronitrile (67ul, 0.34 mmol, 2.0 eq) was added, and reacted at room temperature for 2 hours. After completion of the reaction, the solvent was removed and purified by TLC to give 68mg of methyl 5-amino-5-oxo-4-(1-oxo-4-(2-(1-(quinolin-4-) azetidin-3-) ethyoxy) isoindolin-2-) oxopentanoate as a white solid, yield 82%; ¹H NMR (400 MHz, CDCl₃) δ 8.49 (d, *J* = 5.4 Hz, 1H), 8.02 (d, *J* = 8.3 Hz, 1H), 7.93 (d, *J* = 8.4 Hz, 1H), 7.61 (t, *J* = 7.3 Hz, 1H), 7.48 - 7.38 (m, 3H), 7.02 (dd, *J* = 6.4, 2.4 Hz, 1H), 6.43 (s, 1H), 6.19 (d, *J* = 5.5 Hz, 1H), 5.54 (s, 1H), 4.93 (dd, *J* = 8.8, 6.3 Hz, 1H), 4.59 (dd, *J* = 14.6, 7.9 Hz, 2H), 4.54 - 4.47 (m, 1H), 4.39 (d, *J* = 17.4 Hz, 1H), 4.18 - 4.11 (m, 4H), 3.61 (d, *J* = 6.6 Hz, 3H), 3.17 - 3.02 (m, 1H), 2.48 - 2.17 (m, 7H).

Step 5: methyl 5-amino-5-oxo-4-(1-oxo-4-(2-(1-(quinolin-4-) azetidin-3-) ethoxy) isoindolin-2-) valerate (80mg, 0.16 mmol, 1.0 eq) was dissolved in 10ml of dry tetrahydrofuran, potassium tert-butoxide (18mg, 0.16 mmol, 1eq) was added under ice bath conditions, and the detection of the reaction was started 10 minutes later. After completion of the reaction, 5ul of formic acid was added to quench the reaction, the solvent was rotated away and purified by HPLC to give 11mg of 3-(1-oxo-4-(2-(1-(quinolin-4-) azridin-3-) ethoxy) isoindolin-2-) piperidine-2, 6-dione as a white solid, yield 14%;¹H NMR (400 MHz, DMSO) δ 13.56 (s, 1H), 11.02 (s, 1H), 8.42 (dd, *J* = 6.6, 4.8 Hz, 1H), 8.19 (d, *J* = 9.8 Hz, 1H), 7.96 ― 7.90 (m, 1H), 7.87 (dd, *J* = 8.4, 1.0 Hz, 1H), 7.60 (t, *J* = 7.7 Hz, 1H), 7.50 (t, *J* = 7.8 Hz, 1H), 7.33 (d, *J* = 7.4 Hz, 1H), 7.27 (d, *J* = 7.9 Hz, 1H), 6.42 (d, *J* = 7.1 Hz, 1H), 5.14 (dd, *J* = 13.5, 5.3 Hz, 2H), 4.87 - 4.78 (m, 1H), 4.58 (d, *J* = 11.1 Hz, 1H), 4.42 (dd, *J* = 11.7, 8.7 Hz, 1H), 4.25 (dd, *J* = 18.7, 12.0 Hz, 4H), 3.15 (s, 1H), 3.00 - 2.88 (m, 1H), 2.61 (d, *J* = 15.4 Hz, 1H), 2.40 (ddd, *J* = 26.0, 15.8, 9.0 Hz, 2H), 2.22 (dd, *J* = 13.5, 7.9 Hz, 2H), 2.08 - 1.97 (m, 1H). UPLC - MS (ESI) calculated for C₂₇H₂₆N₄O₄ [M + H]+: 471.20, found:471.81.

### EXAMPLE 70 : 3-(1-oxo-4-(((R)-1-(4-(trifluoromethoxy) phenyl) pyrrolin-3-) methoxy) isoindolin-2-) piperidine-2, 6-dione (70)

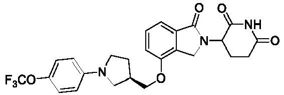

Step 1: The preparation method was the same as that of the intermediate (*R*)-1-(4-trifluoromethoxyphenyl) pyrrolin-3-methanol, (*S*)-1-(4-trifluoromethoxyphenyl)-pyrrolin-3-methanol was obtained, yield 17%.

Step 2: (*S*)-1-(4-trifluoromethoxyphenyl) pyrrolin-3-methanol and intermediate 6 were used as raw materials, and the preparation method was the same as the synthesis route 2, and 36.9 mg of 3-(1-oxo-4-(((*R*)-1-(4-(trifluoromethoxy) phenyl) pyrrolin-3-) methoxy) isoindoline-2-) piperidine-2, 6-dione as a white solid, yield 39%; ¹H NMR (400 MHz, DMSO) δ 10.97 (s, 1H), 7.48 (t, J = 7.8 Hz, 1H), 7.30 (dd, J = 18.5, 7.8 Hz, 2H), 7.14 (d, J = 8.4 Hz, 2H), 6.57 (d, J = 9.1 Hz, 2H), 5.11 (dd, J = 13.3, 5.1 Hz, 1H), 4.40 (d, J = 17.4 Hz, 1H), 4.22 (d, J = 17.4 Hz, 1H), 4.20-4.13 (m, 2H), 3.46 (dd, J = 9.5, 7.5 Hz, 1H), 3.41 - 3.34 (m, 1H), 3.29-3.25 (m, 1H), 3.17 (dd, J = 9.6, 6.3 Hz, 1H), 2.97 - 2.77 (m, 2H), 2.63 - 2.54 (m, 1H), 2.48 - 2.37 (m, 1H), 2.20 (td, J = 12.4, 7.4 Hz, 1H), 2.04 - 1.88 (m, 2H). UPLC - MS (ESI) calculated for C₂₅H₂₄F₃N₃O₅ [M + H]+: 504.17, found:504.24.

### EXAMPLE 71: 3-(1-oxo-4-(((S)-1-(4-(trifluoromethoxy) phenyl) pyrrolin-3-) methoxy) isoindolin-2-) piperidine-2, 6-dione (71)

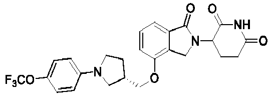

Step 1: (S)-pyrrolidin-3-methanol (101mg, 1mmol), 4-bromotrifluoromethoxybenzene (361.5 mg, 1.5 mmol), Pd (OAc)₂ (13.4 mg, 0.06 mmol), BINAP (75mg, 0.12 mmol) and Cs₂CO₃(652mg, 2mmol) were suspended in dry toluene (10ml) and heated to 90°C, and reacted overnight under the protection of N2, after the reaction was completed, the reaction solution was filtered by diatomite, the filtrate was concentrated under reduced pressure, and the residue was subjected to silica gel column chromatography to obtain (*R*)-1-(4-trifluoromethoxyphenyl) pyrrolin-3-methanol 83mg, pink oil, yield 32%; ¹H NMR (400 MHz, DMSO) δ 7.13 (d, *J* = 8.8 Hz, 2H), 6.53 (d, *J* = 8.8 Hz, 2H), 4.69 (s, 1H), 3.47 - 3.35 (m, 2H), 3.33 - 3.17 (m, 4H), 2.43 (m, 1H), 2.03 (m, 1H), 1.74 (m, 1H).

Step 2: (*R*)-1-(4-trifluoromethoxyphenyl) pyrrolin-3-methanol and intermediate 4 were used as raw materials, and the preparation method was the same as the Example 70, and 9.3 mg of 3-(1-oxo-4-(((*S*)-1-(4-(trifluoromethoxy) phenyl) pyrrolin-3-) methoxy) isoindoline-2-) piperidine-2, 6-dione, yield 10 %; ¹H NMR (400 MHz, DMSO) δ 10.98 (s, 1H), 7.48 (t, *J* = 7.8 Hz, 1H), 7.29 (dd, *J* = 18.7, 7.8 Hz, 2H), 7.14 (d, *J* = 8.4 Hz, 2H), 6.58 (d, *J* = 9.1 Hz, 2H), 5.10 (dd, *J* = 13.3, 5.1 Hz, 1H), 4.33 (d, *J* = 17.5 Hz, 1H), 4.24 (d, *J* = 17.4 Hz, 1H), 4.21 - 4.12 (m, 2H), 3.47 (dd, *J* = 9.5, 7.6 Hz, 1H), 3.41 - 3.24 (m, 2H), 3.17 (dd, *J* = 9.5, 6.2 Hz, 1H), 2.96 - 2.86 (m, 1H), 2.82 (dt, *J* = 13.5, 6.8 Hz, 1H), 2.63 - 2.54 (m, 1H), 2.46 - 2.34 (m, 1H),2.24-2.16(m, 1H), 2.01 - 1.88 (m, 2H). UPLC - MS (ESI) calculated for C₂₅H₂₄F₃N₃O₅ [M + H]+: 504.17, found:504.24.

### EXAMPLE 72: 3-(1-oxo-4-((4-(4-(trifluoromethoxy) phenyl) cyclohexyl) methoxy) isoindolin-2-) piperidine-2, 6-dione (72)

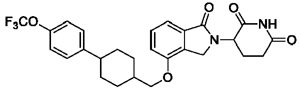

The preparation method referred to the synthetic route 2, and 45.7 mg of the example compound 72 was obtained, yield 52%; ¹H NMR (400 MHz, DMSO) δ 10.96 (s, 1H), 7.49 (td, *J* = 7.8, 5.3 Hz, 1H), 7.35 (dddd, *J* = 22.6, 14.3, 6.7, 5.5 Hz, 6H), 5.11 (ddd, *J* = 13.2, 5.1, 3.7 Hz, 1H), 4.39 (dd, *J* = 17.5, 6.0 Hz, 1H), 4.28 - 4.19 (m, 2H), 3.99 (d, *J* = 5.8 Hz, 1H), 2.99 - 2.84 (m, 1H), 2.61 (ddd, *J* = 31.9, 16.7, 6.4 Hz, 2H), 2.49 ― 2.38 (m, 1H), 2.26 ― 2.15 (m, 1H), 2.04 ― 1.92 (m, 2H), 1.85 (dd, *J* = 10.4, 2.0 Hz, 2H), 1.66 (ddd, *J* = 14.2, 8.9, 3.8 Hz, 3H), 1.57 - 1.42 (m, 1H), 1.26 (ddd, *J* = 20.0, 13.0, 5.0 Hz, 1H). UPLC - MS (ESI) calculated for C₂₇H₂₇F₃N₂O₅ [M + H]+: 517.19, found:517.18.

### EXAMPLE 73:3-(1-oxo-4-((1-(4-(trifluoromethoxy) phenyl) piperidin-4-) methoxy) isoindolin-2-) piperidin-2, 6-dione (73)

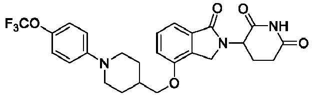

The preparation method referred to the synthetic route 2 and Example 70, and 35.8 mg of the example compound 73 was obtained, yield 42%; ¹H NMR (400 MHz, DMSO) δ 10.97 (s, 1H), 7.48 (t, *J* = 7.8 Hz, 1H), 7.31 (d, *J* = 7.5 Hz, 1H), 7.25 (d, *J* = 8.1 Hz, 1H), 7.17 (d, *J* = 9.0 Hz, 2H), 7.01 (d, *J* = 9.2 Hz, 2H), 5.11 (dd, *J* = 13.3, 5.1 Hz, 1H), 4.39 (d, *J* = 17.5 Hz, 1H), 4.24 (d, *J* = 17.5 Hz, 1H), 4.03 (d, *J* = 5.9 Hz, 2H), 3.74 (d, *J* = 12.4 Hz, 2H), 2.97 - 2.85 (m, 1H), 2.72 (t, *J* = 11.6 Hz, 2H), 2.62 - 2.54 (m, 1H), 2.49 - 2.39 (m, 1H), 2.03 - 1.92 (m, 2H), 1.89 (d, *J* = 13.4 Hz, 2H), 1.44 (qd, *J* = 12.0, 3.7 Hz, 2H). UPLC - MS (ESI) calculated for C₂₆H₂₆F₃N₃O₅ [M + H]+: 518.18, found:518.39.

### EXAMPLE 74: 3-(4-((1-(4-chlorophenyl)-1H-1, 2, 3-triazol-4-yl) methoxy)-1-oxoisoindolin-2-yl) piperidine-2, 6-dione (74)

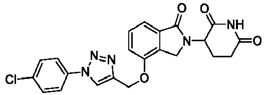

Azide compound was prepared as synthesis method 1 of azides, the compound was prepared as synthesis route 1 and Example 1, ¹H NMR (400 MHz, DMSO) δ 10.97 (s, 1H), 9.01 (s, 1H), 8.00 - 7.92 (m, 2H), 7.72 - 7.65 (m, 2H), 7.56 - 7.46 (m, 2H), 7.36 (d, *J* = 6.4 Hz, 1H), 5.41 (s, 2H), 5.11 (dd, *J* = 13.3, 5.1 Hz, 1H), 4.39 (d, *J* = 17.5 Hz, 1H), 4.23 (d, *J* = 17.5 Hz, 1H), 2.98 - 2.83 (m, 1H), 2.62 - 2.53 (m, 1H), 2.42 (ddd, *J* = 26.7, 13.3, 4.4 Hz, 1H), 2.02 - 1.94 (m, 1H). UPLC - MS (ESI) calculated for C₂₂H₁₈ClN₅O₄ [M + H]+: 452.10, found:452.30.

### EXAMPLE 75: 3-(4-((1-(3, 4-dichlorophenyl)-1H-1, 2, 3-triazol-4-yl) methoxy)-1-isoindolin-2-yl) piperidine-2, 6-dione (75)

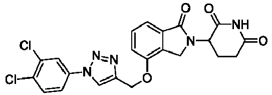

Azide compound was prepared as synthesis method 1 of azides, the compound was prepared as synthesis route 1 and Example 1, ¹H NMR (400 MHz, DMSO) δ 10.97 (s, 1H), 9.07 (s, 1H), 8.29 (t, *J* = 3.0 Hz, 1H), 8.01 ― 7.96 (m, 1H), 7.90 (d, *J* = 8.8 Hz, 1H),7.56-7.44 (m, 2H), 7.34 (dd, *J* = 13.8, 7.0 Hz, 1H), 5.42 (s, 2H), 5.11 (dd, *J* = 13.3, 5.0 Hz, 1H), 4.39 (d, *J* = 17.5 Hz, 1H), 4.23 (d, *J* = 17.5 Hz, 1H), 2.96 - 2.84 (m,1H), 2.61 - 2.53 (m,1H), 2.48 - 2.39 (m, 1H), 2.02 - 1.95 (m, 1H). UPLC - MS (ESI) calculated for C₂₂H₁₇Cl₂N₅O₄ [M + H]+: 486.07, found:486.21.

### EXAMPLE 76: 3-(4-((1-((3S, 5S, 7S)-adamantan-1-yl)-1H-1, 2, 3-triazol-4-yl) methoxy)-1-isoindolin-2-yl) piperidin-2, 6-dione (76)

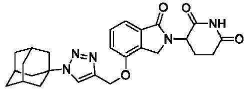

Azide compound was prepared as synthesis method 6 of azides, the compound was prepared as synthesis route 1 and Example 1, ¹H NMR (400 MHz, DMSO) δ 10.97 (s, 1H), 8.36 (s, 1H), 7.55 - 7.44 (m, 2H), 7.34 (d, *J* = 6.8 Hz, 1H), 5.27 (s, 2H), 5.10 (dd, *J* = 13.3, 5.0 Hz, 1H), 4.36 (d, *J* = 17.5 Hz, 1H), 4.19 (d, *J* = 17.5 Hz, 1H), 2.90 (ddd, *J* = 18.7, 13.6, 5.1 Hz, 1H), 2.60 - 2.53 (m, 1H),2.48-2.38 (m, 1H), 2.18 (s, 9H), 2.00 - 1.93 (m, 1H), 1.74 (s, 6H). UPLC - MS (ESI) calculated for C₂₆H₂₉N₅O₄ [M + H]+: 476.22, found:476.45.

### EXAMPLE 77: 3-(6-fluoro-1-oxo-4-((1-(4-(trifluoromethoxy) phenyl)-1H-1, 2, 3-triazol-4-yl) methoxy) isoindolin-2-yl) piperidine-2, 6-dione (77)

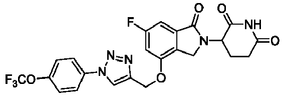

Azide compound was prepared as synthesis method 1 of azides, the compound was prepared as synthesis route 1 and Example 1, ¹H NMR (400 MHz, DMSO) δ 10.98 (s, 1H), 9.03 (s, 1H), 8.09 - 8.02 (m, 2H), 7.65 (d, *J* = 8.5 Hz, 2H), 7.48 (dd, *J* = 11.4, 2.0 Hz, 1H), 7.15 (dd, *J* = 7.3, 2.0 Hz, 1H), 5.43 (s, 2H), 5.11 (dd, *J* = 13.3, 5.1 Hz, 1H), 4.36 (d, *J* = 17.4 Hz, 1H), 4.20 (d, *J* = 17.4 Hz, 1H), 2.90 (ddd, *J* = 17.3, 13.5, 5.0 Hz, 1H), 2.61 - 2.53 (m, 1H), 2.47-2.35 (m, 1H), 2.02-1.92 (m, 1H). UPLC - MS (ESI) calculated for C₂₃H₁₇F₄N₅O₅ [M + H]+: 520.12, found:520.26.

### EXAMPLE 78: 3-(1-oxo-4-((1-(phenyl-D5)-1H-1, 2, 3-triazol-4-yl) methoxy) isoindolin-2-yl) piperidine-2, 6-dione (78)

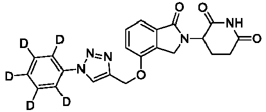

Azide compound was prepared as synthesis method 1 of azides, the compound was prepared as synthesis route 1 and Example 1, ¹H NMR (400 MHz, DMSO) δ 10.97 (s, 1H), 8.98 (s, 1H), 7.57 - 7.46 (m, 2H), 7.36 (dd, *J* = 7.0, 0.9 Hz, 1H), 5.41 (s, 2H), 5.11 (dd, *J* = 13.3, 5.1 Hz, 1H), 4.39 (d, *J* = 17.5 Hz, 1H), 4.23 (d, *J* = 17.5 Hz, 1H), 2.97 - 2.85 (m, 1H), 2.62 - 2.53 (m, 1H), 2.47 - 2.36 (m, 1H), 2.02 - 1.94 (m, 1H). UPLC - MS (ESI) calculated for C₂₂H₁₄D₅N₅O₄ [M + H]+: 423.18, found:423.34.

### EXAMPLE 79: 3-(6-fluoro-1-oxo-4-((1-(phenyl-D5)-1H-1, 2, 3-triazol-4-yl) methoxy) isoindolin-2-yl) piperidine-2, 6-dione (79)

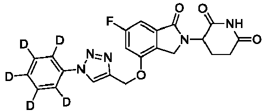

Azide compound was prepared as synthesis method 1 of azides, the compound was prepared as synthesis route 1 and Example 1, ¹H NMR (400 MHz, DMSO) δ 10.98 (s, 1H), 8.99 (s, 1H), 7.48 (dd, *J* = 11.4, 2.0 Hz, 1H), 7.15 (dd, *J* = 7.3, 2.0 Hz, 1H), 5.42 (s, 2H), 5.10 (dd, *J* = 13.3, 5.0 Hz, 1H), 4.36 (d, *J* = 17.5 Hz, 1H), 4.21 (d, *J* = 17.5 Hz, 1H), 2.90 (ddd, *J* = 17.0, 13.7, 5.1 Hz, 1H), 2.61 - 2.53 (m, 1H), 2.47-2.35 (m, 1H), 2.02 - 1.93 (m, 1H). UPLC - MS (ESI) calculated for C₂₂H₁₃D₅FN₅O₄ [M + H]+: 441.17, found:441.34.

### EXAMPLE 80: 3-(6-fluoro-1-oxo-4-((2-(4-(trifluoromethoxy) phenyl) oxazol-5-yl) methoxy) isoindolin-2-yl) piperidine-2, 6-dione (80)

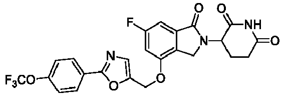

The preparation method was the same as that of Synthetic Route 2 and Example 45, ¹H NMR (400 MHz, DMSO) δ 10.96 (s, 1H), 8.11 (d, *J* = 8.9 Hz, 2H), 7.58 (s, 1H), 7.55 (d, *J* = 8.2 Hz, 2H), 7.47 (dd, *J* = 11.4, 2.0 Hz, 1H), 7.17 (dd, *J* = 7.4, 2.0 Hz, 1H), 5.44 (s, 2H), 4.37 (d, *J* = 17.4Hz, 1H), 4.20 (d, *J* = 17.4 Hz, 1H), 2.89 (ddd, *J* = 17.6, 13.6, 5.2 Hz, 1H), 2.61 - 2.53 (m,1H), 2.48 - 2.39 (m, 1H), 2.00 - 1.92 (m, 1H). UPLC - MS (ESI) calculated for C₂₄H₁₇F₄N₃O₆ [M + H]+: 520.11, found:520.29.

### EXAMPLE 81: 3-(6-fluoro-1-oxo-4-((5-(4-(trifluoromethoxy) phenyl) oxazol-2-yl) methoxy) isoindolin-2-yl) piperidine-2, 6-dione (81)

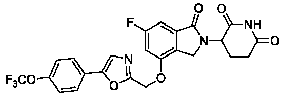

The preparation method was the same as that of Synthetic Route 2 and Example 40, ¹H NMR (400 MHz, DMSO) δ 10.99 (s, 1H), 7.85 (d, *J* = 8.8 Hz, 2H), 7.82 (s, 1H), 7.50 (d, *J* = 8.2 Hz, 2H), 7.44 (dd, *J* = 11.3, 2.0 Hz, 1H), 7.18 (dd, *J* = 7.2, 2.0 Hz, 1H), 5.50 (s, 2H), 5.10 (dd, *J* = 13.3, 5.0 Hz, 1H), 4.40 (d, *J* = 17.2 Hz, 1H), 4.24 (d, *J* = 17.2 Hz, 1H), 2.96 - 2.84 (m,1H), 2.61 - 2.53 (m,1H), 2.48 - 2.39 (m, 1H), 2.06 - 1.92 (m, 1H). UPLC―MS (ESI) calculated for C₂₄H₁₇F₄N₃O₆ [M + H]+: 520.11, found:520.29.

### EXAMPLE 82: 3-(1-oxo-4-((2-(4-(trifluoromethoxy) phenyl) oxazol-5-yl) methyl) amino) isoindolin-2-yl) piperidine-2, 6-dione (82)

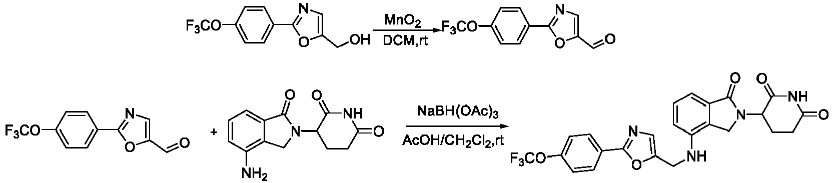

Step 1: (2-(4-(trifluoromethoxy) phenyl) oxazol-5-yl) methanol (170mg, 0.66 mmol) was dissolved in dry dichloromethane, manganese dioxide (570mg, 6.56 mmol) was added under stirring conditions, the reaction system reacted overnight at room temperature, when TLC monitored that the reaction was completed, filtrated with diatomite, the filtrate was concentrated under reduced pressure, and flash column chromatography was used to give 164mg of white solid, yield 98%. ¹H NMR (400 MHz, CDCl₃) δ 9.83 (s, 1H), 8.26 - 8.21 (m, 2H), 7.95 (s, 1H), 7.36 (d, *J* = 8.2 Hz, 2H)

Step 2: 2-(4-(trifluoromethoxy) phenyl) oxazole-5-carboxaldehyde (56mg, 0.220 mmol) and lenalidomide (38mg, 0.147 mmol) were dissolved in 2ml of acetic acid and 2ml of dichloromethane at room temperature. After stirring for 1 hour, sodium triacetoxyborohydride (93mg, 0.44 mmol) was added, and the reaction was carried out overnight at room temperature under nitrogen protection. When TLC was used to monitor the reaction and indicated that the reaction was completed, the reaction solution was concentrated under reduced pressure, saturated sodium bicarbonate solution was added to adjust the pH to about 8, ethyl acetate (30 ml × 2) was added for extraction, the liquid was separated, the organic layer was washed with saturated sodium chloride once, dried over anhydrous sodium sulfate, filtered, the filtrate was concentrated under reduced pressure, and purified by HPLC to obtain 44mg of a white solid with a yield of 60%. ¹H NMR (400 MHz, DMSO) δ 10.99 (s, 1H), 8.04 (d, *J* = 8.9 Hz, 2H), 7.53 (d, *J* = 8.2 Hz, 2H), 7.32 (t, *J* = 7.7 Hz, 1H), 7.27 (s, 1H), 7.00 (d, *J* = 7.4 Hz, 1H), 6.95 (d, *J* = 8.0 Hz, 1H), 6.32 (t, *J* = 5.8 Hz, 1H), 5.09 (dd, *J* = 13.2, 5.1 Hz, 1H), 4.54 (d, *J* = 5.8 Hz, 2H), 4.28 (d, *J* = 17.2 Hz, 1H), 4.17 (d, *J* = 17.2 Hz, 1H), 2.96 - 2.84 (m, 1H), 2.65 - 2.56 (m, 1H), 2.35 - 2.23 (m, 1H), 2.07-2.00 (m, 1H). UPLC - MS (ESI) calculated for C₂₄H₁₉F₃N₄O₅ [M + H]+: 501.14, found:501.28.

### EXAMPLE 83: 3-(4-((1-(2, 6-dichloro-4-(trifluoromethyl) phenyl)-5-methyl-1H-pyrazol-4-yl) methoxy)-1-isoindolin-2-yl)piperidine-2, 6-dione(83)

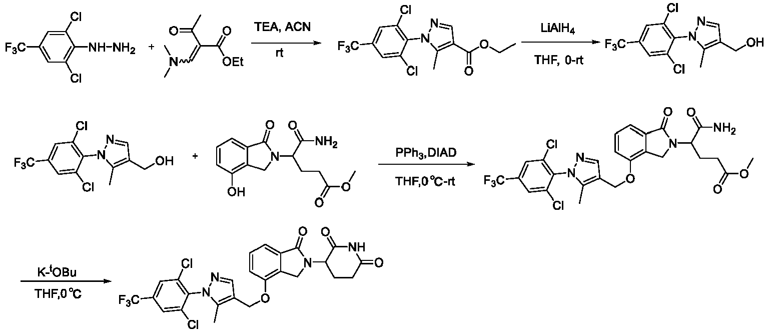

Step 1: triethylamine (284µL, 2.04 mmol) was added to the suspension of (2, 6-dichloro-4-(trifluoromethyl) phenyl) hydrazine (500mg, 2.04 mmol) and ethyl 2-acetyl-3-(dimethylamino) acrylate (378mg, 2.04 mmol) in acetonitrile (20 ml) at room temperature, and stirred overnight. The TLC was used to monitor and indicated that the reaction was completed, concentrated under reduced pressure, and 630mg of product was obtained by rapid silica gel column chromatography, yield 84%.

Step 2: ethyl 1-(2, 6-dichloro-4-(trifluoromethyl) phenyl)-5-methyl-1H-pyrazole-4-carboxylate (630mg, 1.72 mmol) was dissolved in 15ml of dry tetrahydrofuran, cooled under ice bath, 1mol/L tetrahydroaluminum lithium in tetrahydrofuran (2.6 mL) was added dropwise, after dropwise addition, the reaction was raised to room temperature for 1 hour, TLC was used to monitor the completion of the reaction, ice water was added to quench, filtrated, the filtrate was concentrated under reduced pressure, and rapid silica gel column chromatography was performed to obtain 100mg of the product with a yield of 18%. ¹H NMR (400 MHz, DMSO) δ 8.24 (s, 2H), 7.68 (s, 1H), 4.92 (t, *J* = 5.4 Hz, 1H), 4.39 (d, *J* = 5.4 Hz, 2H), 2.03 (s, 3H).

Step 3: (1-(2, 6-dichloro-4-(trifluoromethyl) phenyl)-5-methyl-1H-pyrazol-4-yl) methanol (80mg, 0.246 mmol), methyl 5-amino-4-(4-hydroxy-1-oxoisoindolin-2-yl)-5-oxopentanoate (48mg, 0.164 mmol) and triphenylphosphine (64.5 mg, 0.246 mmol) were placed in a 25ml round bottom flask. The reaction system was replaced with nitrogen, and 5 mL of dry tetrahydrofuran was added. Diisopropyl azodicarboxylate (48µL, 0.246mmol) was added to the reaction system. The reaction system reacted at room temperature for 3h. The reaction was monitored by TLC until completion, and concentrated under reduced pressure, and 72mg of product was obtained by column chromatography with a yield of 73%.

Step 4: the product obtained in the previous step (72mg, 0.12mmol) was dissolved in dry THF, and potassium tert-butoxide (15mg, 0.13mmol) was added at 0°C, and reacted at the same temperature for 30 min, 1N HC1 was added to quench, diluted with ethyl acetate, washed with saturated sodium chloride, dried, and purified by HPLC to obtain 36mg of white solid, yield 53%; ¹H NMR (400 MHz, DMSO) δ 10.96 (s, 1H), 8.26 (s, 2H), 7.89 (s, 1H), 7.52 (t, *J* = 7.8 Hz, 1H), 7.42 (d, *J* = 8.1 Hz, 1H), 7.34 (d, *J* = 7.4 Hz, 1H), 5.19 (s, 2H), 5.10 (dd, *J* = 13.3, 5.1 Hz, 1H), 4.38 (d, *J* = 17.3 Hz, 1H), 4.24 (d, *J* = 17.3Hz, 1H), 2.96 - 2.85 (m, 1H), 2.61 - 2.53 (m,1H), 2.48 - 2.39 (m, 1H), 2.10 (s, 3H), 2.00 - 1.93 (m, 1H). UPLC - MS (ESI) calculated for C₂₅H₁₉Cl₂F₃N₄O₄ [M + H]⁺: 567.08, found:567.21.

### EXAMPLE 84: 3-(4-((1-(2, 6-dichloro-4-(trifluoromethoxy) phenyl)-5-methyl-1H-pyrazol-4-yl) methoxy)-1-isoindolin-2-yl) piperidine-2, 6-dione (84)

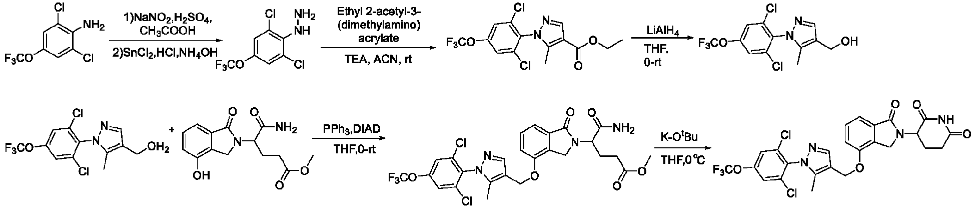

Step 1: concentrated sulfuric acid (1mL) and NaNO₂ (297 mg, 4.31 mmol) were added to a 50mL round bottom flask, cooled to 5-10°C. A solution of 2, 6-dichloro-4-(trifluoromethoxy) aniline (1g, 4.06 mmol) in acetic acid (4 mL) was added dropwise, stirred for 10 minutes, reacted at room temperature for 30 minutes, and then placed at 60°Cand reacted for 1h. The reaction system was cooled to 0°C,a solution of tin dichloride (3.16 g, 16.67 mmol) in 37% hydrochloric acid (2.5 mL) was added, reacted for 20 minutes, filtered, the residue was added to a mixture of 28% ammonia (30 mL) and ice, stirred for minutes, the reaction system was extracted with diethyl ether (100 mL × 2), combined the organic layers, washed with saturated sodium chloride solution, dried, and concentrated under reduced pressure to give 689mg of a white solid with a yield of 65%. ¹H NMR (400 MHz, DMSO) δ 7.50 (s, 2H), 6.12 (s, 1H), 4.46 (s, 2H).

Step 2: triethylamine (367µL, 2.64mmol) was added to the suspension of (2, 6-dichloro-4-(trifluoromethoxy) phenyl) hydrazine (689mg, 2.64mmol) and ethyl 2-acetyl-3-(dimethylamino) acrylate (489mg, 2.04 mmol) in acetonitrile (15 ml) at room temperature, and stirred overnight. The TLC was used to monitor that the reaction was completed, concentrated under reduced pressure, and 883mg of product was obtained by rapid silica gel column chromatography, yield 87%.

Step 3: ethyl 1-(2, 6-dichloro-4-(trifluoromethoxy) phenyl)-5-methyl-1H-pyrazole-4-carboxylate (883mg, 2.30mmol) was dissolved in 15ml of dry tetrahydrofuran, cooled under ice bath, 1mol/L tetrahydroaluminum lithium in tetrahydrofuran (3.45 mL) was added dropwise, after dropwise addition, the reaction was raised to room temperature for 1 hour, TLC was used to monitor the completion of the reaction, ice water was added to quench, filtrated, the filtrate was concentrated under reduced pressure, and rapid silica gel column chromatography was performed to obtain 563mg of the product with a yield of 72%. ¹H NMR (400 MHz, DMSO) δ 7.95 (s, 2H), 7.65 (s, 1H), 4.91 (t, *J* = 5.4 Hz, 1H), 4.38 (d, *J* = 5.4 Hz, 2H), 2.02 (s, 3H).

Step 4: (1-(2, 6-dichloro-4-(trifluoromethoxy) phenyl)-5-methyl-1H-pyrazol-4-yl) methanol (140mg, 0.410mmol), methyl 5-amino-4-(4-hydroxy-1-oxoisoindolin-2-yl)-5-oxopentanoate (80mg, 0.274mmol) and triphenylphosphine (108mg, 0.410mmol) were placed in a 25ml round bottom flask. The reaction system was replaced with nitrogen, and 5 mL of dry tetrahydrofuran was added. Diisopropyl azodicarboxylate (81µL, 0.410mmol) was added to the reaction system. The reaction system reacted at room temperature for 3h. The reaction was monitored by TLC until completion, and concentrated under reduced pressure, and 145mg of product was obtained by column chromatography with a yield of 86%.

Step 5: the product obtained in the previous step (145mg, 0.237mmol) was dissolved in dry THF (5mL), and potassium tert-butoxide (29mg, 0.259mmol) was added at 0°C, and reacted at the same temperature for 30 min, 1N HC1 was added to quench, diluted with ethyl acetate, washed with saturated sodium chloride, dried, and purified by HPLC to obtain 94mg of white solid, yield 68.4%; ¹H NMR (400 MHz, DMSO) δ 10.96 (s, 1H), 7.96 (s, 2H), 7.87 (s, 1H), 7.52 (t, *J* = 7.8 Hz, 1H), 7.42 (d, *J* = 8.0 Hz, 1H), 7.34 (d, *J* = 7.3 Hz, 1H), 5.18 (s, 2H), 5.10 (dd, *J* = 13.3, 5.1 Hz, 1H), 4.38 (d, *J* = 17.4 Hz, 1H), 4.23 (d, *J* = 17.4 Hz, 1H), 2.96 - 2.85 (m, 1H), 2.61 - 2.54 (m, 1H), 2.48 - 2.39 (m, 1H), 2.09 (s, 3H), 2.02-1.94 (m, 1H). UPLC - MS (ESI) calculated for C₂₅H₁₉Cl₂F₃N₄O₅ [M + H]+: 583.08, found:583.26.

### EXAMPLE 85: 3-(4-((1-(2, 6-dichloro-4-(trifluoromethoxy)phenyl)-5-methyl-1H-pyrazol-3-yl) methoxy)-1-isoindolin-2-yl)piperidine-2, 6-dione(85)

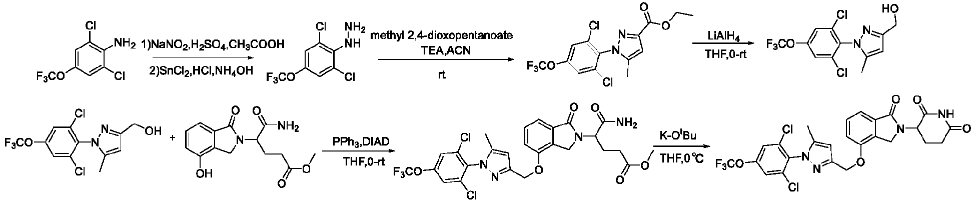

Step 1: concentrated sulfuric acid (1mL) and NaNO₂(297mg, 4.31 mmol) were added to a 50mL round bottom flask, cooled to 5-10°C. A solution of 2, 6-dichloro-4-(trifluoromethoxy) aniline (1g, 4.06mmol) in acetic acid (4 mL) was added dropwise, stirred for 10 minutes, reacted at room temperature for 30 minutes, and then placed at 60°Cand reacted for 1h. The reaction system was cooled to 0°C, a solution of tin dichloride (3.16 g, 16.67mmol) in 37% hydrochloric acid (2.5 mL) was added, reacted for 20 minutes, filtered, the residue was added to a mixture of 28% ammonia (30 mL) and ice, stirred for minutes, the reaction system was extracted with diethyl ether (100 mL × 2), combined the organic layers, washed with saturated sodium chloride solution, dried, and concentrated under reduced pressure to give 689mg of a white solid with a yield of 65%. ¹H NMR (400 MHz, DMSO) δ 7.50 (s, 2H), 6.12 (s, 1H), 4.46 (s, 2H).

Step 2: triethylamine (352µL, 2.53 mmol) was added to the suspension of (2, 6-dichloro-4-(trifluoromethoxy) phenyl) hydrazine (660mg, 2.53mmol) and methyl acetylpyruvate (399.6mg, 2.53 mmol) in acetonitrile (20 mL) at room temperature, and stirred overnight. The TLC was used to monitor that the reaction was completed, concentrated under reduced pressure, and 430mg of product was obtained by rapid silica gel column chromatography, yield 44%.

Step 3: ethyl 1-(2, 6-dichloro-4-(trifluoromethoxy) phenyl)-5-methyl-1H-pyrazole-3-carboxylate (430mg, 1.12mmol) was dissolved in 3ml of dry tetrahydrofuran, cooled under ice bath, 1mol/L tetrahydroaluminum lithium in tetrahydrofuran (1.35 mL) was added dropwise, after dropwise addition, the reaction was raised to room temperature for 4 hours, TLC was used to monitor the completion of the reaction, ice water was added to quench, filtrated, the filtrate was concentrated under reduced pressure, and rapid silica gel column chromatography was performed to obtain 90mg of the product with a yield of 10%. ¹H NMR (400 MHz, CDCl₃) δ 7.33 (d, *J* = 0.5 Hz, 2H), 6.24 (s, 1H), 4.43 (s, 2H), 2.33 (s, 3H).

Step 4: (1-(2, 6-dichloro-4-(trifluoromethoxy) phenyl)-5-methyl-1H-pyrazol-3-yl) methanol (90mg, 0.264mmol), methyl 5-amino-4-(4-hydroxy-1-oxoisoindolin-2-yl)-5-oxopentanoate (51.4mg, 0.176 mmol) and triphenylphosphine (69.2mg, 0.264mmol) were placed in a 25ml round bottom flask. The reaction system was replaced with nitrogen, and 5 mL of dry tetrahydrofuran was added. Diisopropyl azodicarboxylate (52µL, 0.264mmol) was added to the reaction system. The reaction system reacted at room temperature for 2h. The reaction was monitored by TLC until completion, and concentrated under reduced pressure, and 30.8mg of product was obtained by column chromatography with a yield of 28%.

Step 5: the product obtained in the previous step (30.8mg, 0.05mmol) was dissolved in dry THF (1mL), and potassium tert-butoxide (5.6mg, 0.05mmol) was added at 0°C, and reacted at the same temperature for 30 min, 1N HC1 was added to quench, diluted with ethyl acetate, washed with saturated sodium chloride, dried, and purified by HPLC to obtain 20mg of white solid, yield 68%; ¹H NMR (400 MHz, DMSO) δ 11.00 (s, 1H), 7.88 (s, 1H), 7.67 ― 7.52 (m, 1H), 7.45-7.41 (m, 1H), 7.28 (t, *J* = 7.5 Hz, 2H), 6.55 (s, 1H), 5.15 - 5.05 (m, 3H), 4.14 (d, *J* = 17.4 Hz, 1H), 4.04 (d, *J* = 17.3 Hz, 1H), 2.98 - 2.85 (m, 1H), 2.64 - 2.54 (m, 1H), 2.42-2.32(m, 1H), 2.25 (s,3H), 2.04 - 1.93 (m, 1H). UPLC - MS (ESI) calculated for C₂₅H₁₉Cl₂F₃N₄O₅ [M + H]+: 583.08, found:583.26.

### EXAMPLE 86: 3-(4-((1-((1R, 3S, 5R, 7S)-3-hydroxyadamantan-1-yl)-1H-1, 2, 3-triazol-4-yl) methoxy)-1-isoindolin-pyridin-2-yl) piperidin-2, 6-dione (86)

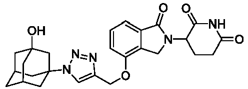

Azide compound was prepared as synthesis method 6 of azides, the compound was prepared as synthesis route 1, yield 47%. ¹H NMR (400 MHz, DMSO) δ 10.97 (s, 1H), 8.37 (s, 1H), 7.54 - 7.49 (m, 1H), 7.46 (d, *J* = 7.6 Hz, 1H), 7.34 (d, *J* = 6.8 Hz, 1H), 5.27 (s, 2H), 5.10 (dd, *J* = 13.3, 5.1 Hz, 1H), 4.80 (s, 1H), 4.36 (d, *J* = 17.5 Hz, 1H), 4.20 (d, *J* = 17.5 Hz, 1H), 2.96 - 2.84 (m, 1H), 2.61 - 2.53 (m, 1H), 2.48 - 2.36 (m, 1H), 2.31 (s, 2H), 2.11 - 1.93 (m, 7H), 1.71 - 1.50 (m, 6H). UPLC - MS (ESI) calculated for C₂₆H₂₉N₅O₅ [M + H]⁺: 492.22, found:492.39.

### EXAMPLE 87: 3-(4-((1-((1R, 3R, 5R, 7R)-adamantan-2-yl)-1H-1, 2, 3-triazol-4-yl) methoxy)-1-isoindolin-2-yl) piperidin-2, 6-dione (87)

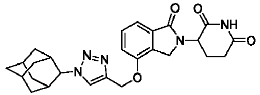

Azide compound was prepared as synthesis method 6 of azides, the compound was prepared as synthesis route 1, yield 22%. ¹H NMR (400 MHz, DMSO) δ 10.97 (s, 1H), 8.37 (s, 1H), 7.54 ― 7.43 (m, 2H), 7.34 (d, *J* = 6.6 Hz, 1H), 5.31 (s, 2H), 5.10 (dd, *J* = 13.3, 5.1 Hz, 1H), 4.57-4.55 (m, 1H), 4.35 (d, *J* = 17.5 Hz, 1H), 4.20 (d, *J* = 17.5 Hz, 1H), 2.97 - 2.84 (m, 1H), 2.66 (s, 2H), 2.61 - 2.53 (m, 1H), 2.42 (ddd, *J* = 17.5, 13.4, 4.5 Hz, 1H), 2.01 - 1.89 (m, 6H), 1.80 - 1.67 (m, 5H), 1.60 (d, *J* = 12.7 Hz, 2H). UPLC - MS (ESI) calculated for C₂₆H₂₉N₅O₄ [M + H]+: 476.22, found:476.45.

### EXAMPLE 88: 3-(4-((1-(1-((1S, 3S)-adamantan-1-yl) ethyl)-1H-1, 2, 3-triazol-4-yl) methoxy)-1-isoindolin-2-yl) piperidin-2, 6-dione (88)

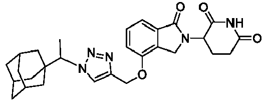

Azide compound was prepared as synthesis method 6 of azides, the compound was prepared as synthesis route 1, yield 46%. ¹H NMR (400 MHz, DMSO) δ 10.97 (s, 1H), 8.21 (s, 1H), 7.52 - 7.46 (m, 1H), 7.43 (d, *J* = 7.8 Hz, 1H), 7.33 (d, *J* = 7.1 Hz, 1H), 5.30 (s, 2H), 5.10 (dd, *J* = 13.3, 5.1 Hz, 1H), 4.39 - 4.27 (m, 2H), 4.20 (d, *J* = 17.4 Hz, 1H), 2.96 - 2.83 (m, 1H), 2.61 - 2.53 (m, 1H), 2.42 (ddd, *J* = 17.6, 13.6, 4.5 Hz, 1H), 2.01 - 1.86 (m, 4H), 1.62 (d, *J* = 11.9 Hz, 3H), 1.51 (d, *J* = 9.5 Hz, 6H), 1.43 (d, *J* = 7.1 Hz, 3H), 1.27 (d, *J* = 11.8 Hz, 3H). UPLC - MS (ESI) calculated for C₂₈H₃₃N₅O₄ [M + H]+: 504.25, found:504.43.

### EXAMPLE 89: 3-(4-((1-((1R, 3R, 5S, 7R)-3, 5-dimethyladamantin-1-yl)-1H-1, 2, 3-triazol-4-yl) methoxy)-1-isoindolin-2-yl) piperidin-2, 6-dione (89)

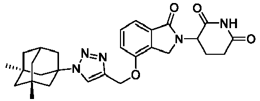

Azide compound was prepared as synthesis method 6 of azides, the compound was prepared as synthesis route 1, yield 55%. ¹H NMR (400 MHz, DMSO) δ 10.97 (s, 1H), 8.37 (s, 1H), 7.54 - 7.48 (m, 1H), 7.46 (d, *J* = 7.7 Hz, 1H), 7.34 (d, *J* = 7.2 Hz, 1H), 5.27 (s, 2H), 5.10 (dd, *J* = 13.3, 5.0 Hz, 1H), 4.35 (d, *J* = 17.5 Hz, 1H), 4.19 (d, *J* = 17.5 Hz, 1H), 2.97 - 2.83 (m, 1H), 2.64 - 2.55 (m, 1H), 2.42 (ddd, *J* = 26.4, 13.5, 4.6 Hz, 1H), 2.028-2.22(m, 1H), 2.05 - 1.92 (m, 3H), 1.83 (q, *J* = 11.8 Hz, 4H), 1.46 (d, *J* = 12.1 Hz, 2H), 1.37 (d, *J* = 12.3 Hz, 2H), 1.30 - 1.18 (m, 2H), 0.90 (s, 6H). UPLC - MS (ESI) calculated for C₂₈H₃₃N₅0₄ [M + H]+: 504.25, found:504.44.

### EXAMPLE 90: 3-(1-oxo-4-((2-(3-(trifluoromethoxy) phenyl) oxazol-5-yl) methyl) amino) isoindolin-2-yl) piperidine-2, 6-dione (90)

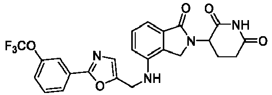

The preparation method referred to Example 82, yield 58%. ¹H NMR (400 MHz, DMSO) δ 11.01 (s, 1H), 7.95 (d, *J* = 7.9 Hz, 1H), 7.80 (s, 1H), 7.68 (t, *J* = 8.0 Hz, 1H), 7.53 (d, *J* = 8.4 Hz, 1H), 7.34 - 7.27 (m, 2H), 7.00 (d, *J* = 7.3 Hz, 1H), 6.96 (d, *J* = 8.0 Hz, 1H), 6.32 (t, *J* = 5.8 Hz, 1H), 5.11 (dd, *J* = 13.2, 5.1 Hz, 1H), 4.55 (d, *J* = 5.8 Hz, 2H), 4.29 (d, *J* = 17.2 Hz, 1H), 4.17 (d, *J* = 17.2 Hz, 1H), 2.99 - 2.84 (m, 1H), 2.65 - 2.57 (m, 1H), 2.30 (qd, *J* = 13.2, 4.3 Hz, 1H), 2.08-2.01 (m, 1H). UPLC - MS (ESI) calculated for C₂₄H₁₉F₃N₄O₅ [M + H]+: 501.13, found:501.29.

### EXAMPLE 91: 3-(4-((2-(3, 4-dichlorophenyl) oxazol-5-yl) methoxy)-1-oxoisoindol-2-yl) piperidine-2, 6-dione

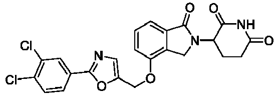

Step 1: a solution of LHMDS (1mol/L, 7.44 ml, 7.44 mmol) in tetrahydrofuran was added to a solution of ethyl oxazole-5-carboxylate (1g, 7.09 mmol) in tetrahydrofuran (25 mL) dropwise at -78°C. After 1h, a solution of diiodoethane (2.31 g, 8.184 mmol) in tetrahydrofuran (10 ml) was added dropwise, reacted at the same temperature for 1h, warmed to room temperature for reaction, monitored by TLC, after the reaction was completed, 100ml of cold ether and saturated sodium thiosulfate were added, extracted and separated, washed the organic layer once with saturated sodium chloride, spin-dried, and column chromatography. Ethyl 2-iodinoxole-5-carboxylate was obtained (white solid, 1.5 g, yield 50%). ¹H NMR (400 MHz, CDCl3) δ 7.65 (s, 1H), 4.39 (q, *J* = 7.1 Hz, 2H), 1.38 (t, *J* = 7.1 Hz, 3H).

Step 2: Ethyl 2-iodooxazol-5-carboxylate (215mg, 0.81 mmol), 3, 4-dichlorophenylboric acid (200 mg, 1.05 mmol), potassium carbonate (336 mg, 2.43mmol), Pd( PPh3)4 (92 mg, 0.08mmol) were added to a 100 ml two-necked flask, dioxane (5 mL) and water (1 mL) were added, refluxed overnight under N2 protection, diluted with water, extracted with ethyl acetate (EA), and the water layer was extracted once with EA, combined the organic layers, washed with saturated NaCl, dried, spin-dried, column chromatography. The product of ethyl 2-(3, 4-dichlorophenyl) oxazol-5-carboxylate(125mg) was obtained;

Step 3: tetrahydroaluminum lithium was added to a solution of ethyl 2-(3, 4-dichlorophenyl) oxazol-5-carboxylate (125mg, 0.439 mmol) in THF (3 mL) at 0 °C, raised to room temperature and reacted for 0.5 h. After the reaction was completed, ethyl acetate was added to quench, and spun dried under reduced pressure. 83mg of (2-(3, 4-dichlorophenyl) oxazol-5-yl) methanol was obtained by silica gel column chromatography, yield 78%;

Step 4: the preparation method was the same as the synthesis route 2 and Example 40, 30 mg of white solid was obtained, yield 47%. ¹H NMR (400 MHz, DMSO) δ 10.97 (s, 1H), 8.15 (d, *J* = 2.0 Hz, 1H), 7.95 (dd, *J* = 8.4, 2.0 Hz, 1H), 7.83 (d, *J* = 8.4 Hz, 1H), 7.61 ― 7.48 (m, 3H), 7.38 (d, *J* = 7.0 Hz, 1H), 5.43 (s, 2H), 5.10 (dd, *J* = 13.3, 5.1 Hz, 1H), 4.40 (d, *J* = 17.6 Hz, 1H), 4.23 (d, *J* = 17.6 Hz, 1H), 2.96 ― 2.85 (m, 1H), 2.61 - 2.54 (m, 1H), 2.45 - 2.34 (m, 1H), 2.01 - 1.92 (m, 1H). UPLC - MS (ESI) calculated for C₂₃H₁₇Cl₂N₃O₅[M + H]+: 486.05, found:486.24.

### EXAMPLE 92: 3-(4-((1-(4-cyclopropoxy-2-fluorophenyl)-1H-1, 2, 3-triazol-4-yl) methoxy)-1-oxoisoindol-2-yl) piperidine-2, 6-dione

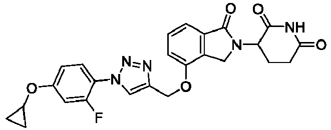

Step 1: cyclopropanol (500mg, 8.61 mmmol) was dissolved in 30ml of a dry DMF solution and cooled to 0 °C. 60% sodium hydride was added, and reacted at the same temperature for 30 minutes, 3, 4-difluoronitrobenzen was added, raised to room temperature and reacted overnight, TLC was used to monitor that the reaction was completed, water was added under ice bath conditions to quench, extracted with ethyl acetate, the organic layer was washed with saturated sodium chloride solution, dried over anhydrous sodium sulfate, concentrated under reduced pressure, and 1-cyclopropoxy-2-fluoro-4-nitrobenzene (670mg) was obtained by silica gel column chromatography with a yield of 39.5%.

Step 2: 1-cyclopropoxy-2-fluoro-4-nitrobenzene (670mg, 3.40 mmol) was dissolved in 20mL of methanol, 67mg of palladium carbon was added, the reaction system was reacted overnight under the condition of atmospheric hydrogen, after the reaction was completed, filtrated, concentrated under reduced pressure to obtain 4-cyclopropoxy-3-fluoroaniline (530mg).

Step 3: The preparation method for synthesizing 4-azido-1-cyclopropoxy-2-fluorobenzene was the same as that of synthesis method 1 of azides.

Step 4: 4-azido-1-cyclopropoxy-2-fluorobenzene and intermediate 6 were used as raw materials, the preparation method was the same as that of synthetic route 1 and Example 1, and 3-(4-((1-(4-(cyclopropoxy-2-fluorophenyl))-1H-1, 2, 3-triazol-4-yl) methoxy)-1-indole oxyisocyanate-2-yl) phospholipid-2, 6-dione was obtained; ¹H NMR (400 MHz, DMSO) δ 10.96 (s, 1H), 8.91 (s, 1H), 7.86 (dd, *J* = 11.9, 2.6 Hz, 1H), 7.75 (d, *J* = 8.9 Hz, 1H), 7.61 (t, *J* = 8.9 Hz, 1H), 7.56 ―7.45 (m, 2H), 7.36 (d, *J* = 7.1 Hz, 1H), 5.39 (s, 2H), 5.11 (dd, *J* = 13.3, 5.1 Hz, 1H), 4.38 (d, *J* = 17.5 Hz, 1H), 4.23 (d, *J* = 17.5 Hz, 1H), 4.09 - 4.01 (m, 1H), 2.96 - 2.84 (m, 1H), 2.62 - 2.53 (m, 1H), 2.45 - 2.35 (m, 1H), 2.02 - 1.93 (m, 1H), 0.89 - 0.71 (m, 4H). UPLC-MS (ESI) calculated for C₂₅H₂₂FN₅O₅ [M + H]⁺: 492.16, found 492.32.

### EXAMPLE 93: 3-(1-oxo-4-((((5-(4-(trifluoromethoxy) phenyl) oxazol-2-yl) methyl) amino) isoindol-2-yl) piperidine-2, 6-dione

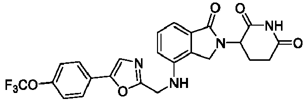

Step 1: 4-trifluoromethoxybenzaldehyde (800mg, 4.21 mmol) was dissolved in 20mL of methanol, 4-methylbenzenesulfonyl methyl isonitrile (904mg, 4.63 mmol) was added under stirring conditions and heated to reflux for 1h. After the reaction was completed, concentrated under reduced pressure to remove the solvent, saturated sodium bicarbonate aqueous solution was added to the residue, extracted with dichloromethane, the organic layer was washed with water and saturated sodium chloride successively, dried, filtered, the solvent was removed under reduced pressure, and the residue was subjected to silica gel column chromatography to obtain 887mg of 4-trifluoromethoxyphenyl oxazole as a yellow solid with a yield of 82%; ¹H NMR (400 MHz, CDCl₃) δ 7.93 (s, 1H), 7.69 (d, *J* = 8.9 Hz, 2H), 7.36 (s, 1H), 7.28 (d, *J* = 8.2 Hz, 2H).

Step 2: 4-trifluoromethoxyphenyl oxazole (879mg, 3.84mmol) was dissolved in 30ml of dry THF under the protection of nitrogen, and the reaction solution was cooled to -78°C, n-butyllithium (2.5 mol/L, 1.69 mL, 4.22mmol) was added dropwise. The reaction was continued for 30min, DMF (325ul, 4.22mmol) was added to the reaction solution, and the reaction solution was continued to react for 1h at -78°C, then raised to room temperature and reacted for 2h. After the reaction was completed, the reaction solution was adjusted to pH5 with 1N HCl, extracted with ethyl acetate, the organic phase was washed with saturated sodium chloride, dried over anhydrous sodium sulfate, filtered, and concentrated under reduced pressure to obtain the product (500mg).

Step 3: The 5-(4-(trifluoromethoxy) phenyl) oxazol-2-carboxaldehyde obtained in the previous step was used as a raw material, and the preparation method was the same as that of reductive amination conditions in Example 82, and 3-(1-oxo-4-((((5-(4-(trifluoromethoxy) phenyl) oxazol-2-yl) methyl) amino) isoindol-2-yl) piperidine-2, 6-dione (12mg) was obtained. ¹H NMR (400 MHz, DMSO) δ 11.02 (s, 1H), 7.81 ― 7.72 (m, 2H), 7.68 (s, 1H), 7.47 (d, *J* = 8.2 Hz, 2H), 7.30 (t, *J* = 7.7 Hz, 1H), 6.96 (dd, *J* = 32.8, 7.6 Hz, 2H), 6.49 (t, *J* = 6.1 Hz, 1H), 5.12 (dd, *J* = 13.3, 5.1 Hz, 1H), 4.59 (d, *J* = 6.1 Hz, 2H), 4.30 (d, *J* = 17.2 Hz, 1H), 4.19 (d, *J* = 17.2 Hz, 1H), 2.99 - 2.86 (m, 1H), 2.62 (d, *J* = 17.0 Hz, 1H), 2.32 (qd, *J* = 13.2, 4.3 Hz, 1H), 2.04 (dd, *J* = 9.0, 3.6 Hz, 1H). UPLC-MS (ESI) calculated for C₂₄H₁₉F₃N₄O₅: 501.13, found 501.28.

### EXAMPLE 94: 3-(1-oxo-4-((((2-(2-(trifluoromethoxy) phenyl) oxazol-2-yl) methyl) amino) isoindolin-2-yl) piperidine-2, 6-dione

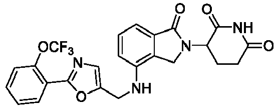

The synthetic route and preparation method were the same as those of Example 82, and 3-(1-oxo-4-((((2-(2-(trifluoromethoxy) phenyl) oxazol-5-yl) methyl) amino) isoindolin-2-yl) piperidine-2, 6-dione was obtained. ¹H NMR (400 MHz, DMSO) δ 11.02 (s, 1H), 7.81 ― 7.72 (m, 2H), 7.68 (s, 1H), 7.47 (d, *J* = 8.2 Hz, 2H), 7.30 (t, *J* = 7.7 Hz, 1H), 6.96 (dd, *J* = 32.8, 7.6 Hz, 2H), 6.49 (t, *J* = 6.1 Hz, 1H), 5.12 (dd, *J* = 13.3, 5.1 Hz, 1H), 4.59 (d, *J* = 6.1 Hz, 2H), 4.30 (d, *J* = 17.2 Hz, 1H), 4.19 (d, *J* = 17.2 Hz, 1H), 2.99 - 2.86 (m, 1H), 2.62 (d, *J* = 17.0 Hz, 1H), 2.32 (qd, *J* = 13.2, 4.3 Hz, 1H), 2.04 (dd, *J* = 9.0, 3.6 Hz, 1H). UPLC-MS (ESI) calculated for C₂₄H₁₉F₃N₄O₅: 501.13, found 501.28.

### II. Test Examples

The present invention also tested the activity of the multi-substituted isoindoline compounds on three types of hematological tumor cell lines. Representative cell lines are: multiple myeloma cell line (MM.1S), mantle cell lymphoma cell line (Mino), acute myeloid leukemia cell line (MV-4-11). The cell proliferation inhibitory activity of these three representative cell lines was tested. The experimental materials required for pharmacological experiments were commercially purchased unless otherwise specified.

### 1. The effect of the compound on the proliferation of MM.1S cells

MM.1S cells were cultured with 1640 plus 10% fetal bovine serum and collected. The cell concentration was diluted according to the action time of 7 days, and 180ul cell suspension was added to each well of the 96-well cell plate to make the cell count to be 20,000. 20ul of DMSO with a final concentration of 0.2% was added to the control cell wells. The compound was diluted 5-fold with the lOmM stock solution, and 20ul was also added to the compound cell wells (the final concentration of DMSO is 0.2%). The cells were placed in a 37°C, 5% CO₂ incubator and incubated for 7 days. After preparing the reaction solution according to the MTS kit (Promega, G5430), 20µL was added to each well, incubated in a 37°C, 5% CO₂ incubator for 3-4 h. Read the 490nm absorbance value with a microtiter plate, and used the 690nm absorbance value as the background value and OD490-OD690 as the final initial data. The formula for calculating the inhibition rate of the compound is: inhibition rate = (OD _{DMSO}-OD _{compound})/(OD _{DMSO}-O _{blank}) × 100%. The compound's proliferation inhibition IC50 was fitted by Graph Pad Prism 5.0. The experiment was repeated three times, and three parallel experiments were used to calculate the average and standard deviation each time. The cell viability test results were shown in Table 1: A means cell viability IC₅₀ <150 nM, B means cell viability 150 nM ≤ IC₅₀ ≤ 20 µM, C means cell viability IC₅₀> 20 µM.

**Table 1. Inhibitory activity of compound on proliferation of MM.1S cells**

| **Serial number** | **Cell inhibitory activity (IC₅₀)** | **Serial number** | **Cell inhibitory activity (IC₅₀)** |
|---|---|---|---|
| **1** | A | **2** | B |
| **3** | B | **4** | A |
| **5** | A | **6** | A |
| **7** | A | **8** | A |
| **9** | A | **10** | A |
| **11** | A | **12** | A |
| **13** | B | **14** | A |
| **15** | A | **16** | A |
| **17** | A | **18** | A |
| **19** | A | **20** | A |
| **21** | A | **22** | A |
| **23** | A | **24** | A |
| **25** | A | **26** | A |
| **27** | A | **28** | A |
| **29** | A | **30** | A |
| **31** | A | **32** | A |
| **33** | A | **34** | B |
| **35** | B | **36** | B |
| **37** | B | **38** | B |
| **39** | A | **40** | A |
| **41** | A | **42** | A |
| **43** | A | **44** | A |
| **45** | A | **46** | A |
| **47** | A | **48** | B |
| **49** | A | **50** | A |
| **51** | A | **52** | A |
| **53** | A | **54** | B |
| **55** | A | **56** | A |
| **57** | A | **58** | A |
| **59** | A | **60** | C |
| **61** | A | **62** | A |
| **63** | C | **64** | B |
| **65** | B | **66** | B |
| **67** | A | **68** | A |
| **69** | B | **70** | A |
| **71** | B | **72** | A |
| **73** | A | **74** | A |
| **75** | A | **76** | A |
| **77** | A | **78** | A |
| **79** | A | **80** | A |
| **81** | A | **82** | A |
| **83** | A | **84** | A |
| **85** | A | **86** | C |
| **87** | A | **88** | A |
| **89** | C | **90** | A |
| **91** | A | **92** | B |
| **93** | A | **94** | B |
| **Lenalidomide** | A | **Pomalidomide** | A |
| **CC-122** | A | **CC-220** | A |

Based on the cell growth inhibitory activity test results of the above compounds, the compounds of some embodiments of the present invention have good inhibitory activity on the growth of multiple myeloma MM.1S cells, and the activities of some compounds are equivalent to or better than the positive compounds. On the other hand, the development of these structurally diverse compounds provides an alternative source for obtaining more active drug molecules and molecules with better pharmaceutical properties. Therefore, the compounds of the present invention can be used to prevent and treat diseases related to the regulation of CRBN (CRL4 ^{CRBN} E3 ubiquitin ligase) activity, such as multiple myeloma or including but not limited to other potential tumor diseases, pain, nervous system diseases and immune system diseases.

### 2. The effect of the compound on the proliferation of Mino cells

Mino cells were cultured with 1640 plus 10% fetal bovine serum and collected. The cell concentration was diluted according to the action time of 3 days, and 90ul cell suspension was added to each well of the 96-well cell plate to make the cell count to be 8000. 10ul of DMSO with a final concentration of 0.2% was added to the control cell wells. The compound was diluted 5-fold with the lOmM stock solution, and 10ul was also added to the compound cell wells (the final concentration of DMSO was 0.2%). The cells were placed in a 37°C, 5% CO₂ incubator and incubated for 3 days. After preparing the reaction solution according to the MTS kit (Promega, G5430), 20µL was added to each well, incubated in a 37°C, 5% CO₂ incubator for 3-4 h. Read the 490nm absorbance value with a microtiter plate, and used the 690nm absorbance value as the background value and OD490-OD690 as the final initial data. The formula for calculating the inhibition rate of the compound was: inhibition rate = (OD _{DMSO}-OD compound)/(OD _{DMSO}-O _{blank}) × 100%. The compound's proliferation inhibition IC50 was fitted by Graph Pad Prism 5.0. The experiment was repeated three times, and three parallel experiments were used to calculate the average and standard deviation each time. The cell viability test results were shown in Table 2: A means cell viability IC₅₀ <150 nM, B means cell viability 150 nM ≤ IC₅₀ ≤ 20 µM, C means cell viability IC₅₀> 20 µM.

**Table 2. Inhibitory activity of compound on proliferation of Mino cells**

| **Compound number** | **Inhibitory activity (IC₅₀)** | **Compound number** | **Inhibitory activity (IC₅₀)** |
|---|---|---|---|
| **17** | A | **41** | B |
| **18** | A | **42** | A |
| **19** | B | **43** | A |
| **20** | A | **44** | A |
| **23** | A | **45** | A |
| **24** | A | **70** | B |
| **25** | A | **71** | B |
| **26** | A | **90** | A |
| **28** | A | **93** | B |
| **29** | B | **Lenalidomide** | C |
| **30** | A | **Pomalidomide** | B |
| **39** | B | **CC-122** | B |
| **40** | A | **CC-220** | A |

Based on the cell growth inhibitory activity test results of the above compounds, the compounds of some embodiments of the present invention have good inhibitory activity on the growth of mantle cell lymphoma Mino cells, and the activities of some compounds are equivalent to or better than the positive compounds. On the other hand, the development of these structurally diverse compounds provides an alternative source for obtaining more active drug molecules and molecules with better pharmaceutical properties. Therefore, the compound of the present invention broadens the scope of application of dosamine drugs in the treatment of blood tumor diseases, and can be used to expand to other indications of hematological tumors, such as an active molecule of mantle cell lymphoma disease, and used as a medicine or diagnostic reagent for the prevention or treatment of such diseases.

Therefore, the compound of the present invention can be used as a powerful new type of CRBN modulator for the prevention and treatment of diseases related to the regulation of CRBN (CRL4CRBNE3 ubiquitin ligase) activity, such as multiple myeloma, mantle cell lymphoma or including but not limited to other potential tumor diseases, pain, nervous system diseases and immune system diseases.

### 3. The effect of the compound on proliferation of MV-4-11 cells

MV-4-11 cells were cultured with IMDM plus 10% fetal bovine serum and collected. The cell concentration was diluted according to the action time of 7 days, and 180ul of cell suspension was added to each well of a 96-well cell plate to make the cell count to be 2000. 20ul of DMSO with a final concentration of 0.2% was added to the control cell wells. The compound was diluted 5-fold with the lOmM stock solution, and 20ul was also added to the compound cell wells (the final concentration of DMSO was 0.2%). The cells were placed in a 37°C, 5% CO₂ incubator and incubated for 7 days. After preparing the reaction solution according to the MTS kit (Promega, G5430), 20µL was added to each well, incubated in a 37°C, 5% CO₂ incubator for 3-4 h. Read the 490nm absorbance value with a microtiter plate, and used the 690nm absorbance value as the background value and OD490-OD690 as the final initial data. The formula for calculating the inhibition rate of the compound was: inhibition rate = (OD _{DMSO}-OD compound)/(OD _{DMSO}-O _{blank}) × 100%. The compound's proliferation inhibition IC₅₀ was fitted by Graph Pad Prism 5.0. The experiment was repeated three times, and three parallel experiments were used to calculate the average and standard deviation each time. The cell viability test results were shown in Table 3: A means cell viability IC₅₀ <1 µM, B means cell viability 1 µM ≤ IC₅₀ ≤ 20 µM, C means cell viability IC₅₀> 20 µM.

**Table 3. Inhibitory activity of compound on proliferation of MV-4-11 cells**

| **Compound** | **Inhibitory activity (IC₅₀)** | **Compound** | **Inhibitory activity (IC₅₀)** |
|---|---|---|---|
| **18** | A | **75** | A |
| **20** | B | **76** | A |
| **26** | A | **77** | A |
| **28** | A | **81** | A |
| **55** | B | **CC-122** | C |
| **70** | B | **Lenalidomide** | C |
| **71** | B | **Pomalidomide** | C |
| **74** | A | **CC-220** | C |

Based on the test results of the cell growth inhibitory activity of the compounds of the above partial examples on the acute myeloid leukemia cell line (MV-4-11), it was found that some of the compounds of the examples of the present invention had very good inhibitory activity against the acute leukemia cell MV-4-11 cells. The IC₅₀ of multiple compounds was at the nanomolar level, and the best activity IC₅₀ of the tested compounds in the Table can reach < 10 nM. However, the cytostatic activity (IC50) of the positive compounds (either lenalidomide or pomalidomide), which are already on the market, and those compounds (CC-122 or CC-220) which are currently in clinical practice on acute leukemia cell MV-4-11 cells is greater than 20µM. From the test results in the above table, it is found that the inhibitory activity of some compounds of the present invention on the proliferation of acute leukemia cells MV-4-11 cells is stronger than that of the related positive compounds, and the best compound has an activity of more than 2000 times that of the positive compound.

Therefore, the compound of the present invention broadens the scope of application of dosamine drugs in the treatment of hematological tumors diseases, and can be used to expand to other indications of hematological tumors, such as as an inhibitor of acute leukemia, and as a medicine for the treatment of such diseases. Therefore, the compound of the present invention can be used as a powerful new type of CRBN modulator for the prevention and treatment of diseases related to the regulation of CRBN (CRL4CRBNE3 ubiquitin ligase) activity, such as multiple myeloma, mantle cell lymphoma, acute leukemia or including but not limited to other potential tumor diseases, pain, nervous system diseases and immune system diseases.

### 4. Activity test of the compound in other cell lines

The human triple negative breast cancer cells MDA-MB-468 and MDA-MB-231 used in this experiment were purchased from the Shanghai Cell Bank, in which L-15 medium added with 10% fetal bovine serum (FBS) and 1% double antibody was used. At 37°C, MDA-MB-468 and MDA-MB-231 cells were cultured in an incubator without CO2. Colorectal cancer cells HCT-116 were cultured in McCOY's 5A medium with 1% double antibody and 10% fetal bovine serum (FBS); prostate cancer cells DU145 were cultured in MEM medium with 1% double antibody and 10% fetal bovine serum (FBS); prostate cancer cells PC-3 were cultured in F-12 K medium with 1% double antibody and 10% fetal bovine serum (FBS); grew at 37°C, 5% CO2.

In the cell activity test experiment, 90µL cell suspension with appropriate concentration was added to a 96-well cell culture plate according to the cell growth, each compound to be tested was gradient diluted with the corresponding medium, 10µL diluted compound was added to 90µL cells, and then incubated at 37°C for 4 days. Cell proliferation was analyzed by WST-8, which could be reduced by lactate dehydrogenase in the cells to a yellow formazan. 10µL of WST-8 reagent (DOJINDO) was added to the cells and reacted for more than 1 hour at 37°C, DMSO-treated cells were used as positive control. The absorption value of 490nm was read by enzyme-labeled plate, and the absorption value of 690nm was taken as the background value, and OD490-OD690 was taken as the final original data, and the data was processed by GraphPad Prism6 software. The formula for calculating the inhibition rate of the compound was: inhibition rate = (OD _{DMSO}-OD _{compound})/(OD _{DMSO}-O _{blank}) × 100%. The compound's proliferation inhibition IC₅₀ was fitted by Graph Pad Prism 5.0. The experiment was repeated three times, and three parallel experiments were used to calculate the average and standard deviation each time. The cell viability test results were shown in Table 4: A means cell viability IC₅₀ <1 µM, B means cell viability 1µM ≤ IC₅₀ ≤ 20 µM, C means cell viability IC₅₀> 20 µM, NT means not tested.

**Table 4. Inhibitory activity of compound on proliferation of other tumor cells**

| **Compound** | **DU145** | **PC-3** | **MDA-MB-231** | **HCT-116** |
|---|---|---|---|---|
| **Lenalidomide** | C | C | c | c |
| **45** | B | B | C | B |
| **30** | B | B | B | B |

From the above table, we could find that the compounds (45, 30) of the present invention also have certain activities in human prostate cancer cell lines (PC-3, DU145), triple negative breast cancer (MDA-MB-231) and human colon cancer cell lines (HCT116). Therefore, the compounds of the present invention can be used for the preparation of drugs for the prevention and treatment other potential tumor diseases, pain, nervous system diseases and immune system diseases.

### 5. TNF-a activity inhibition experiment and method:

All operations of this experiment were carried out according to the conventional experimental process of this kind of experiment at present. Peripheral blood from healthy volunteers was collected by routine standard procedure and cultured in 1640 medium (+10% FBS) to obtain PBMC. After recovery, PBMC was centrifuged and resuspended in serum-free medium. After counting, adjusted the density to be 6.25X10^5/ml; then inoculated 160ul to a 96-well plate, 1X10^5/well; 20ul 10X compound and DMSO were added, and incubated for 1 hour in an incubator; then 20ul 10XLPS was added resulting the final concentration of 1ug/ml and incubated in an incubator for 72 hours. Centrifuged the cell plate at 1500rpm and aspirated 50ul supernatant according to the ELISA operation. After operating according to the kit, a microplate reader was used to read at 450nm. The concentration of compound was 10nM, and DMSO was added as control group. Materials used in this experiment: 96 well plate (Corning, # 3599), ELISA kit (Thermo), LPS (Sigma, USA). The test results were shown in Table 5.

**Table 5. TNF-a activity inhibition assay of compound**

| **Compound** | **Inhibition rate of TNF-a (%)** |
|---|---|
| **Lenalidomide** | <50 |
| **CCC-135 (compound 17)** | > 50 |

From the test results in the above table, it was found that some compounds of the examples of the present invention could be used to inhibit or regulate the activity of TNF-α. Therefore, the compound represented by formula (I) provided in the present invention could be used for manufacture of a medicament for the treatment or prevention of diseases, disorders or conditions that are produced by TNF-α or or abnormal regulated by TNF-α activity.

### 6. Experiments and methods of compound modulating IL-2 expression changes:

All operations of this experiment were carried out according to the conventional experimental process of this kind of experiment at present. Peripheral blood from healthy volunteers was collected by routine standard procedure and cultured in 1640 medium (+10% FBS) to obtain PBMC. After recovery, PBMC was centrifuged and resuspended in serum-free medium. After counting, adjusted the density to be 6.25X10^5/ml; then inoculated 160ul to a 96-well plate, 2 holes each, 1X10^5/well; 20ul 10X compound and DMSO were added, and incubated for 1 hour in an incubator; then 20ul 10X anti-human CD3 was added resulting the final concentration of 10ug/ml and incubated in an incubator for 24 hours. Centrifuged the cell plate at 1500rpm and aspirated 50ul supernatant according to the ELISA operation. After operating according to the kit, a microplate reader was used to read at 450nm. The concentration of compound was 10nM, and DMSO was added as control group. Materials used in this experiment: 96 well plate (Corning, # 3599), ELISA kit (Thermo), LPS (Sigma, USA). The test results were shown in Table 5.

**Table 6. The experimental test of the compound's expression change on IL-2**

| **Compound** | **Increase multiple of IL-2 Expression** |
|---|---|
| **Lenalidomide** | > 3 |
| **CCC-135 (compound 17)** | > 3 |

From the test results in the above table, it was found that some compounds of the examples of the present invention could be used to regulate the expression of IL-2. Therefore, the compound represented by formula (I) provided in the present invention could be used for manufacture of a medicament for the treatment or prevention of diseases, disorders or conditions that are produced by IL-2 or or abnormal regulated by IL-2 activity.

### 7. Experiments and methods of compound modulating IFNγ expression changes:

All operations of this experiment were carried out according to the conventional experimental process of this kind of experiment at present. Peripheral blood from healthy volunteers was collected by routine standard procedure and cultured in 1640 medium (+10% FBS) to obtain PBMC. After recovery, PBMC was centrifuged and resuspended in serum-free medium. After counting, adjusted the density to be 6.25X10^5/ml; then inoculated 160ul to a 96-well plate, 2 holes each, 1X10^5/well; 20ul 10X compound and DMSO were added, and incubated for 1 hour in an incubator; then 20ul 10X anti-human CD3 was added resulting the final concentration of 10ug/ml and incubated in an incubator for 24 hours. Centrifuged the cell plate at 1500rpm and aspirated 50ul supernatant according to the ELISA operation. After operating according to the kit, a microplate reader was used to read at 450nm. The concentration of compound was 10nM, and DMSO was added as control group. Materials used in this experiment: 96 well plate (Corning, # 3599), ELISA kit (Thermo), LPS (Sigma, USA). It was found from the test results of the following table that the compounds of the examples of the present invention could be used to regulate the expression of IFNγ.

**Table 7. The experimental test of the compound's expression change on IFNγ**

| **Compound** | **Increase multiple of IFNγ expression** |
|---|---|
| **Lenalidomide** | > 1.5 |
| **CCC-135 (compound 17)** | > 1.5 |

It was found from the test results of the above table that the compounds of the examples of the present invention could be used to regulate the expression of IFNγ. Therefore, the compound represented by formula (I) provided in the present invention could be used for manufacture of a medicament for the treatment or prevention of diseases, disorders or conditions that are produced by IFNγ or abnormal regulated by IFNγ activity.

### 8. Verification experiment of interaction between compound and CRBN

Studies have shown that lenalidomide immunomodulators in hematological tumor cell lines regulate the activity of CRBN-ubiquitin ligase complex by binding to CRBN, selectively induce ubiquitination and degradation of transcription factors IKZF1 and IKZF3, thereby achieving the role of treating malignant hematological tumors (Science, 2014, 343, 301; Science, 2014, 343, 305; Nature, 2015, 523, 183.). By using high-efficiency affinity magnetic nanoparticles "FG beads", thalidomide analogues were pre-attached to the magnetic beads, and the thalidomide FG beads could catch CRBN protein (Leukemia, 2012, 26, 2326; Science, 2010, 327, 1345). In this experiment, NP-400 cell lysate was used to lyse the blood tumor Mino cells, and centrifuged to obtain a clear cell lysate and divided it into three samples evenly. Thalidomide FG beads were added to the three samples, and the Thalidomide FG beads and the cell lysate were combined and incubated at 4°C for 6 hours. After the incubation was completed, separated the magnetic beads with a magnetic stand, resuspended the separation with NP-400 lysate, repeated 3 times to obtain magnetic beads removed excess cell lysate. The three groups of magnetic bead samples were respectively incubated with NP-400 lysate containing DMSO (control group), NP-400 lysate containing 500 mM of Example Compound 17, and NP-400 lysate containing 1 mM lenalidomide at 25°C for 15 minutes. After eluting twice, the eluates were combined to obtain the eluate. The eluate was denatured by heating with SDS Loading Buffer, and the amount of CRBN in each sample was detected by Western blotting with CRBN antibody (Proteintech). The experimental results were shown in Fig 1.

From the above experimental results, it can be found that example compound 17 can elute CRBN from the magnetic beads bound to thalidomide. Compared with DMSO group and positive compound lenalidomide group. Its principle of action is similar to that of lenalidomide, but DMSO alone cannot compete with the binding of CRBN and thalidomide. Therefore, the example compound and CRBN have a good function. Therefore, the compounds of the examples in the present invention can be used as CRL4CRBNE3 ubiquitin ligase modulators, selectively induce substrate proteins to undergo ubiquitination and degradation by regulating the activity of the CRBN-ubiquitin ligase complex, and can be used for the manufacture of a medicament or diagnostic reagent for the prevention or treatment of diseases related to CRL4CRBNE3 ubiquitin ligase.

In summary, the present invention provides a class of substituted isoindoline compounds with novel structures, in which some representative compounds exhibit very strong proliferation inhibitory activity on the tested haematological tumor cells. In addition, some of the representative compounds provided by the present invention also have certain activity in other tumor cell lines. Therefore, the compound with novel structure of the present invention can be used for the manufacture of a medicament or diagnostic reagent for the prevention or treatment of diseases related to CRL4CRBNE3 ubiquitin ligase which can further improve the therapeutic effect of tumor treatment and expand the clinical needs of new indications of domide drugs; it is expected to overcome the application limitations of existing domide drugs. This feature can not only effectively make up for the shortcomings of existing domide drugs, but also expand their indications to new areas. Therefore, it has very strong research potential and application prospects.

## Claims

1. A compound represented by formula (I), a tautomer, enantiomer, diastereomer, racemate, metabolite, metabolic precursor, isotopic compound, pharmaceutically acceptable salt, ester, prodrug or hydrate thereof: 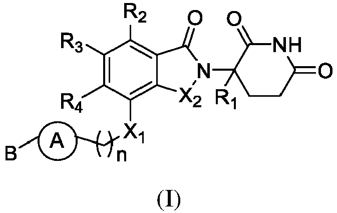 wherein X₁ is -CH₂-, -NH- or -O-;
X₂ is -CH₂- or -CO-;
R₁ is hydrogen, deuterium, fluorine or linear or branched C₁-C₆ hydrocarbyl; R₂ and R₄ are each independently selected from hydrogen or deuterium;
R₃ is selected from hydrogen, deuterium or halogen;
n is 1, 2 or 3;
Ⓐ is selected from the following groups: 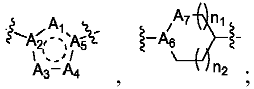
A₁ is elected from C, N, O, S or NR₅, wherein R₅ is selected from C₁-C₆ alkyl, C₁-C₆ haloalkyl or C₃-C₆ cycloalkyl;
A₃ or A₄ is each independently selected from C, N, O or S;
when A₁, A₃ or A4 is selected from C, A₁, A₃ or A₄ each can be independently substituted by methyl or ethyl;
A₂ or A₅ is each independently selected from C or N;
A₇ is selected from C, N, O or S;
A₆ is C or N, when A₆ is N, the connection mode between Ⓐ and B is 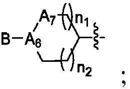
n₁ is 0, 1, 2 or 3;
n₂ is 0, 1, 2 or 3;
B is (6-10 membered aryl)-(CH₂)_{b1}-(CHR₆)_{b2}-, (5-10 membered heteroaryl)-(CH₂)_{b1}-(CHR₆)_{b2}-, (5-14 membered heterocyclyl)-(CH₂)_{b1}-(CHR₆)_{b2}-, (5-16 membered cycloalkyl)-(CH₂)_{b1}-(CHR₆)_{b2}- , the aryl, heteroaryl, heterocyclyl or cycloalkyl is substituted with one or more of the following groups: deuterium, halogen, cyano, nitro, hydroxyl, carboxyl, aminocarbonyl, C₁-C₆ alkyl, C₁-C₆ alkoxyalkyl, C₁-C₆ haloalkyl, C₂-C₁₀ alkenyl, C₂-C₁₀ alkynyl, hydroxyl substituted C₁-C₆ alkyl, C₁-C₆ alkoxy, C₁-C₆ alkylcarbonyl, C₁-C₆ alkylaminocarbonyl, C₁-C₆alkylsulfonyl, C₁-C₆ haloalkoxy, hydroxyl substituted C₁-C₆ alkoxy, alkoxy substituted C₁-C₆ alkoxy, cyano-substituted C₁-C₆ alkoxy, C₃-C₈ cycloalkyl, C₃-C₈ cycloalkyloxy, C₃-C₈heterocyclyl, C₃-C₈ heterocyclyloxy, C₃-C₈heterocyclylmethylene, halogen-substituted or unsubstituted phenyl, halogen-substituted or unsubstituted benzyl, halogen-substituted or unsubstituted phenoxy, C₅-C₆ heteroaryl, -NHC(O)Ra₁, -NHC(O)ORa₂, -NRa₃Ra₄, wherein Ra₁, Ra₂, Ra₃ and Ra₄ are each independently hydrogen, C1-6 alkyl unsubstituted or substituted by halogen, hydroxyl, cyano, or C3-6 cycloalkyl unsubstituted or substituted by halogen, hydroxyl, cyano;
b₁ is 0, 1, 2 or 3;
b₂ is 0 or 1;
R6 is selected from deuterium, C₁-C₆ alkyl, C₁-C₆ haloalkyl, hydroxyl substituted C₁-C₆ alkyl, C₁-C₆ alkoxyalkyl, C₁-C₆ haloalkoxyalkyl, -CH₂NHC(O)Ra₅, -CH₂NRa₆Ra₇, wherein Ra₅, Ra6 and Ra₇ are each independently hydrogen, C1-3 alkyl unsubstituted or substituted by halogen, hydroxyl, or C3-6 cycloalkyl unsubstituted or substituted by halogen, hydroxyl;
when X₁ is -O-, and Ⓐ is selected from 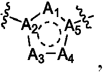 B is not

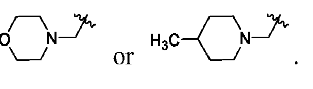

2. The compound, the tautomer, enantiomer, diastereomer, racemate, metabolite, metabolic precursor, isotopic compound, pharmaceutically acceptable salt, ester, prodrug or hydrate thereof of claim 1:
wherein X₁ is -CH₂-, -NH- or -O-;
X₂ is -CH₂- or -CO-;
R₁ is hydrogen, deuterium, fluorine or linear or branched C₁-C₆ hydrocarbyl;
R2 and R₄ are each independently selected from hydrogen or deuterium;
R₃ is selected from hydrogen, deuterium or halogen;
n is 1, 2 or 3;
Ⓐ is selected from 5-membered heteroaromatic ring containing 1-3 heteroatoms selected from N, O or S, 4-6-membered heterocycle containing 1-3 heteroatoms selected from N, O or S, and a 4-6-membered aliphatic ring, wherein the carbon atom on the 5-membered heteroaromatic ring is optionally substituted by methyl or ethyl;
when A₆ is N, the connection mode between Ⓐ and B is 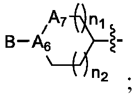
Ⓐ is preferably selected from 5-membered heteroaromatic ring containing one heteroatom selected from N, O or S, Ⓐ is preferably selected from the following groups: 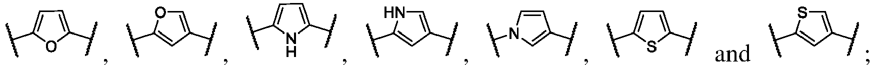
or Ⓐ is 5-membered heteroaromatic ring containing two heteroatoms selected from N, O or S, Ⓐ is preferably selected from the following groups: 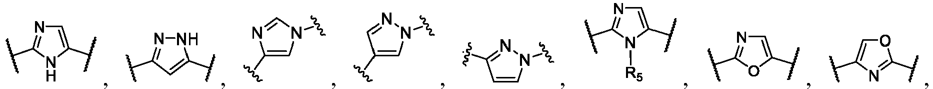 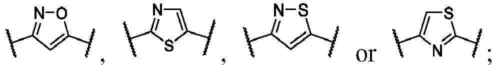
Ⓐ is 5-membered heteroaromatic ring containing three heteroatoms selected from N, O or S, Ⓐ is preferably selected from the following groups: 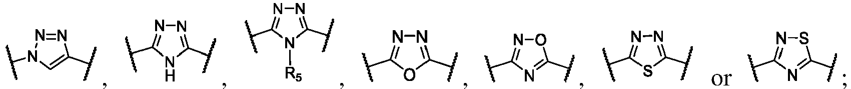
Ⓐ is 4-membered aliphatic ring or heterocycle, Ⓐ is preferably selected from the following groups: 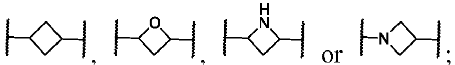
when Ⓐ is 5-membered aliphatic ring or heterocycle, Ⓐ is preferably selected from the following groups: 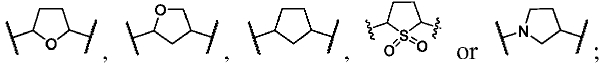
when Ⓐ is 6-membered aliphatic ring or heterocycle, Ⓐ preferably is selected from the following groups: 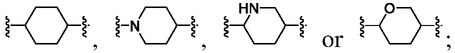
wherein R₅ is selected from C₁-C₆ alkyl, halogen substituted C₁-C₆ alkyl or C₃-C₆ cycloalkyl;
B is (6-10 membered aryl)-(CH₂)_{b1}-(CHR₆)_{b2}-, (5-10 membered heteroaryl)-(CH₂)_{b1}-(CHR₆)_{b2}-, (5-14 membered heterocyclyl)-(CH₂)_{b1}-(CHR₆)_{b2}-, (5-16 membered cycloalkyl)-(CH₂)_{b1}-(CHR₆)_{b2}-, the aryl, heteroaryl, heterocyclyl or cycloalkyl is substituted with one or more groups selected from the group consisting of deuterium, halogen, cyano, nitro, hydroxy, carboxy, aminocarbonyl, C₁-C₆ alkyl, C₂-C₁₀ alkenyl, C₂-C₁₀ alkynyl, C₁-C₆ alkoxyalkyl, C₁-C₆ haloalkyl, hydroxyl substituted C₁-C₆ alkyl, C₁-C₆ alkoxy, C₁-C₆ alkoxycarbonyl, C₁-C₆ alkylaminocarbonyl, C₁-C₆ alkylsulfonyl, C₁-C₆ haloalkoxy, hydroxyl substituted C₁-C₆ alkoxy, cyano substituted C₁-C₆ alkoxy, C₃-C₈ cycloalkyl, C₃-C₈ cycloalkyloxy, C₃-C₈ heterocyclyl, C₃-C₈ heterocyclyloxy, C₃-C₈ heterocyclylmethylene, halogen-substituted or unsubstituted phenyl, halogen-substituted or unsubstituted benzyl, halogen-substituted or unsubstituted phenoxy, C₅-C₆ heteroaryl, -NHC(O)Ra₁, -NHC(O)ORa₂, -NRa₃Ra₄, wherein Ra₁, Ra₂, Ra₃ and Ra₄ are each independently hydrogen, C1-6 alkyl unsubstituted or substituted by halogen, hydroxy, cyano, or C3-6 cycloalkyl unsubstituted or substituted by halogen, hydroxy, cyano;
b₁ is 0, 1, 2 or 3;
b₂ is 0 or 1;
R6 is selected from deuterium, C₁-C₆ alkyl, C₁-C₆ haloalkyl, hydroxyl substituted C₁-C₆ alkyl, C₁-C₆ alkoxyalkyl, C₁-C₆ haloalkoxyalkyl, -CH₂NHC(O)Ra₅, -CH₂NRa₆Ra₇, wherein Ra₅, Ra6 and Ra₇ are each independently hydrogen, C1-3 alkyl unsubstituted or substituted by halogen, hydroxyl, or C3-6 cycloalkyl unsubstituted or substituted by halogen, hydroxyl;
when X₁ is -O-, and Ⓐ is 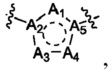 B is not

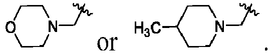

3. The compound, the tautomer, enantiomer, diastereomer, racemate, metabolite, metabolic precursor, isotopic compound, pharmaceutically acceptable salt, ester, prodrug or hydrate thereof of claim 1:
wherein R₃ is halogen;
X₁ is -CH₂, -NH- or -O-;
X₂ is -CH₂- or -CO-;
R₁ is hydrogen, deuterium, fluorine or methyl;
R2 and R₄ are each independently selected from hydrogen or deuterium;
n is 1, 2 or 3;
Ⓐ is 5-membered heteroaromatic ring containing 1-3 heteroatoms selected from N, O or S, 4-6-membered heterocycle containing 1-3 heteroatoms selected from N, O or S, or a 4-6-membered aliphatic ring, wherein the carbon atom on the 5-membered heteroaromatic ring is optionally substituted by methyl or ethyl;
when A₆ is N, the connection mode between Ⓐ and B is 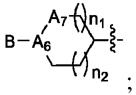
Ⓐ is 5-membered heteroaromatic ring containing one heteroatom selected from N, O or S, preferably Ⓐ is selected from the group consisting of: 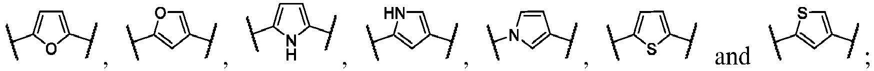
or Ⓐ is 5-membered heteroaromatic ring containing two heteroatoms selected from N, O or S, preferably Ⓐ is selected from the following groups: 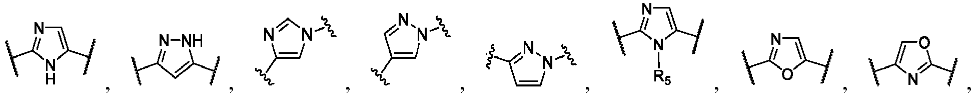 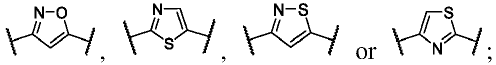
Ⓐ is 5-membered heteroaromatic ring containing three heteroatoms selected from N, O or S, preferably Ⓐ is selected from the following groups: 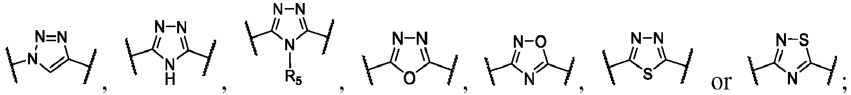
Ⓐ is 4-membered aliphatic ring or heterocycle, preferably Ⓐ is selected from the following groups: 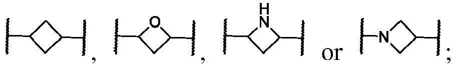
when Ⓐ is 5-membered aliphatic ring or heterocycle, Ⓐ is preferably selected from the following groups: 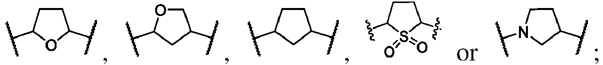
when Ⓐ is 6-membered aliphatic ring or heterocycle, Ⓐ preferably is selected from the following groups: 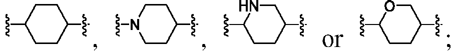
wherein R₅ is selected from C₁-C₆ alkyl, halogen substituted C₁-C₆ alkyl or C₃-C₆ cycloalkyl;
B is (6-10 membered aryl)-(CH₂)_{b1}-(CHR₆)_{b2}-, (5-10 membered heteroaryl)-(CH₂)_{b1}-(CHR₆)_{b2}-, (5-14 membered heterocyclyl)-(CH₂)_{b1}-(CHR₆)_{b2}-, (5-16 membered cycloalkyl)-(CH₂)_{b1}-(CHR₆)_{b2}-, the aryl, heteroaryl, heterocyclyl or cycloalkyl is substituted with one or more groups selected from the group consisting of deuterium, halogen, cyano, nitro, hydroxy, carboxy, aminocarbonyl, C₁-C₆ alkyl, C₂-C₁₀ alkenyl, C₂-C₁₀ alkynyl, C₁-C₆ alkoxyalkyl, C₁-C₆ haloalkyl, hydroxyl substituted C₁-C₆ alkyl, C₁-C₆ alkoxy, C₁-C₆ alkoxycarbonyl, C₁-C₆ alkylaminocarbonyl, C₁-C₆ alkylsulfonyl, C₁-C₆ haloalkoxy, hydroxyl substituted C₁-C₆ alkoxy, cyano substituted C₁-C₆ alkoxy, C₃-C₈ cycloalkyl, C₃-C₈ cycloalkyloxy, C₃-C₈ heterocyclyl, C₃-C₈ heterocyclyloxy, C₃-C₈ heterocyclylmethylene, halogen-substituted or unsubstituted phenyl, halogen-substituted or unsubstituted benzyl, halogen-substituted or unsubstituted phenoxy, C₅-C₆ heteroaryl, -NHC(O)Ra₁, -NHC(O)ORa₂, -NRa3Ra4, wherein Ra₁, Ra2, Ra₃ and Ra₄ are each independently hydrogen, C1-6 alkyl unsubstituted or substituted by halogen, hydroxy, cyano, or C3-6 cycloalkyl unsubstituted or substituted by halogen, hydroxy, cyano;
b₁ is 0, 1, 2 or 3;
b₂ is 0 or 1;
R6 is selected from deuterium, C₁-C₆ alkyl, C₁-C₆ haloalkyl, hydroxyl substituted C₁-C₆ alkyl, C₁-C₆ alkoxyalkyl, C₁-C₆ haloalkoxyalkyl, -CH₂NHC(O)Ra₅, -CH₂NRa₆Ra₇, wherein Ra₅, Ra6 and Ra₇ are each independently hydrogen, C1-3 alkyl unsubstituted or substituted by halogen, hydroxyl, or C3-6 cycloalkyl unsubstituted or substituted by halogen, hydroxyl.

4. The compound, the tautomer, enantiomer, diastereomer, racemate, metabolite, metabolic precursor, isotopic compound, pharmaceutically acceptable salt, ester, prodrug or hydrate thereof of claim 1:
wherein X₁ is -CH₂- or -NH-;
X₂ is -CH₂- or -CO-;
R₁ is hydrogen, deuterium, fluorine or methyl;
R₃ is selected from hydrogen, deuterium or fluorine;
R₂, R₄, n, Ⓐ and B have the same definition as claim 1.

5. The compound, the tautomer, enantiomer, diastereomer, racemate, metabolite, metabolic precursor, isotopic compound, pharmaceutically acceptable salt, ester, prodrug or hydrate thereof of claim 1, wherein the compound of formula (I) is the compound of formula (I-1) to (I-12): 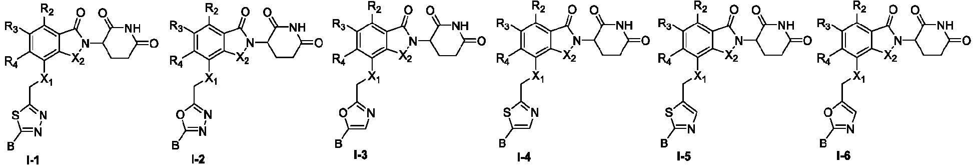 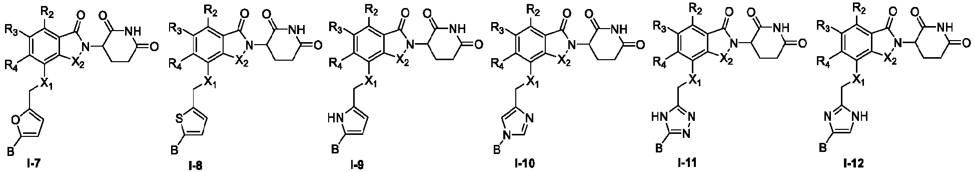 wherein X₁ is -CH₂-, -NH- or -O-;
X₂ is -CH₂- or -CO-;
R₂ and R₄ are each independently selected from hydrogen or deuterium;
R₃ is selected from hydrogen, deuterium or fluorine;
B has the same definition as claim 1, when X₁ is -O-, B is not

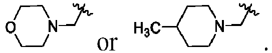

6. The compound, the tautomer, enantiomer, diastereomer, racemate, metabolite, metabolic precursor, isotopic compound, pharmaceutically acceptable salt, ester, prodrug or hydrate thereof of claim 1, wherein the compound of formula (I) is the compound of formula (1-13) to (I-18): 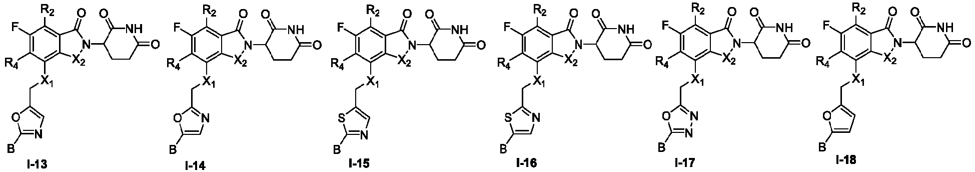 wherein X₁ is -CH₂-, -NH- or -O-;
X₂ is -CH₂- or -CO-;
R₂ and R₄ are each independently selected from hydrogen or deuterium;
B has the same definition as claim 1.

7. The compound, the tautomer, enantiomer, diastereomer, racemate, metabolite, metabolic precursor, isotopic compound, pharmaceutically acceptable salt, ester, prodrug or hydrate thereof of claim 1, wherein the compound of formula (I) is the compound of formula (1-19) to (I-24): 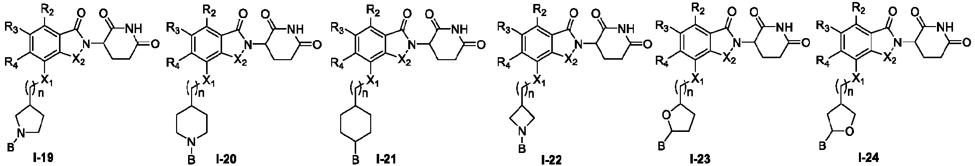 wherein X₁ is -CH₂-, -NH- or -O-;
X₂ is -CH₂- or -CO-;
R₂ and R₄ are each independently selected from hydrogen or deuterium;
R₃ is selected from hydrogen, deuterium or fluorine;
n is 1, 2 or 3;
B has the same definition as claim 1.

8. The compound, the tautomer, enantiomer, diastereomer, racemate, metabolite, metabolic precursor, isotopic compound, pharmaceutically acceptable salt, ester, prodrug or hydrate thereof of claim 1, wherein the compound of formula (I) is one of the following compounds:
| **Compound number** | **Compound structure** | **Compound number** | **Compound structure** |
|---|---|---|---|
| **1** | 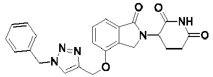 | **2** | 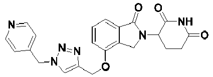 |
| **3** | 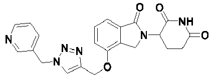 | **4** | 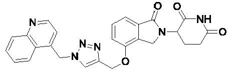 |
| **5** | 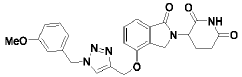 | **6** | 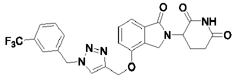 |
| **7** | 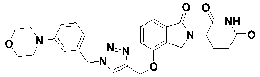 | **8** | 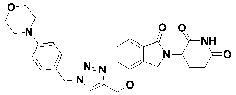 |
| **9** | 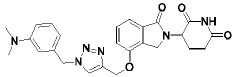 | **10** | 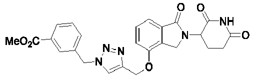 |
| **11** | 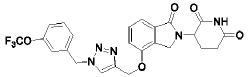 | **12** | 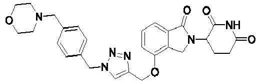 |
| **13** | 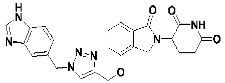 | **14** | 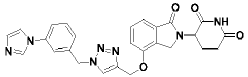 |
| **15** | 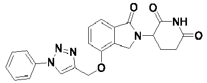 | **16** | 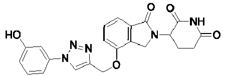 |
| **17** | 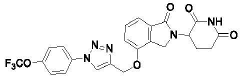 | **18** | 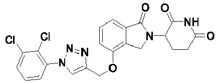 |
| **19** | 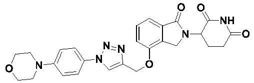 | **20** | 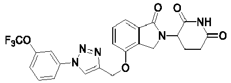 |
| **21** | 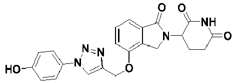 | **22** | 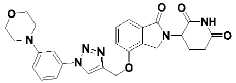 |
| **23** | 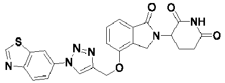 | **24** | 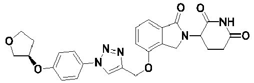 |
| **25** | 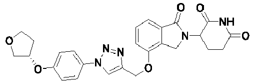 | **26** | 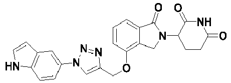 |
| **27** | 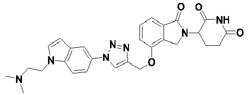 | **28** | 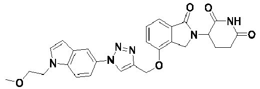 |
| **29** | 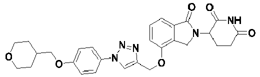 | **30** | 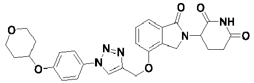 |
| **31** | 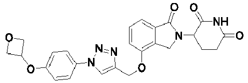 | **32** | 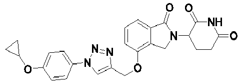 |
| **33** | 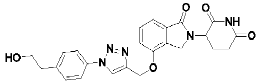 | **34** | 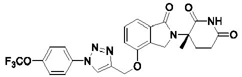 |
| **35** | 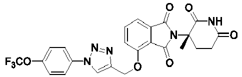 | **36** | 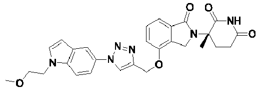 |
| **37** | 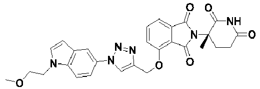 | **38** | 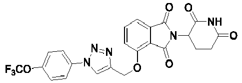 |
| **39** | 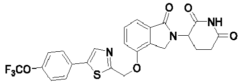 | **40** | 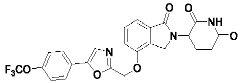 |
| **41** | 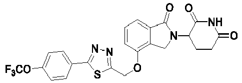 | **42** | 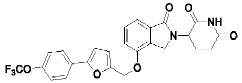 |
| **43** | 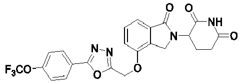 | **44** | 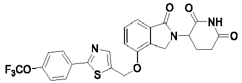 |
| **45** | 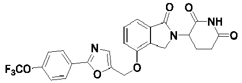 | **46** | 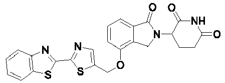 |
| **47** | 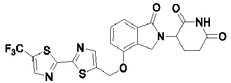 | **48** | 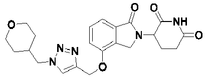 |
| **49** | 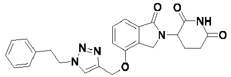 | **50** | 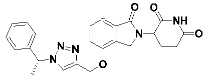 |
| **51** | 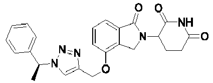 | **52** | 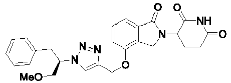 |
| **53** | 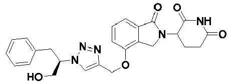 | **54** | 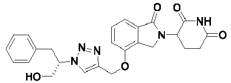 |
| **55** | 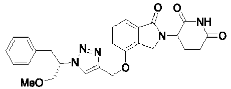 | **56** | 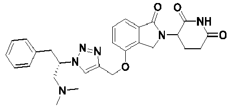 |
| **57** | 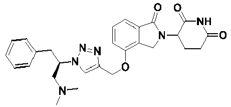 | **58** | 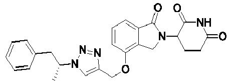 |
| **59** | 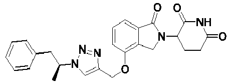 | **60** | 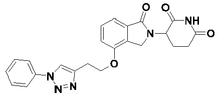 |
| **61** | 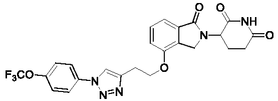 | **62** | 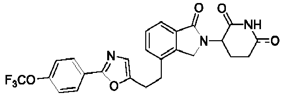 |
| **63** | 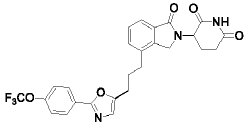 | **64** | 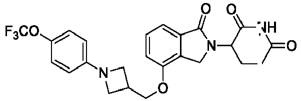 |
| **65** | 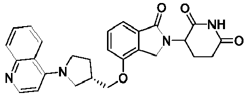 | **66** | 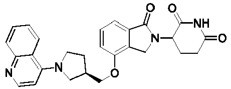 |
| **67** | 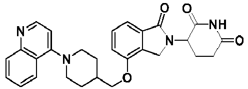 | **68** | 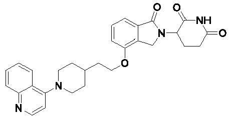 |
| **69** | 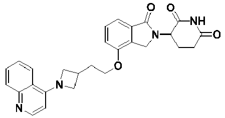 | **70** | 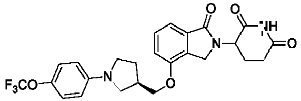 |
| **71** | 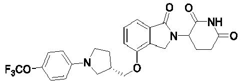 | **72** | 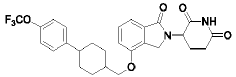 |
| **73** | 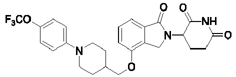 | **74** | 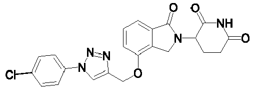 |
| **75** | 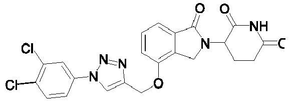 | **76** | 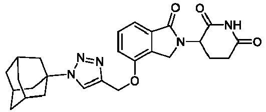 |
| **77** | 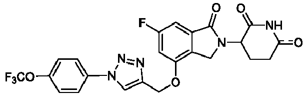 | **78** | 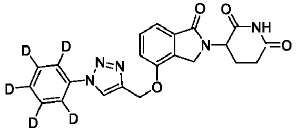 |
| **79** | 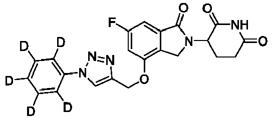 | **80** | 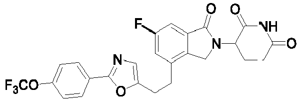 |
| **81** | 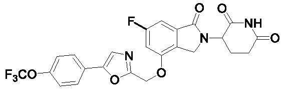 | **82** | 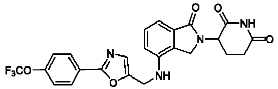 |
| **83** | 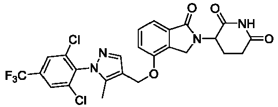 | **84** | 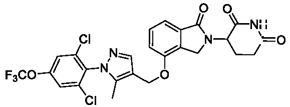 |
| **85** | 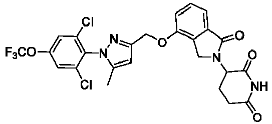 | **86** | 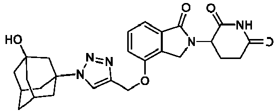 |
| **87** | 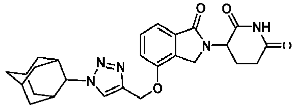 | **88** | 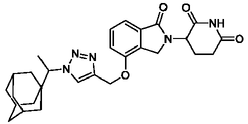 |
| **89** | 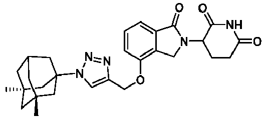 | **90** | 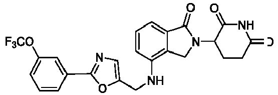 |
| **91** | 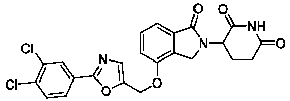 | **92** | 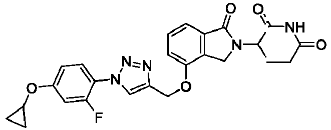 |
| **93** | 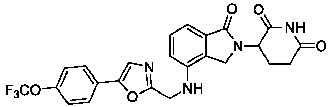 | **94** | 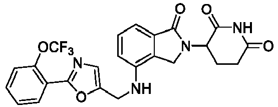 |

9. A method for preparing the compound of formula (I) of claims 1-8, wherein the method is selected from one of the following methods:
**synthesis method 1:** 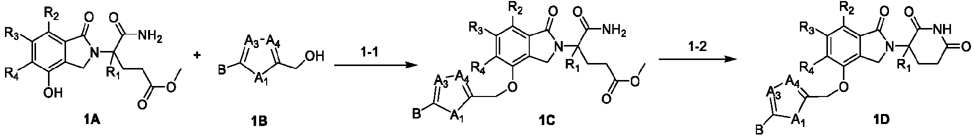 wherein R₁, R₂, R₃, R₄, A₁, A₃, A₄ and B have the same definitions as claim 1;
step 1-1: compound 1A and 1B were reacted under triphenylphosphine and diisopropyl azodicarboxylate to obtain compound 1C;
step 1-2: compound 1C was reacted to obtain compound 1D in the presence of potassium tert-butoxide;
**synthesis method 2:** 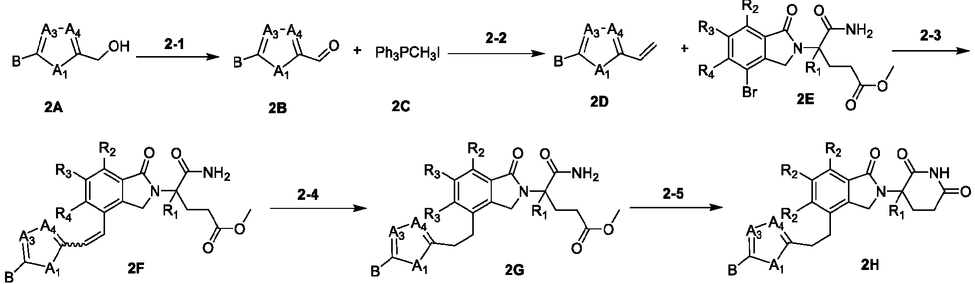 wherein R₁, R₂, R₃, R₄, A₁, A₃, A₄ and B have the same definitions as claim 1;
step 2-1: compound 2A was reacted to obtain compound 2B in the presence of manganese dioxide;
step 2-2: compound 2B and compound 2C were reacted in the presence of potassium tert-butoxide in tetrahydrofuran to obtain compound 2D;
step 2-3: compound 2D and compound 2E were reacted under the conditions of palladium catalyst, phosphine ligand, and organic base to obtain compound 2F;
step 2-4: compound 2F was reacted under palladium carbon and hydrogen at normal pressure to obtain compound 2G;
step 2-5: compound 1C was reacted to obtain compound 2H in the presence of potassium tert-butoxide.

10. A use of the compound, the tautomer, enantiomer, diastereomer, racemate, metabolite, metabolic precursor, isotopic compound, pharmaceutically acceptable salt, ester, prodrug or hydrate thereof of any one of claims 1-8 for the manufacture of a medicament for the prevention or treatment of disease related to CRL4^{CRBN} E3 ubiquitin ligase, preferably, the disease related to CRL4^{CRBN} E3 ubiquitin ligase is cancer, pain, central nervous system disease or immune system disease.

11. A use of the compound, the tautomer, enantiomer, diastereomer, racemate, metabolite, metabolic precursor, isotopic compound, pharmaceutically acceptable salt, ester, prodrug, or hydrate thereof of any one of claims 1-8 for the manufacture of a medicament for the treatment or prevention the diseases, disorders or conditions that are produced by TNF-α or regulated by TNF-α activity, produced by IL-2 or regulated by IL-2 activity, produced by IFNγ or abnormally regulated by IFNγ activity.

12. A pharmaceutical composition comprising therapeutically effective doses of the compound, the tautomer, enantiomer, diastereomer, racemate, metabolite, metabolic precursor, isotopic compound, pharmaceutically acceptable salt, ester, prodrug, hydrate, crystalline hydrate or solvate thereof of claim 1 and other pharmaceutically acceptable carriers.

13. The pharmaceutical composition of claim 12, it further comprises one or more ingredients with pharmaceutically therapeutic activity to produce synergistic effects in the prevention or treatment of specific diseases or dysfunctions; or to reduce or eliminate the toxic and side effects of one or more other ingredients with pharmaceutically therapeutic activity in the prevention or treatment of specific diseases or dysfunctions.

14. The pharmaceutical composition of claim 12, it further comprises one or more therapeutic agents selected from dexamethasone, rituximab, trastuzumab, PD-1 inhibitor, PDL-1 inhibitor, pemetrexed, topotecan, adriamycin, bortezomib, gemcitabine, dacarbazin, clarithromycin, vincristine, cytarabine, prednisone, docetaxel, clofarabine injection, HDAC inhibitor, androgen receptor inhibitor, androgen biosynthesis inhibitor, BTK inhibitor, erythrocyte growth hormone, minocycline, Elotuzumab, Palbociclib, Nivolumab, Pembrolizumab, Panobinostat, Ublituximab, Romidepsin, Eltrombopag, CAR-T and melphalan.

15. The use of claim 11, wherein the disease, disorder or condition is selected from Myelodysplastic syndrome, Multiple myeloma, Mantle cell lymphoma, Non-Hodgkin's lymphoma, Chronic lymphocytic leukemia, Chronic myelomonocytic leukemia, Myelofibrosis, Burkitt's lymphoma, Hodgkin's lymphoma, Large cell lymphoma, Diffuse large B-cell lymphoma, Follicular lymphoma, Ciliary body and chronic melanoma, Melanoma of iris, Recurrent interocular melanoma, T-cell lymphoma, Erythroid lymphoma, monoblast and monocytic leukemia, Myeloid leukemia, Central nervous system lymphoma, Brain tumors, meningiomas, Spinal cord tumor, Thyroid cancer, Non-small cell lung cancer, Ovarian cancer, skin cancer, Renal cell carcinoma, Astrocytoma, Amyloidosis, type I complex local pain syndrome, malignant melanoma, radiculopathy, myelofibrosis, glioblastoma, gliosarcoma, malignant glioma, refractory plasmacytoma, extraocular extension melanoma, solid tumor, papillary and follicular thyroid cancer, breast cancer, prostate cancer, hepatocellular carcinoma or primary macroglobulinemia.
